(19) Europäisches Patentamt
European Patent Office
Office européen des brevets

(11) Numéro de publication: **0 356 275 B1**

(12) **FASCICULE DE BREVET EUROPEEN**

(45) Date de publication de fascicule du brevet: **20.07.94**

(51) Int. Cl.[5]: **C08B 37/10**, C08B 37/08

(21) Numéro de dépôt: **89402086.6**

(22) Date de dépôt: **21.07.89**

# (54) Glycosaminoglycanes sélectivement O-acylés.

(30) Priorité: **21.07.88 FR 8809885**

(43) Date de publication de la demande:
**28.02.90 Bulletin 90/09**

(45) Mention de la délivrance du brevet:
**20.07.94 Bulletin 94/29**

(84) Etats contractants désignés:
**AT BE CH DE ES FR GB GR IT LI LU NL SE**

(56) Documents cités:
**EP-A-02 874 477**
**FR-A- 2 584 728**
**US-A- 4 331 697**

**CHEMICAL ABSTRACTS OF JAPAN, vol. 75, 1971, page 100, abstract no. 7728a, Columbus, Ohio, US; & JP-A-71 09 327 (SUMITOMO CHEMICAL CO. LTD) 09-03-1971**

(73) Titulaire: **ELF SANOFI**
**32-34, rue Marbeuf**
**F-75008 Paris(FR)**

(72) Inventeur: **Petitou, Maurice**
**Tour Mexico**
**65 rue du Javelot**
**F-75645 Paris Cédex 13(FR)**
Inventeur: **Lormeau, Jean-Claude**
**34 rue du 8 mai**
**F-76150 Maromme(FR)**
Inventeur: **Choay, Jean**
**21 rue St.Guillaume**
**F-75007 Paris(FR)**

(74) Mandataire: **Polus, Camille et al**
**c/o Cabinet Lavoix**
**2, Place d'Estienne d'Orves**
**F-75441 Paris Cedex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

L'invention concerne des glycosaminoglycanes sélectivement O-acylés, leur procédé de préparation ainsi que les compositions pharmaceutiques les contenant et leur usage en thérapeutique.

Par le terme "glycosaminoglycane" on entend les substances constituées d'enchaînements d'acides uroniques (acide D-glucuronique ou acide L-iduronique) et de sucres aminés, ces derniers étant des glucosamines ou des galactosamines.

Les glycosaminoglycanes d'origine naturelle sont constitués par des mélanges plus ou moins homogènes d'enchaînements d'unités disaccharidiques formées par une sous-unité uronique (acide glucuronique ou acide iduronique) et par une sous-unité osamine (glucosamine ou galactosamine) unies par une liaison 1_>4 ou 1_>3.

Dans les glycosaminoglycanes, les groupements hydroxyles sont diversement substitués par des groupements fonctionnels, en particulier des groupes sulfates, les groupements aminés des osamines étant substitués par des groupes sulfates et/ou acétyles.

Les glycosaminoglycanes considérés ici comprennent 6 types de produit: héparine, héparane sulfate, chondroïtine sulfate A, chondroïtine sulfate C, chondroïtine sulfate B, plus précisément dénommée dermatane sulfate, et acide hyaluronique. Ils sont caractérisés par les deux structures disaccharidiques de base (A) et (B) suivantes:

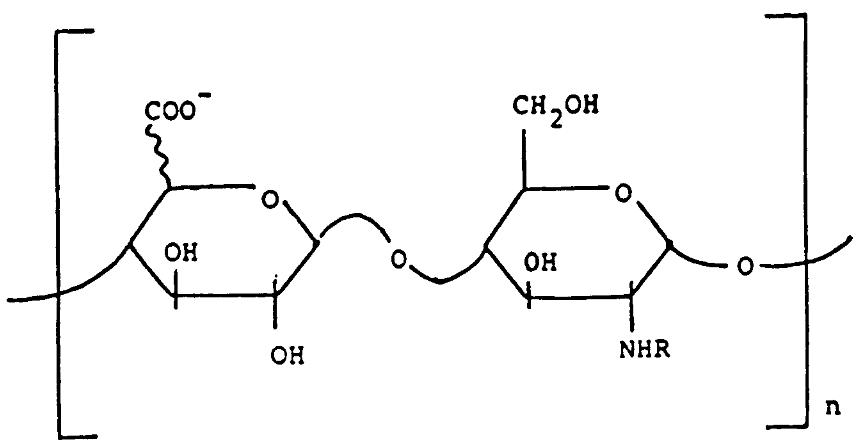

(A)

dans laquelle R est un groupe $SO_3^-$ et/ou un groupe acétyle et le signe ≀ indique que le groupe carboxyle peut être au-dessous du plan du cycle (unité iduronique) ou au-dessus du plan du cycle (unité glucuronique),
et

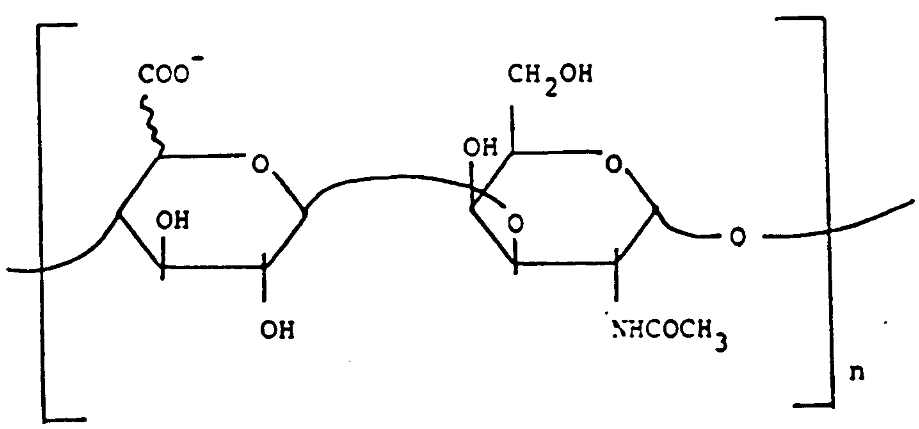

(B)

Les glycosaminoglycanes ayant la structure de base (A) ci-dessus sont dénommés glycosaminoglycanes (ou GAGs) de type héparinique et comprennent les héparines et les héparanes sulfates et les glycosaminoglycanes ayant la structure de base (B) ci-dessus sont dénommés GAGs de type chondroïtine sulfate et comprennent les chondroïtines sulfates A et C et le dermatane sulfate.

L'acide hyaluronique a la structure de base (B) dans laquelle la galactosamine est remplacée par une glucosamine.

Comme mentionné ci-dessus, tant dans les GAGs de type héparinique que dans les GAGs de type chondroïtine sulfate, un pourcentage plus ou moins élevé des groupes hydroxyles des n unités disaccharidiques est présent sous forme d'esters de l'acide sulfurique.

Ainsi, l'héparine peut être représentée par la structure de base (A) ci-dessus, dans laquelle n peut varier de 1 à 80, R étant dans une majorité des n unités un groupe $SO_3^-$ et, dans le reste des cas, un groupe acétyle, les groupes OH en position 6 de la glucosamine et en position 2 de l'acide uronique sont dans la majorité des espèces sulfatés, alors que le groupe OH en position 3 de la glucosamine n'est sulfaté que dans une minorité des espèces; le OH en position 3 de l'acide uronique est pratiquement non sulfaté, ledit acide uronique étant dans la majorité des espèces un acide iduronique.

Les produits ayant la structure de base (A) comprennent également l'héparine N-désulfatée N-acétylée qui peut être préparée comme décrit dans Nagasawa K. et Inoue Y., Methods in Carbohydrate Chemistry, Academic Press, 1980, vol. 8, pp. 291-294. Cette héparine est représentée par la formule (A) dans laquelle R est un groupe acétyle.

L'héparine contient également des espèces ayant la structure (A), avec n variable de 3 à 15 et le même profil chimique. Lesdites espèces peuvent être isolées par fractionnement selon des méthodes connus (par exemple US 4,692,435) et sont nommées héparines de bas poids moléculaire (b.p.m.).

Des héparines b.p.m. ayant la distribution moléculaire et les propriétés biologiques essentiellement identiques à celles des héparines b.p.m. obtenues par fractionnement ont été préparées par fragmentation de l'héparine selon les méthodes suivantes:

- méthode de dépolymérisation nitreuse et réduction (par exemple EP 37 319 ou US 4,686,288) qui coupe la molécule en attaquant les sous-unités glucosamines N-sulfate pour donner une terminaison 2,5-anhydromannitol

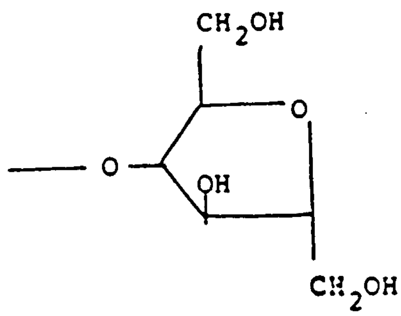

(A')

éventuellement sulfatée sur l'hydroxyle primaire en position 6;

- méthode de dépolymérisation enzymatique (par exemple EP 244 235 et 244 236) ou alcaline (par exemple EP 40 144) qui coupe la molécule en attaquant les sous-unités uroniques pour donner une terminaison acide uronique $\alpha$-$\beta$ insaturé

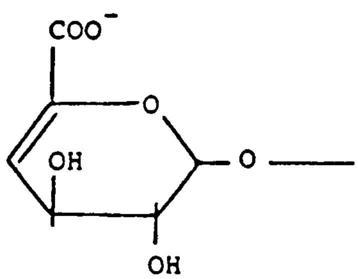

(A")

éventuellement 2-sulfaté;

- méthode de dépolymérisation oxydative (par exemple US 4,281,108 et EP 121067) qui coupe la molécule par oxydation tout en conservant les terminaisons "naturelles".

D'autres héparines b.p.m. qui ont une distribution moléculaire différente de celle de l'héparine naturelle et des propriétés biologiques particulières peuvent être préparées par oxydation periodique de l'héparine naturelle suivie de dépolymérisation par $\beta$-élimination ou hydrolyse acide. L'oxydation periodique coupe les

motifs acide uronique non sulfatés entre les carbones en position 2 et 3. On sait que de tels motifs sont présents dans le site de liaison à l'antithrombine III (AT III), inhibiteur plasmatique de diverses sérineprotéases de la coagulation, et dont l'activité est fortement accrue par l'héparine agissant comme cofacteur. L'oxydation periodique permet donc d'obtenir des produits dépourvus du site de liaison à l'AT III, et donc essentiellement dépourvus d'activité anticoagulante.

Des héparines de b.p.m. de ce type peuvent être obtenues en particulier en mettant en oeuvre le procédé comprenant les étapes suivantes, comme il est décrit dans la demande publiée sous le numéro EP-A-0 287 477.

- l'oxydation ménagée de l'héparine réalisée en faisant réagir de l'héparine, en solution aqueuse à une concentration finale de 0,5 à 5% (p/v) avec un sel de l'acide periodique à une concentration finale de 0,5 à 1% (p/v), à un pH situé entre 4,5 et 6,5, de préférence à un pH de 5, à une température de 0 à 10 °C, durant environ 15 à 48 heures, à l'abri de la lumière;
- la dépolymérisation des chaînes d'héparine obtenues, par addition d'une base forte telle que la soude, à un pH supérieur à 11 environ, notamment compris entre 11 et 12, avantageusement de 11,2 à 11,6, de préférence aux environs de 11,5;
- la réduction des fragments de dépolymérisation à l'aide d'un agent réducteur et, après élimination le cas échéant de l'agent réducteur n'avant pas réagi,
- la récupération des fragments d'héparine réduits, par précipitation à l'aide d'un solvant dans lequel ils sont insolubles;
- l'isolement des fragments recherchés par fractionnement à l'aide d'alcool, en présence d'un sel minéral, d'une solution aqueuse obtenue en remettant en solution dans l'eau le précipité précédemment isolé et en récupérant le précipité formé.

Ces héparines b.p.m. répondent à la formule (C) ci-dessous:

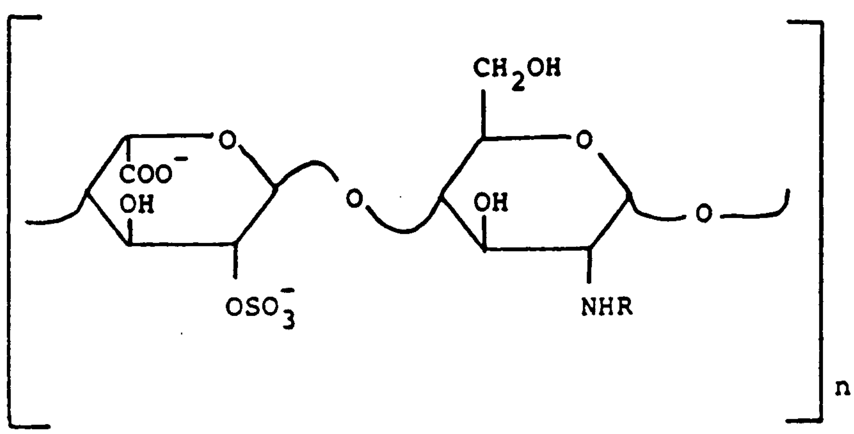

(C)

dans laquelle n peut varier de 6 à 15, R est dans environ au moins 90% des n unités un groupe $SO_3^-$ et, dans le reste des cas, un groupe acétyle, les groupes OH en position 3 de la glucosamine pouvant être sulfatés, et les groupes OH en position 6 de la glucosamine étant sulfatés dans 70% des espèces. De plus, à raison de 1 motif pour 2 chaînes au moins de cette héparine b.p.m., l'acide iduronique est remplacé par un motif acide uronique (D-glucuronique ou L-iduronique) non sulfaté ouvert entre les carbones en position 2 et 3, de formule suivante:

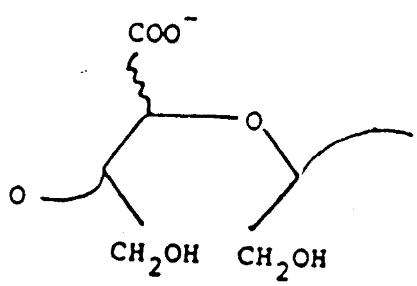

De telles héparines b.p.m. peuvent être encore fractionnées par gel filtration selon des techniques connues afin d'obtenir des mélanges de fragments homogènes au regard de leur masse moléculaire, dépourvus du site de liaison à l'ATIII.

D'autres glycosaminoglycanes de type héparinique sont constitués par des GAGs de formule (A) ou (C), dans lesquels la fonction carboxylique portée par l'acide uronique est estérifiée, par exemple par condensation d'un alcool en présence d'un agent de condensation de type carbodiimide, comme décrit dans le brevet FR nº 2 159 724, ou encore par alkylation du carboxyle à l'aide d'un halogénure d'alkyle en présence d'une base faible.

De même, des glycosaminoglycanes de type chondroïtine sulfate sont constitués par des glycosaminoglycanes de formule (B) dans lesquels les fonctions carboxyliques des acides uroniques sont estérifiées, par exemple par alkylation du carboxyle à l'aide d'un halogénure d'alkyle en présence d'une base faible.

L'obtention de tels esters pour l'acide hyaluronique est décrite dans le brevet EP nº 215 453, par traitement de l'acide hyaluronique sous forme de sel d'ammonium quaternaire par un alcool en présence d'un catalyseur ou par un agent éthérifiant.

L'héparane sulfate est représenté par la formule (A) ci-dessus, dans laquelle n peut varier de 1 à 80, R représente dans la majorité des espèces un groupe acétyle et dans un minorité d'espèces $SO_3^-$ . En outre, dans la grande majorité des espèces, les groupes OH en position 6 de la glucosamine sont sulfatés, l'acide uronique étant dans la majorité des espèces l'acide glucuronique.

Les chondroïtines sulfates A et C sont représentées par la formule (B) ci-dessus dans laquelle n peut varier de 1 à 80 et, respectivement, les groupes 4-OH et 6-OH de la glucosamine sont sulfatés, l'acide uronique étant dans la majorité des espèces un acide glucuronique.

Le dermatane sulfate a une structure substantiellement identique à celle de la chondroïtine sulfate A, mais la sous-unité acide uronique est un acide iduronique dans la majorité des espèces.

Des fragments de dermatane sulfate, répondant à la formule (B) ci-dessus, dans laquelle n peut varier de 1 à 20, peuvent être obtenus par oxydation periodique suivie d'une réduction au borohydrure de sodium et d'une hydrolyse acide, comme décrit dans FRANSSON L.A. et CARLSTEDT I,, Carbohydr. Res., 36 - (1974), 349-358.

Les glycosaminoglycanes possèdent de nombreuses activités biologiques, parmi lesquelles on peut citer leurs activités vis-à-vis des facteurs de la coagulation, qui peuvent s'exercer par l'intermédiaire de diverses protéines plasmatiques. En ce qui concerne l'héparine, il est également connu dans la littérature que l'héparine ou certains de ses dérivés possédant ou non une activité anticoagulante peuvent avoir une activité régulatrice de la prolifération des cellules musculaires lisses de la paroi vasculaire (GUYTON et al, Circ. Res., 46 (1980), 625-634) ou encore une activité inhibritrice de l'héparanase, enzyme impliquée dans les mécanismes de dissémination métastasique (demande EP nº 254 067). Par ailleurs, les glycosaminoglycanes font partie de la famille plus large des polysaccharides sulfatés, certains ayant montré à des degrés plus ou moins importants une activité anti virale (BABA et al, Antimicrob Agents Chemother, 32 (1988) 1742-1745 ) et en particulier une activité anti-VIH (virus de l'immunodéficience humaine) (BABA et al, Proc. Natl. Acad. Sci., 85 (1988) 6132-6136).

Dans la suite du texte, on utilisera le terme GAG pour désigner un glycosaminoglycane, celui-ci ayant soit une structure naturelle telle qu'obtenue par extraction, hémi synthèse ou synthèse, soit une structure chimiquement modifiée sur les groupements fonctionnels carboxyles ou aminés,préalablement à la réaction d'acylation donnant les GAGs sélectivement O-acylés.

Malgré leurs activités pharmacologiques de grand intérêt, les GAGs naturels présentent l'inconvénient d'avoir une demi-vie relativement courte, obligeant à des administrations répétées. Cet inconvénient a été en partie pallié par l'utilisation dans la prévention et le traitement des thromboses veineuses, de dérivés d'héparine de faible masse moléculaire, par voie souscutanée, abaissant ainsi la fréquence d'administration à une injection par jour.

Néanmoins, il est d'un grand intérêt de pouvoir disposer de dérivés possédant une action retard, ce qui permettrait de diminuer encore la fréquence d'administration de ces produits en augmentant leur durée d'action.

Il peut également être de grand intérêt de disposer de dérivés "retard"non anticoagulants tels que ceux de l'héparine N-désulfatée N-acétylée dont l'activité comme inhibiteur d'héparanase, enzyme impliquée dans les phénomènes de dissémination métastasiques, est rappelée plus haut.

La littérature décrit de nombreux dérivés de glycosaminoglycanes modifiés de façon à améliorer leur pharmacocinétique, notamment des esters de la fonction carboxylique des acides uroniques ou des fonctions hydroxyles.

On a utilisé en particulier l'estérification partielle ou totale des fonctions carboxyles de l'héparine, traitée sous forme de sel d'ammonium quaternaire, en solvant inerte, par un alcool ou un dérivé halogéné, en

présence éventuellement d'un agent de condensation (Br. FR n° N2 159 724 et EP n° 44 228). Des dérivés d'acide hyaluronique obtenus par estérification à l'aide d'alcool en présence d'un catalyseur ou par réaction avec un agent éthérifiant ont également été décrits (Br. EP n° 216 453).

Par ailleurs, différents moyens ont été décrits dans la littérature en vue d'estérifier les GAGs sur les fonctions hydroxyles primaires et secondaires.

La demande de brevet français publiée sous le numéro 2.584.728, décrit un procédé de sulfatation de glycosaminoglycanes ou de fragments de glycosaminoglycanes, permettant d'introduire des groupes esters sulfuriques à la place des hydroxyles primaires.

La publication Can. J. Res., 25B,(1947), 472-476, décrit un dérivé acétylé de l'héparine correspondant à une mole de groupe acétyle pour quatre unités saccharidiques. Le produit est préparé par action du cétène sur l'héparine dans l'acétone. Bien que dans cette publication il soit spécifié que le produit obtenu est un dérivé O-acétylé, il est bien connu que le cétène en présence de groupes carboxyliques donne des anhydrides. (cf. J. March, Advanced Organic Chemistry ; Reactions, Mechanisms and Structure, J. Willey and Sons Eds. 1985, pp. 686-687 : "With ketenes, carboxylic acids give anhydrides and acetics anhydride is prepared industrially in this manner :

$$CH_2 = C = O + CH_3 - COOH \longrightarrow CH_3 - C(=O) - O - COCH_3$$ ")

Par conséquent, cette publication décrit en fait une héparine dans laquelle des groupes O-acétyles sont associés à des anhydrides mixtes entre le groupe $COO^-$ de l'acide uronique et le groupe acétyle provenant du cétène, ce qui peut expliquer leur instabilité dans l'eau.

Le brevet FR 2 100 735 décrit des esters partiels hydrolysables d'héparine et d'un acide organique non toxique, notamment l'acide 4-chlorophénoxyisobutyrique, l'acide 4-chlorophénoxyacétique, l'acide cholique, l'acide nicotinique, l'acide pyridylacétique, l'acide N-oxy-pyridylacétique ou l'acide linoléique. La méthode de préparation décrite dans le brevet ci-dessus, qui est caractérisée par la réaction d'un sel quaternaire de l'héparine avec l'acide activé par une carbodiimide, donne non seulement le dérivé O-acylé, mais également en quantité importante un produit secondaire stable sous forme d'un dérivé ester O-acylisourée.En outre, un tel type de réaction est susceptible de favoriser l'obtention d'anhydrides et des réactions intramoléculaires entre les fonctions acides et les fonctions hydroxyles de l'héparine.

Le brevet JP 74/048533 décrit des O-esters de chondroïtine sulfate avec des acides aromatiques, arylaliphatiques ou hétérocycliques éventuellement substitués dotés d'activité prolongée. La méthode de préparation décrite dans ce document, qui est caractérisée par la réaction de l'acide chondroïtesulfurique avec un chlorure d'acide, donne comme réaction secondaire une N-acylation.

Le brevet WO 83/00150 décrit de façon générale des prodrogues de médicaments préparées en utilisant les différentes fonctions des GAGs, dont l'hydroxyle et, de façon spécifique, un O-ester du chondroïtinesulfate avec la pénicilline V. La préparation de ce produit est effectuée via carbodiimide et comporte donc les réactions secondaires mentionnées ci-dessus.

Le brevet EP-0.046.828 et le brevet équivalent US-4.331.697, décrivent des O-esters de l'héparine avec des acides insaturés, notamment l'acide acrylique ou métacrylique qui, greffés sur des matériaux biomédicaux, leur confèrent une activité antithrombotique de longue durée. Ce greffage est effectué par liaison covalente au niveau des liaisons $\alpha$-$\beta$ insaturées de ces esters avec la surface desdits matériaux qui entrent en contact avec le sang. Selon ce document, les O-esters alpha, béta-insaturés sont préparés par réaction de l'héparine avec le chlorure ou l'anhydride d'un acide carboxylique alpha, béta-insaturé. De plus, le descriptif qui indique indifféremment l'utilisation des chlorures ou des anhydrides des acides alpha, béta-insaturés comme réactifs ne spécifie pas quels types d'O-esters de l'héparine sont ainsi obtenus.

Le brevet EP 256 880 décrit l'estérification d'héparine ou d'héparine de bas poids moléculaire par l'action de chlorures d'acide dans le formamide et la pyridine afin de donner des dérivés à perméabilité transmembranaire accrue. Or, cette méthode conduit à des dérivés qui subissent une désulfatation partielle et sont O- mais également N-acétylés, et dans lesquels le rapport sulfate/carboxyle est altéré. Un procédé analogue est aussi décrit dans Chemical Abstracts, Vol 75, 1971, page 100, abstract 7728a. Selon cette méthode, on procède à une O-acylation des chondroïtines sulfates à l'aide de chlorures d'acides gras dans des solvants organiques ou aqueux alcalins.

Le brevet FR 3 066M décrit l'acétylation du N-monométhylhéparinamide par l'action de l'anhydride acétique dans le formamide et la pyridine. Le produit de départ est un dérivé d'héparine dont les fonctions

carboxyles sont remplacées par les fonctions amides.

Le procédé utilisé pour l'acétylation ne met pas en oeuvre un sel soluble en milieu organique. De ce fait, la réaction d'acétylation ne peut être contrôlée avec précision et ne permet d'obtenir qu'un faible rendement d'acétylation. De plus, si l'on applique le procédé décrit à de l'héparine dont les fonctions carboxyles ne sont pas bloquées, on obtient une formation importante d'anhydrides mixtes entre les groupes carboxyles de l'héparine et l'anhydride acétique, ces produits secondaires indésirables n'étant pas éliminés du milieu.

Le brevet JP 5128602 décrit l'acylation de l'héparine par l'action d'un anhydride d'acide dans le formamide. Dans le procédé utilisé, l'héparine ne se trouve pas sous la forme d'un sel soluble en milieu organique, ce qui ne permet pas de contrôler la réaction d'acylation avec précision et entraîne l'obtention d'un faible taux d'acétylation. De plus, le procédé décrit entraîne la présence dans le milieu d'anhydrides mixtes, produits secondaires de la réaction.

Il était donc intéressant de disposer de produits plus spécifiques dont le procédé d'obtention permet une bonne reproductibilité.

On a maintenant trouvé qu'en faisant réagir un sel soluble en milieu organique d'un GAG, tel qu'un sel d'ammonium tertiaire ou quaternaire, avec l'anhydride d'un acide carboxylique dans un solvant organique aprotique polaire, on obtient une acylation sélective des hydroxyles libres, sans altérer les groupes fonctionnels carboxyliques ou aminés du GAG mis en oeuvre. La mise en oeuvre du GAG sous forme d'un sel soluble en milieu organique permet avantageusement de contrôler la réaction d'acylation avec une grande précision. De plus, le taux d'acylation est aisément modulable et peut être élevé sans que cela n'entraîne d'altération sur le reste de la molécule. Notamment, on peut obtenir un taux d'acylation de 0,1 à 3 groupes acyles par unité disaccharidique, et en particulier de 0,5 à 2 groupes acyles.

On a également trouvé qu'aucun dérivé N-acylé ne se forme et que, lorsque des anhydrides se forment au niveau des fonctions carboxyliques, l'utilisation d'une base faible permet aisément de les retransformer en acide libre.

De manière avantageuse, l'acylation sélective des GAGs selon le procédé de l'invention permet une modulation de leurs activités biologiques, qui dans certains cas se trouve fortement accrue.

On a finalement trouvé que les GAGs O-acylés ainsi obtenus ont une activité pharmacologique de plus longue durée.

Ainsi, la présente invention a pour objet, selon l'un des aspects, des glycosaminoglycanes sélectivement O-acylés, répondant à la formule I suivante :

$$A - [G - U]_n - B \qquad (I)$$

dans laquelle :

- G représente un groupe (a) de formule :

(a) 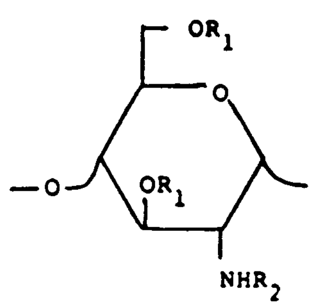

ou un groupe (a') de formule :

(a')

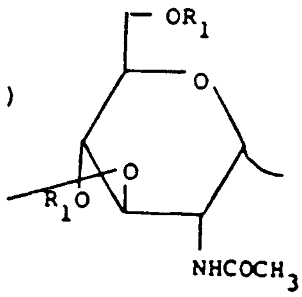

ou un groupe (b) de formule :

(b)

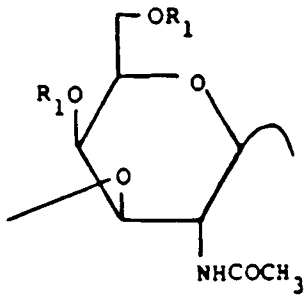

- U représente un groupe (c) de formule :

(c)

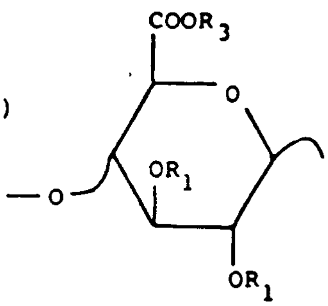

ou un groupe (d) de formule :

(d)

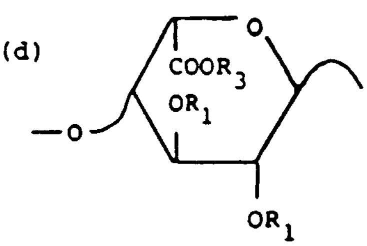

ou le résidu du groupe (c) ou du groupe (d) après oxydation periodique suivie d'une $\beta$-élimination ou d'une hydrolyse acide;

- A représente un groupe $R_1$, un groupe $R_1$-(c), ou un groupe $R_1$-(d), ou un groupe (e) de formule :

(e)

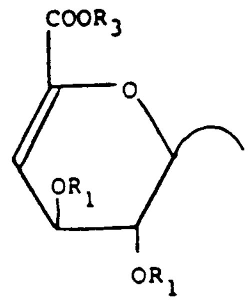

ou le résidu du groupe (c) ou du groupe (d) après oxydation periodique suivie d'une $\beta$-élimination ou d'une hydrolyse acide ;
- B représente un groupe O-$R_1$, un groupe (a)-$OR_1$, un groupe (a′)-$OR_1$, un groupe (b)-$OR_1$, un groupe (f) de formule :

(f)

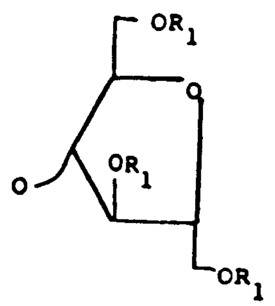

ou un groupe (g) de formule :

(g)

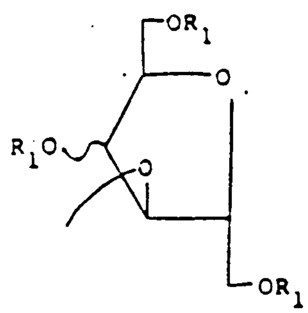

ou représente un groupe (a), un groupe (a'), ou un groupe (b) auxquels reste accroché un résidu de (c) ou de (d) tel que présent après oxydation periodique suivie d'une $\beta$-élimination ou d'une hydrolyse acide ;
- $R_1$ représente H, $SO_3^-$ ou un radical acyle d'un acide carboxylique ou dicarboxylique non $\alpha,\beta$-insaturé, choisi parmi :
  . un groupe alcanoyle de 1 à 18 atomes de carbone ;
  . un groupe alcanoyle de 2 à 3 atomes de carbone substitué par:
- un groupe cycloalkyle de 3 à 7 atomes de carbone,
- un radical hydrocarboné aliphatique insaturé de 4 à 16 atomes de carbone ;
  . un groupe benzoyle éventuellement substitué par un ou plusieurs radicaux alkyles de 1 à 4 atomes de carbone, atomes d'halogène ou groupement $NO_2$ ou $OCH_3$ ;
  . un groupe cycloalkyle (3-7 C) carbonyle ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;

- $R_3$ représente un atome d'hydrogène, un radical alkyle de 1 à 10 atomes de carbone, de préférence de 1 à 4 atomes de carbone, un radical phényl-alkyle de 7 à 12 atomes de carbone, ou un cation métallique alcalin ou alkalino-terreux ;
- n étant un nombre entier de 1 à 80,

$R_1$ étant acyle dans la proportion d'au moins 0,1 à 3 groupes acyles par unité disaccharidique, de préférence de 0,5 à 2 groupes acyles, et leurs sels pharmaceutiquement acceptables,
susceptibles d'être obtenus à partir d'un glycosaminoglycane choisi dans le groupe constitué par :
l'héparine, un mélange de fragments d'héparine ayant une masse moléculaire inférieure à 10.000 daltons, un mélange de fragments d'héparine ayant une masse moléculaire moyenne se situant entre 2.000 et 7.000 daltons, un mélange de fragments d'héparine ayant une masse moléculaire moyenne d'environ 4.500 daltons, un mélange de fragments d'héparine ayant une masse moléculaire moyenne d'environ 2.500 daltons, ou un mélange de fragments d'héparine de même masse moléculaire, une fraction ou un fragment d'héparine dépourvus de site de fixation à l'antithrombine III,
le dermatane sulfate et ses fragments,
que :

(1) on transforme en un sel d'amine tertiaire ou en un sel d'ammonium quaternaire dudit glycosaminoglycane, soluble dans un solvant organique aprotique polaire ;
(2) on traite ce sel par un anhydride de formule :

$R_1$-O-$R_1$

dans laquelle,
$R_1$ représente un radical acyle tel qu'indiqué précédemment, dans ledit solvant organique aprotique polaire, en présence de quantités catalytiques de pyridine ou d'une dialkylaminopyridine et d'un accepteur de protons à une température située entre 0°C et 100°C ;
(3) on précipite le produit ainsi obtenu par action d'une solution d'acétate de sodium dans l'éthanol et,
(4) on isole le glycosaminoglycane sélectivement O-acylé par dissolution dans l'eau du précipité ainsi obtenu et par dialyse, et l'on transforme éventuellement le sel de sodium du glycosaminoglycane sélectivement O-acylé ainsi obtenu en un autre sel pharmaceutiquement acceptable.

Dans un mode préféré de l'invention, les glycosaminoglycanes choisis sont des glycosaminoglycanes de type héparinique, à savoir, l'héparine les dérivés de l'héparine obtenus soit par fractionnement, soit par hémi-synthèse ou synthèse, l'héparane sulfate et ses dérivés obtenus par fractionnement, hémi-synthèse ou synthèse.

Les glycosaminoglycanes de ce type, sélectivement O-acylés selon la présente invention répondent à la formule II suivante :

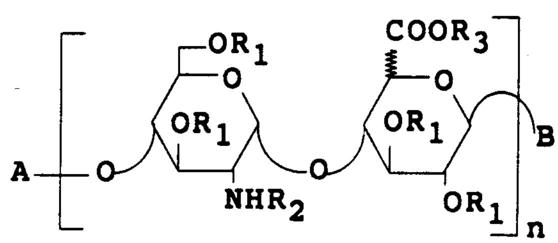

(II)

dans laquelle :

- A a les significations données plus haut dans la formule (I) ;
- $R_1$ représente H et/ou $SO_3^-$ et/ou un radical acyle, tel que défini dans la formule (I) ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, de préférence de 1 à 4 atomes de carbone, un radical phényl-alkyle de 7 à 12 atomes de carbone, ou un cation métallique alcalin ou alkalino-terreux ;
- B représente (a)-$OR_1$ ou (f), (a) et (f) étant tels que définis dans la formule (I), ou $OR_1$, ou un groupe (a) auquel reste accroché un résidu de (c) ou de (d) tel que présent après oxydation periodique suivie d'une $\beta$-élimination ou d'une hydrolyse acide ;
- n est un nombre entier de 1 à 80,

Une famille préférée IIa de composés de l'invention sont des composés de formule II :

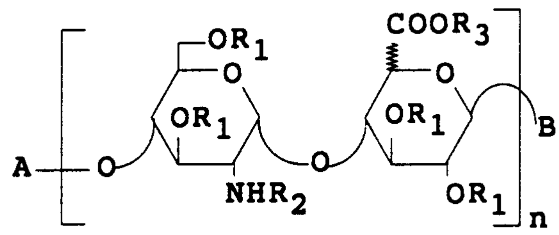

(II)

dans laquelle :

- A a les significations donnés plus haut dans la formule (I) ;
- $R_1$ représente H et/ou $SO_3^-$ et/ou un radical acyle tel que défini dans la formule (I),
- $R_2$ représente $SO_3^-$ et/ou un radical acétyle, sous réserve que la proportion de N-acétyl glucosamine soit au plus égale à celle de l'héparine lorsque $R_1$ représente un radical acétyle,
- $R_3$ représente un atome d'hydrogène, un radical alkyle de 1 à 10 atomes de carbone, de préférence de 1 à 4 atomes de carbone, un radical alkyle substitué de 7 à 12 atomes de carbone, ou un cation métallique alcalin ou alcalino-terreux,
- B représente (a)-$OR_1$ ou (f), (a) et (f) étant tels que définis dans la formule (I), ou $OR_1$, ou un groupe (a) auquel reste accroché un résidu de (c) ou de (d) tel que présent après oxydation periodique suivie d'une $\beta$-élimination ou d'une hydrolyse acide,
- n est un nombre entier de 1 à 80, sous réserve que, lorsque A représente $R_1$, un groupe $R_1$-(c) ou un groupe $R_1$-(d) et que B représente O-$R_1$, n soit un nombre entier de 1 à 16.

Une autre famille préférée IIb de composés de l'invention correspond à des composés de formule II :

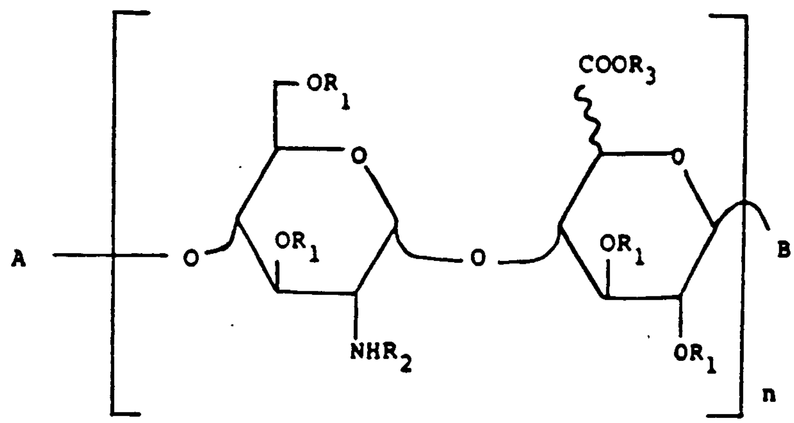

(II)

dans laquelle

- A a les significations donnés plus haut dans la formule (I) ;
- $R_1$ représente H et/ou $SO_3^-$ et/ou un radical acyle, d'un acide carboxylique ou dicarboxylique, non $\alpha,\beta$-insaturéchoisi parmi :
    - ♦ un groupe alcanoyle de 4 à 18 atomes de carbone ;
    - ♦ un groupe alcanoyle de 2 à 3 atomes de carbone substitué par :
        - un groupe cycloalkyle de 3 à 7 atomes de carbone,
        - un radical hydrocarboné aliphatique insaturé de 4 à 16 atomes de carbone ;
    - ♦ un groupe benzoyle éventuellement substitué par un ou plusieurs radicaux alkyles de 1 à 4 atomes de carbone, atomes d'halogène ou groupement $NO_2$ ou $OCH_3$ ;
    - ♦ un groupe cycloalkyle (3-7 C) carbonyle ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- n est un nombre entier de 1 à 80,

$R_1$ étant acyle dans la proportion d'au moins 0,5 à 2 groupes acyles par unité disaccharidique, de

préférence 1 groupe acyle.

Des composés avantageux de l'invention sont des composés appartenant aux familles (IIa) et (IIb), dans lesquelles $R_3$ représente un atome d'hydrogène ou un cation métallique alcalin ou alcalino-terreux.

D'autres groupes de composés avantageux correspondent aux composés appartenant aux familles (IIa) et (IIb), dans lesquelles $R_3$ représente un radical alkyle de 1 à 10 atomes de carbone, de préférence de 1 à 4 atomes de carbone, ou un radical alkyle substitué de 7 à 12 atomes de carbone.

Dans un aspect préféré de l'invention, on utilise des mélanges de fragments d'héparine ayant une masse moléculaire inférieure à 10.000 daltons, en particulier soit ceux ayant une masse moléculaire moyenne se situant entre 2.000 et 7.000 daltons, soit ceux ayant une masse moléculaire moyenne d'environ 4.500 daltons, soit encore ceux ayant une masse moléculaire moyenne d'environ 2.500 daltons.

On peut avantageusement utiliser pour les obtenir un procédé de dépolymérisation à l'acide nitreux, comme décrit par exemple dans le brevet EP 37319. Les glycosaminoglycanes sélectivement O-acylés de l'invention sont alors représentés par la formule III suivante :

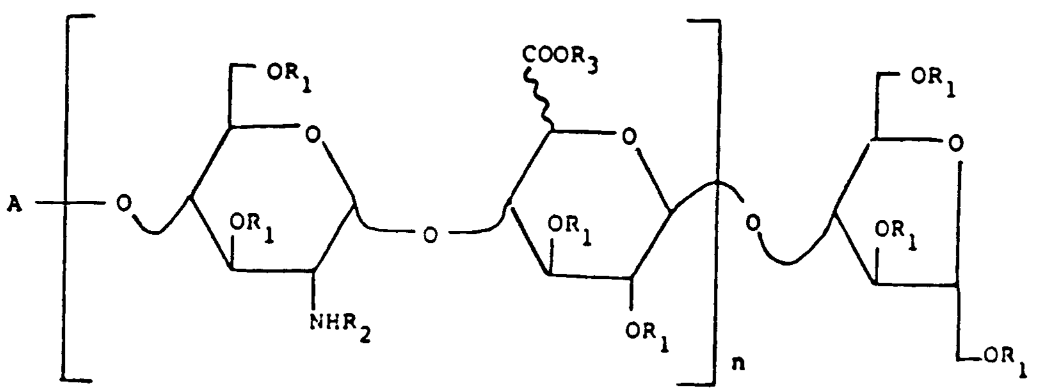

(III)

dans laquelle :

- A représente $R_1$, ou $R_1$-(c) ou $R_1$-(d), tels que définis dans la formule (I) ;
- $R_1$ représente H, et/ou $SO_3^-$ et/ou un groupe acyle tel que défini dans la formule (I) ;
- $R_2$ représente $-SO_3^-$ , et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, et/ou un radical alkyle de 1 à 10 atomes de carbone, de préférence de 1 à 4 atomes de carbone, ou un radical phényl-alkyle de 7 à 12 atomes de carbone, ou un cation métallique alcalin ou alcalino-terreux;
- n est un nombre entier de 3 à 12.

Des composés préférés de l'invention correspondent aux composés de formule (III) dans lesquels $R_1$ est un radical alcanoyle de 4 à 10 atomes de carbone.

Pour obtenir des mélanges de fragments d'héparine ayant une masse moléculaire inférieure à 10.000 daltons, on peut également utiliser une méthode de dépolymérisation enzymatique, par exemple comme décrit dans les brevets EP 244235 et 244236, ou de dépolymérisation alcaline, par exemple comme décrit dans le brevet EP 40144.

Dans ce cas, les glycosaminoglycanes sélectivement O-acylés selon l'invention sont représentés par la formule IV suivante :

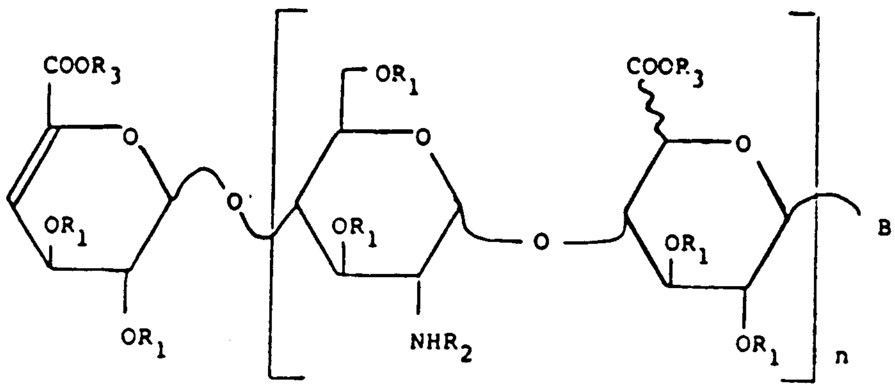

(IV)

dans laquelle

- $R_1$ représente H, et/ou $SO_3^-$, et/ou un groupe acyle tel que défini dans la formule (I) ;
- $R_2$ représente $SO_3^-$, et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, et/ou un radical alkyle de 1 à 10 atomes de carbone, de préférence de 1 à 4 atomes de carbone, ou un radical phényl-alkyle de 7 à 12 atomes de carbone, ou un cation métallique alcalin ou alcalino-terreux;
- B représente (a)-$OR_1$, tel que défini dans la formule (I), ou $OR_1$;
- n est un nombre entier de 2 à 20.

Parmi les composés de formule (IV), des composés avantageux sont ceux dans lesquels $R_1$ est un radical alcanoyle de 4 à 10 atomes de carbone.

Dans un autre aspect avantageux de l'invention, on peut utiliser un mélange de fragments d'héparine homogènes au regard de leur masse moléculaire, un fragment d'héparine obtenu par synthèse, homogène tant en ce qui concerne sa masse moléculaire qu'en ce qui concerne sa fonctionnalisation.

Dans un autre aspect intéressant de l'invention, on peut choisir comme glycosaminoglycane des dérivés de l'héparine dépourvus de site de liaison à l'antithrombine III (AT III), soit que les chaînes d'héparine aient été fractionnées de manière à éliminer les chaînes oligosaccharidiques comportant le site de liaison à l'AT III en ayant recours par exemple à une chromatographie d'affinité sur résine Sépharose-AT III ou à des chromatographies d'échange d'ions, comme il est décrit dans E. Sache et al, Thromb. Res., 25, (1982), pp. 442-458, soit que ces sites aient été détruits par exemple par dépolymérisation au périodate suivie d'une $\beta$-élimination ou d'une hydrolyse acide.

De tels composés peuvent répondre à la formule V suivante :

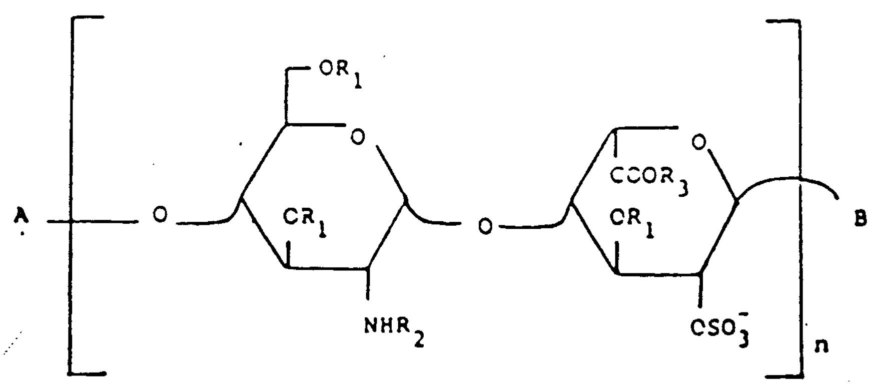

(V)

dans laquelle :

- A représente $R_1$-(c), $R_1$-(d) ou le résidu de (c) ou de (d) après oxydation periodique suivie d'une $\beta$-élimination ou d'une hydrolyse acide ;
- B représente (a)-$OR_1$, ou un groupe (a) auquel reste accroché un résidu de (c) ou de (d) tel que présent après oxydation periodique suivie d'une $\beta$-élimination ou d'une hydrolyse acide ;
- $R_1$ a les significations données dans la formule (I) ;

- $R_2$ représente $SO_3^-$, ou un radical acétyle, la proportion de $SO_3^-$ étant d'environ 90% ;
- $R_3$ représente un atome d'hydrogène ou un cation métallique alcalin ou alcalino-terreux;
- n est un nombre entier de 1 à 80.

Des composés particulièrement avantageux peuvent être obtenus à partir de composés préparés par le procédé mettant en oeuvre une oxydation periodique suivie de $\beta$-élimination alcaline, d'une réduction et d'un fractionnement comme décrit plus haut. Ces composés répondent à la formule VI suivante :

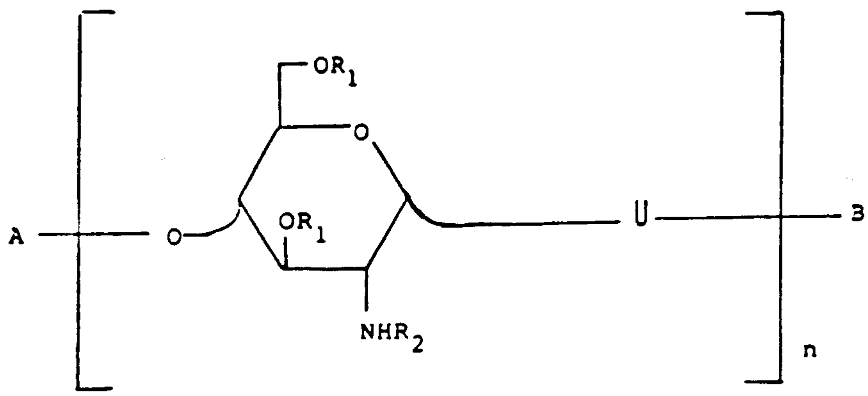

(VI)

dans laquelle :

- A représente $R_1$, $R_1$-(c), $R_1$-(d), ou le résidu de (c), ou de (d) après oxydation periodique suivie d'une $\beta$-élimination;
- U représente :

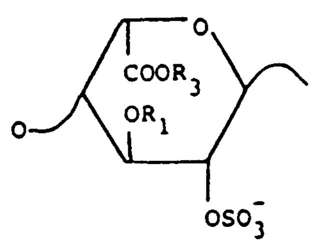

ou, à raison d'un motif pour deux chaînes au moins, un acide uronique (D-glucuronique ou L-iduronique) non sulfaté ouvert entre les carbones en positions 2 et 3, de formule :

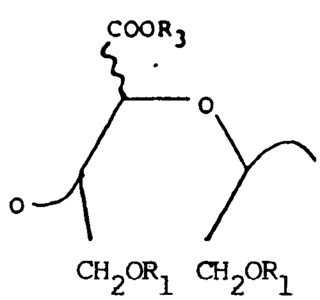

- B représente (a)-$OR_1$, ou $OR_1$, ou un groupe (a) auquel reste accroché un résidu de (c) ou de (d) tel que présent après oxydation periodique suivie d'une $\beta$-élimination;
- $R_1$ a les significations données dans la formule (I);
- $R_2$ représente $SO_3^-$, ou un radical acétyle, la proportion de $SO_3^-$ étant d'environ au moins 90%;
- $R_3$ représente un atome d'hydrogène ou un cation métallique alcalin ou alcalino-terreux;
- n est un nombre entier de 2 à 18.

Des composés avantageux répondant à la formule VI sont ceux dans lesquels n est un nombre entier de 7 à 15 pour les espèces majoritaires qui les constituent.

Des composés avantageux répondant aux formules V et VI, et en particulier ceux répondant à la formule VI dans lesquels n est un nombre entier de 7 à 15 pour les espèces majoritaires, sont ceux dans lesquels $R_1$ est un radical alcanoyle de 2 à 10 atomes de carbone, avantageusement de 4 à 10, de préférence 4 ou 6 atomes de carbone.

D'autres composés préférés répondant à la formule VI sont des mélanges de composés homogènes au regard de leur masse moléculaire, obtenus par gel-filtration, dans lesquels n est un nombre entier de 2 à 12.

Parmi les composés de structure héparinique dépourvus de site de fixation à l'AT III, et donc dépourvus d'activité anticoagulante, une famille de composés avantageux correspond à des dérivés d'héparine N-désulfatée N-acétylée sélectivement O-acylés répondant à la formule VII suivante :

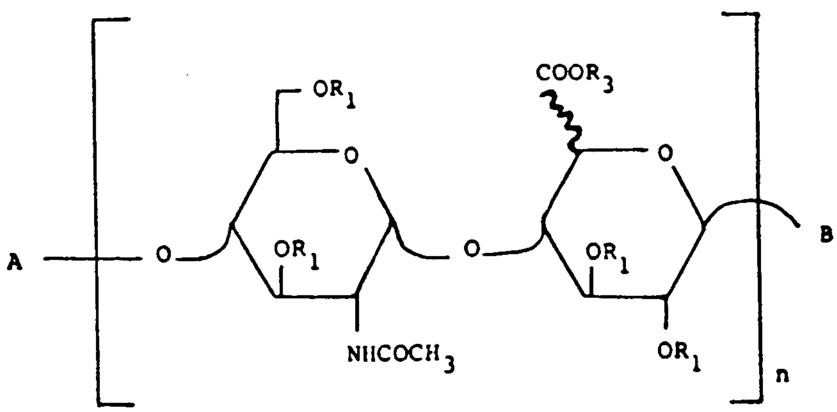

(VII)

dans laquelle :

- A représente $R_1$, $R_1$-(c), ou $R_1$-(d), tels que définis dans la formule (I);
- $R_1$ représente un radical alcanoyle de 2 à 18 atomes de carbone;
- $R_3$ représente un atome d'hydrogène ou un cation métallique alcalin ou alcalino-terreux;
- B représente (a)-$OR_1$, tel que défini dans la formule (I), ou $OR_1$;
- n est un nombre entier de 1 à 80.

Dans un autre aspect avantageux de l'invention, les glycosaminoglycanes choisis sont de type chondroïtine-sulfate, à savoir les chondroïtines 4- et 6-sulfate, les dermatane sulfate, et leurs fragments.

Les glycosaminoglycanes de ce type, sélectivement O-acylés selon l'invention sont représentés par la formule VIII suivante :

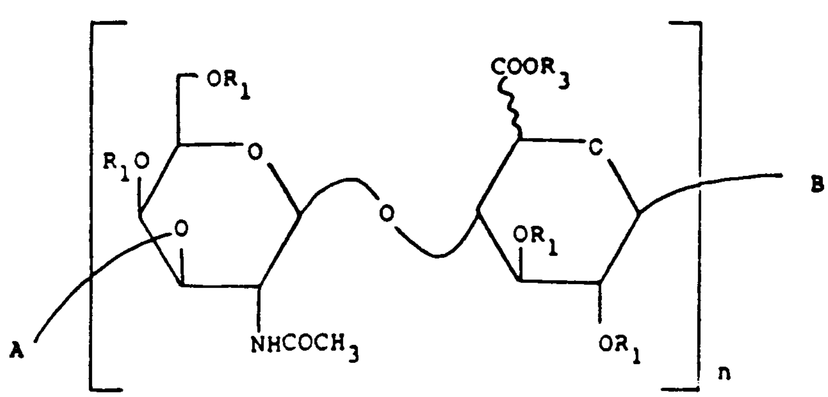

(VIII)

dans laquelle :

- A a les significations de la formule (I);
- $R_1$ représente H et/ou $SO_3^-$ et/ou un groupe acyle tel que défini dans la formule (I) ;

- $R_3$ représente un atome d'hydrogène, un radical alkyle de 1 à 10 atomes de carbone, de préférence de 1 à 4 atomes de carbone, ou un radical phényl-alkyle de 7 à 12 atomes de carbone, ou un cation métallique alcalin ou alcalino-terreux;
- B représente (b)-$OR_1$ ou (g), tels que définis dans la formule (I), ou $OR_1$, ou un groupe (b) auquel reste accroché un résidu de (c) ou de (d) tel que présent après oxydation periodique suivie d'une $\beta$-élimination ou d'une hydrolyse acide ;
- n est un nombre entier de 1 à 80;

Parmi les composés de formule (VIII), une famille avantageuse correspond aux composés dans lesquels $R_3$ représente un atome d'hydrogène ou un cation métallique alcalin ou alcalino-terreux.

D'autres composés avantageux de formule (VIII) sont ceux dans lesquels $R_3$ représente un radical alkyle de 1 à 10 atomes de carbone, de préférence de 1 à 4 atomes de carbones, ou un radical phényl-alkyle de 7 à 12 atomes de carbone.

Une autre famille avantageuse de composé de formule (VIII) correspond aux composés dans lesquels $R_1$ est un radical alcanoyle de 4 à 10 atomes de carbone.

L'invention a également pour objet un procédé de préparation des glycosaminoglycanes sélectivement O-acylés.

Les procédé de la présente invention est caractérisé en ce que l'on transforme le glycosaminoglycane en un sel soluble en milieu organique et en ce que l'on traite ce sel par un agent d'acylation capable d'acyler sélectivement les groupements hydroxyles primaires et secondaires de ce glycosaminoglycane, sans altérer les groupements fonctionnels $NHR_2$, ou $COOR_3$ présents sur ce glycosaminoglycane avant la réaction d'acylation, la réaction s'effectuant dans un solvent aprotique polaire en présence d'un catalyseur et en présence d'une base susceptible de capter l'acide libéré lors de l'acylation, à une température de 0 °C à 100 °C.

Le produit est alors précipité à l'aide d'un solvant miscible à l'eau tel que l'éthanol auquel on peut ajouter un adjuvant favorisant la précipitation tel qu'un sel minéral. Le glycosaminoglycane O-acylé est isolé par dissolution dans l'eau du précipité et avantageusement dialysé en présence d'une base faible.

D'une manière avantageuse, le procédé de l'invention est mis en oeuvre selon les étapes suivantes :

**(1)** on transforme un glycosaminoglycane de formule IX suivante :

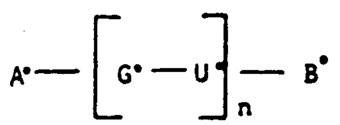

(IX)

dans laquelle :

- G° représente un groupe (a)° de formule :

(a)°

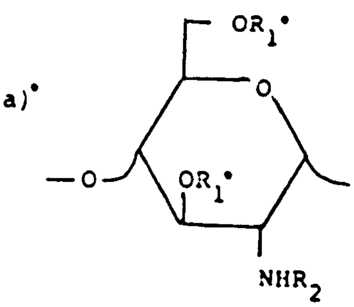

ou un groupe (a')° de formule :

(a')°

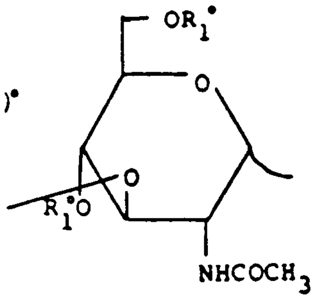

ou un groupe (b)° de formule :

(b)°

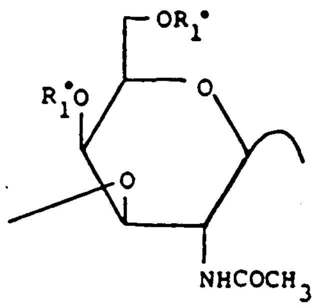

- U° représente un groupe (c)° de formule:

(c)°

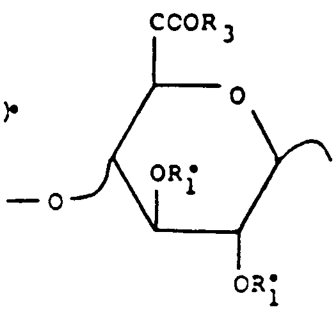

ou un groupe (d)° de formule :

(d)°

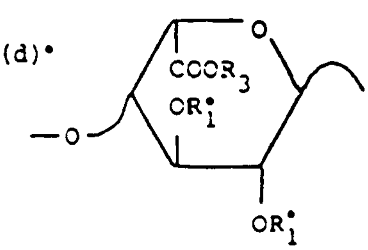

ou le résidu du groupe (c)° ou du groupe (d)° après oxydation periodique suivie d'une $\beta$-élimination ou d'une hydrolyse acide;

- A° représente un groupe $R_1$°, un groupe $R_1$°-(c)°, un groupe $R_1$°-(d)°, ou un groupe (e)° de formule :

(e)°

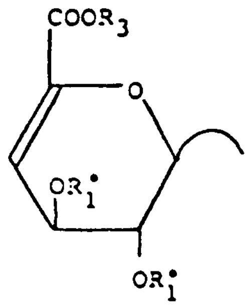

ou le résidu du groupe (c)° ou du groupe (d)° après oxydation periodique suivie d'une $\beta$-élimination ou d'une hydrolyse acide;

- B° représente un groupe $OR_1$°, un groupe (a)°-$OR_1$°, un groupe (a')-$OR_1$°, un groupe (b)°-$OR_1$°, ou un groupe (f)° de formule :

(f)°

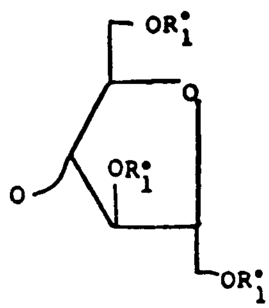

ou un groupe (g)° de formule :

(g)°

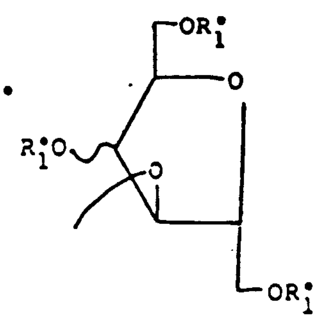

ou représente les groupes (a)°, (a')°, ou (b)° auxquels reste accroché un résidu de (c)° ou de (d)-° tel que présent après oxydation periodique suivie d'une $\beta$-élimination ou d'une hydrolyse acide;

- $R_1$° représente H ou $SO_3^-$ ;
- $R_2$ représente $SO_3^-$ , ou un radical acétyle, sous réserve que la proportion de N-acétyl glucosamine soit au plus égale à celle de l'héparine lorsque $R_1$ représente un radical acétyle;
- $R_3$ représente un atome d'hydrogène, ou un radical alkyle de 1 à 10 atomes de carbone ou un radical phényl-alkyle de 7 à 12 atomes de carbone ou un cation métallique alcalin ou alcalino-terreux;
- n est un nombre entier de 1 à 80

en un sol dudit glycosaminoglycane soluble dans un solvant organique aprotique polaire;

**(2)** on traite ce sel par un anhydride de formule :

Acyle - O - Acyle

dans laquelle Acyle est tel que défini dans la formule (I), dans ledit solvant organique aprotique polaire, en présence de quantités catalytiques de pyridine ou d'une dialkylaminopyridine et d'un accepteur de protons;

**(3)** on précipite le produit ainsi obtenu par action d'une solution d'acétate de sodium dans l'éthanol; et

**(4)** on isole le glycosaminoglycane sélectivement O-acylé par dissolution dans l'eau du précipité ainsi obtenu et par dialyse en présence d'une base faible, et l'on transforme éventuellement le sel de sodium du glycosaminoglycane sélectivement O-acylé ainsi obtenu en un autre sel pharmaceutiquement acceptable.

Dans un aspect avantageux du procédé de l'invention, le glycosaminoglycane mis en oeuvre dans l'étape (1) est choisi dans le groupe constitué par l'héparine, un mélange de fragments d'héparine ayant une masse moléculaire inférieure à 10.000 daltons, un mélange de fragments d'héparine ayant une masse moléculaire moyenne se situant entre 2.000 et 7.000 daltons, un mélange de fragments d'héparine ayant une masse moléculaire moyenne d'environ 4.500 daltons, un mélange de fragments d'héparine ayant une masse moléculaire moyenne d'environ 2.500 daltons, un mélange de fragments d'héparine homogènes au regard de leur masse moléculaire, un fragment d'héparine obtenu par synthèse, homogène tant en ce qui concerne sa masse moléculaire qu'en ce qui concerne sa fonctionnalisation.

Dans un autre aspect avantageux du procédé de l'invention, le glycosaminoglycane mis en oeuvre dans l'étape (1) est de l'héparine, une fraction ou un fragment d'héparine dépourvus de site de liaison à l'antithrombine III.

Le glycosaminoglycane mis en oeuvre dans l'étape (1) du procédé de l'invention peut encore avantageusement être choisi dans le groupe constitué par le dermatane sulfate et ses fragments, ou les chondroïtines 4- et 6-sulfate et leurs fragments.

D'une manière avantageuse, le sel de glycosaminoglycane mis en oeuvre dans l'étape (1) du procédé de l'invention est un sel d'amine tertiaire, en particulier un sel de tributylammonium, ou un sel d'ammonium quaternaire, en particulier un sel de tétrabutylammonium.

Dans un autre aspect avantageux du procédé de l'invention, l'anhydride mis en oeuvre dans l'étape (2) est l'anhydride d'un acide alcanoïque contenant de 2 à 10 atomes de carbone, avantageusement de 4 à 10, de préférence 4 ou 6 atomes de carbone.

Le solvant aprotique polaire dans lequel est conduite l'étape (2) du procédé de l'invention peut être avantageusement choisi parmi le groupe constitué par le diméthylformamide, l'hexaméthylphosphorotriamide, la pyridine, ou encore un mélange de ces solvants entre eux ou avec le dichlorométhane, et le catalyseur parmi le groupe constitué par des amines telles que la pyridine, et la diméthylaminopyridine.

La base destinée à neutraliser l'acidité peut être la pyridine (qui peut servir à la fois de solvant, de catalyseur et de base), la triéthylamine ou la tributylamine.

De manière avantageuse, la dialyse effectuée dans l'étape (4) se fait en présence d'une base faible telle que le bicarbonate de sodium afin d'éliminer d'éventuels produits secondaires tels que des anhydrides.

De manière avantageuse, la température et la durée de la réaction peuvent varier, en fonction du taux d'acylation souhaité, respectivement de 0°C à 100°C, en particulier de 0°C à 50°C, et avantageusement à température ambiante, pendant 1 à 24 heures par example.

Les composés de l'invention, sélectivement O-acylés, présentent in vivo une activité prolongée. Par exemple, lorsque ces composés sont des composés de type héparinique possédant le site de liaison à l'AT III, et sont donc susceptibles d'exercer une activité antithrombotique, cette activité est nettement prolongée dans le temps par rapport au même composé n'ayant pas subi une O-acylation sélective.

Les dérivés hépariniques sélectivement O-acylés selon l'invention, posédant ou non le site de liaison à l'AT III, ces derniers étant pratiquement dépourvus d'activité anticoagulante présentent également diverses activités biologiques, notamment :

- l'inhibition de la prolifération des cellules musculaires lisses, qui présente un grand intérêt pour éviter les resténoses dans les interventions telles que l'angioplastie, les pontages et greffes veineux et artériels, les transplantations d'organes, en particulier les transplantations cardiaques,
- l'inhibition de l'héparanase et de l'héparitinase, enzymes impliquées dans les disséminations métastasiques,
- des propriétés anti-virales, en particulier vis-à-vis des rétro-virus, notamment vis-à-vis des différents HV (virus de l'immunodéficience humaine) ce qui présente un grand intérêt dans le traitement du SIDA,
- des propriétés d'inhibition de l'élastase leucocytaire, d'augmentation des taux circulants des inhibiteurs d'élastase ainsi que d'inhibition sélective de la synthèse du collagène de type III et de la fibronectine, ce qui leur confère un grand intérêt pour le traitement des maladies dans lesquelles un

déséquilibre du système élastase-anti-élastase est impliqué, telles que l'emphysème, ainsi que pour le traitement des maladies dégénératives du tissu conjonctif telles que l'artériosclérose et le diabète.

Les glycosaminoglycanes sélectivement O-acylés de l'invention peuvent donc constituer le principe actif de médicaments de grand intérêt dans de nombreuses indications.

L'invention a donc également pour objet des compositions pharmaceutiques caractérisées en ce qu'elles renferment à titre de substance active une quantité efficace d'au moins un glycosaminoglycane O-acylé selon l'invention, en association avec un véhicule pharmaceutiquement acceptable.

De manière avantageuse, le glycosaminoglycane sélectivement O-acylé se trouve sous forme d'un sel pharmaceutiquement acceptable, tel qu'un sel de sodium de magnésium ou de calcium.

Dans une réalisation avantageuse de l'invention, les compositions pharmaceutiques sont caractérisées en ce que le véhicule pharmaceutique est approprié pour l'administration par voie orale, et qu'elles se présentent sous forme de gélules gastrorésistantes, de comprimés ou tablettes, de pilules, ou encore sous forme de solutions buvables, renfermant avantageusement de 50 mg à 5 g par unité de prise, de préférence de 100 à 1000 mg pour des gélules, tablettes ou pilules, et de 10 à 150 mg pour les solutés buvables, par exemple une à trois fois par jour ; ou encore en ce que ces compositions se présentent sous forme de solution injectable, stérile ou stérilisable, pour l'administration par voie intraveineuse, intramusculaire ou sous-cutanée, ces solutions renfermant avantageusement de 50 à 200 mg/ml de glycosaminoglycane sélectivement O-acylé lorsqu'elles sont destinées à l'injection par voie sous-cutanée ou de 20 à 200 mg/ml de glycosaminoglycane sélectivement O-acylé lorsqu'elles sont dstinées à l'injection par voie intraveineuse ou par perfusion.

Ces intervalles de doses n'ont qu'une valeur indicative, les doses administrées devant, dans chaque cas, être évaluées par le clinicien, compte tenu de l'état du malade et de sa réactivité personnelle à l'égard des médicaments.

L'invention concerne également à titre de réactif biologique les glycosaminoglycanes sélectivement O-acylés de l'invention, qui peuvent être utilisés comme références ou étalons dans des études comparatives pour des études de relations structure/activité dans différents systèmes physiologiques dans lesquels les glycosaminoglycanes peuvent être impliqués.

L'invention sera mieux comprise à l'aide des exemples d'application qui suivent, et qui ne possèdent aucun caractère limitatif.

***EXEMPLE1* : *Préparation d'héparine O–acétylée (IC 1938) à partir du sel de tétrabutylammonium de l'héparine***

*a) Préparation du sel de tétrabutylammonium de l'héparine*:

Le sel de sodium de l'héparine (10 g) dissous dans l'eau (500 ml) est percolé au travers d'une colonne de résine échangeuse de cations (Dowex 50 W x 4, sous forme $H^+$). La solution obtenue est neutralisée par de l'hydroxyde de tétrabutylammonium. Après lyophilisation on obtient le sel de tétrabutylammonium de l'héparine (19,55 g).

*b) Acétylation* :

1,05 g d'héparinate de tétrabutylammonium est dissous dans du diméthylformamide anhydre (5 ml). Après refroidissement à 0° C, on ajoute goutte à goutte de l'anhydride acétique (1 ml ; 10,46 mmoles) puis de la triéthylamine (1,45 ml ; 10,46 mmoles) et de la diméthylaminopyridine (64 mg ; 0,5 mmole). Le mélange est abandonné 24 heures à température ambiante. Après addition d'eau (5 ml), la solution est dialysée pendant 72 heures contre de l'eau distillée. Le sel de tétrabutylammonium est converti en sel de sodium par passage sur une résine Dowex 50, $H^+$ à 0° C, suivie de neutralisation par de la soude 1N. Après lyophilisation, on obtient 0,49 g d'héparinate de sodium sélectivement 0-acétylé présentant les caractéristiques suivantes:

Rapport sulfate/carboxyle : 2,28 meq/g
(produit de départ : 2,20 meq/g)
Titre APTT : 91 ui/mg

Spectre $^{13}C$ RMN (méthanol 51,6 ppm, standard interne)

- signal à 23,4 ppm : $CH_3$ de $CH_3$-CO-O-
- signal à 24,5 ppm (faible : $CH_3$ de $CH_3$-CO-NH-
  (identique au produit de départ)

Le spectre RMN indique la présence d'environ deux groupes acétyles par unité disaccharidique.

***EXEMPLE 2** : **Préparation d'héparine O−acétylée (IC 1938) à partir du sel de tributylammonium de l'héparine***

*a) Préparation du sel de tributylammonium de l'héparine*:

Le sel de sodium de l'héparine (10 g) est dissous dans l'eau (500 ml) puis percolé au travers d'une colonne de résine échangeuse de cations (Dowex 50 W x 4, sous forme $H^+$). La solution est neutralisée par addition d'une solution de tributylamine à 10 % dans l'éthanol. Après lavage à l'éther et lyophilisation, on obtient le sel de tributylammonium de l'héparine (14,77 g).

*b) Acétylation* :

A une solution refroidie à O° C du sel ci-dessus (4 g) dans du diméthylformamide anhydre (50 ml), on ajoute de la diméthylaminopyridine (250 mg), de l'anhydride acétique (3,9 ml) et de la tributylamine (9,7 ml). Après 24 heures à température ambiante, de l'eau (1,5 ml) est ajoutée. Le mélange est ensuite versé dans une solution d'éthanol saturé en acétate de sodium. Après lavage par l'éthanol, le précipité est dissous dans l'eau, puis dialysé contre du bicarbonate à 5 % dans l'eau, puis contre de l'eau. On obtient, après lyophilisation, l'héparinate de sodium 0-acétylé (1,81 g).

Le spectre infra-rouge présente une forte bande ester à 1730 $cm^{-1}$.

Après saponification, le produit présente :

- un rapport sulfate/carboxyle de 2,38 meq/g (produit de départ: 2,40 meq/g)
- un titre APTT de : 85 ui/mg

***EXEMPLE 3** : **Préparation d'héparine 0−acétylée (IC 1938) en utilisant la catalyse par la pyridine***

Le sel de tétrabutylammonium de l'héparine (0,58 g) est dissous dans un mélange (1/1 ; v/v) de pyridine et de diméthylformamide (10 ml). De l'anhydride acétique est ajouté (0,5 ml), puis le mélange est abandonné à temperature ambiante. Après 24 heures, on ajoute une solution aqueuse d'acétate de sodium (1 M, 15 ml) puis on verse le mélange dans de l'éthanol (80 ml) refroidi à 0° C. Après centrifugation le précipité est redissous dans l'eau (10 ml), puis de l'éthanol est ajouté (160 ml) suivi d'acétate de sodium aqueux (1 M, 10 ml). Le précipité est ensuite repris par l'eau et lyophilisé, donnant l'héparine O-acétylée (0,22 g).

***EXEMPLE 4** : **Préparation d'héparine O−propionylée (IC 1939) à des degrés divers***

A une solution, refroidie à 0° C, d'héparinate de tétrabutylammonium (1,85 g) obtenu comme décrit dans l'exemple 1, dans le diméthylformamide (10 ml), on ajoute goutte à goutte de l'anhydride propionique (2,7 ml), puis de la triéthylamine (2,9 ml) et de la diméthylaminopyridine (128 mg). Aux temps 1 h, 2 h, 4 h, 8 h et 24 h, une partie du mélange réactionnel est prélevée, diluée par un égal volume d'eau et dialysée pendant 24 heures dans de l'eau distillée. Après passage sur une résine Dowex 50 $H^+$ et neutralisation par la soude, les produits obtenus sont lyophilisés et présentent les caractéristiques suivantes :

| Temps de réaction | Masse | Sulfate/Carboxyle meq/g | APTT (ui/mg) |
|---|---|---|---|
| 24 h | 496 mg | 2,31 | 80 |
| 8 h | 138 mg | 2,35 | 88 |
| 4 h | 122 mg | 2,33 | 94 |
| 2 h | 120 mg | 2,42 | 109 |
| 1 h | 120 mg | 2,33 | 120 |
| (produit de départ) | | 2,40 | 150 |

Le spectre RMN du proton des produits a été enregistré dans l'eau avec le 3,3,3-triméthylsilylpropionate (TSP) comme standard interne. Il présente des signaux à ~ 1,1 ppm et ~ 2,4 ppm caractéristiques respectivement des résidus $CH_3$ et $CH_2$ de $CH_3$-$CH_2$-CO-O et des signaux entre ~ 3 et ~ 5 ppm caractéristiques des protons du squelette osidique.

La comparaison de l'intensité des signaux du propionyl et du squelette entre le produit traité pendant 1 heure et celui soumis à 24 heures de réaction illustre l'influence de la durée de la réaction : en effet, on observe une décroissance des signaux des protons du squelette, ce qui indique une augmentation du taux

de substitution.

Le spectre du carbone (méthanol 51,6 ppm, standard interne) des signaux à ~ 10,8 et ~ 30 ppm, caractéristiques des radicaux $CH_3$ et $CH_2$ des esters propionates.

***EXEMPLE 5 : Préparation d'héparine O-butyrylée (IC 1940)***

*A) Utilisation du sel de tétrabutylammonium de l'héparine* :

A une solution du sel de tétrabutylammonium de l'héparine (0,55 g) obtenu comme décrit dans l'exemple 1, dans le diméthylformamide (5 ml) on ajoute, à 0° C de l'anhydride butyrique et de la diméthylaminopyridine. Après 24 heures à température ambiante, de l'eau (5 ml) est ajoutée, puis le mélange réactionnel est dialysé pendant 72 heures contre de l'eau distillée. Après échange sur Dowex 50 $H^+$, neutralisation par la soude et lyophilisation, on obtient de l'héparine 0-butyrylée sous forme de sel de sodium (0,32 g).

Rapport sulfate/carboxyle : 2,15 meq/g
(produit de départ : 2,20 meq/g)
Titre APTT : 59 ui/mg

Le spectre de RMN du proton (TSP, standard interne) montre la présence de signaux à 0,9 ppm ; 1,6 ppm et 2,4 ppm, caractéristiques des groupements $CH_3$-$CH_2$ de $CH_3$-$CH_2$-$CH_2$-CO-O- et des signaux entre 3 et 6 ppm, caractéristiques du squelette osidique.

L'analyse du spectre RMN indique la présence d'environ une chaîne butyryle par unité disaccharidique.

*B) Utilisation du sel de tributylammonium de l'héparine*:

A une solution, refroidie à 0° C, d'héparinate de tributylamine (4 g) obtenu comme décrit dans l'exemple 2 dans le diméthylformamide (50 ml), on ajoute de la diméthylaminopyridine (0,25 g), de l'anhydride butyrique (6,7 ml) et de la tributylamine (9,7 ml). On laisse le mélange réactionnel à température ambiante pendant 24 heures. De l'eau est ajoutée (1,5 ml) puis, après 30 minutes, une solution saturée d'acétate de sodium dans l'éthanol (250 ml). Le précipté est ensuite lavé trois fois à l'éthanol, puis dialysé contre une solution à 5 % de bicarbonate, puis contre de l'eau. On obtient, après lyophilisation, le sel de sodium de l'héparine O-butyrylée (2,1 g).

Titre APTT : 29 ui/mg

Après saponification des esters par la soude 0,5 M pendant 2 heures à 0° C, le produit obtenu présente un rapport sulfate/carboxyle de 2,36 meq/g (2,40 meq/g dans le produit de départ).

***EXEMPLE 6 : Préparation d'héparine O-hexanoylée (IC 1941)***

*A) Utilisation du sel de tétrabutylammonium de l'héparine* :

A une solution de sel de tétrabutylammonium de l'héparine (0,55 g) obtenu comme décrit dans l'exemple 1, dans le diméthylformamide (5 ml), on ajoute à 0° C de l'anhydride caproïque (1 ml ; 6 mmoles), de la triéthylamine (0,84 ml, 6 mmoles) et de la diméthylaminopyridine. Après 24 heures à température ambiante, de l'eau (5 ml) est ajoutée, puis le mélange réactionnel est dialysé pendant 72 heures contre une solution de bicarbonate à 5 %, puis contre de l'eau distillée. Après échange sur Dowex 50 $H^+$, neutralisation par la soude et lyophilisation, on obtient l'héparine O-hexanoylée sous forme de sel de sodium (0,30 g).

Titre APTT : 25 ui/mg

Le spectre RMN du proton (TSP, standard interne) présente des signaux à 0,8 ; 1,2 ; 1,5 ; 2,3 ppm, caractéristiques des $CH_3$-$CH_2$ de $CH_3$-$(CH_2)_8$-CO-O-

*B) Utilisation du sel de tributylammonium de l'héparine*:

A une solution, refroidie à 0° C, d'héparinate de tributylammonium (4 g) obtenu comme décrit dans l'exemple 2 dans le diméthylformamide (50 ml), on ajoute de la diméthylaminopyridine (0,25 g), de la tributylamine (9,7 ml) et de l'anhydride hexanoïque (10,6 ml). Après 24 heures à 20° C, de l'eau est ajoutée (1,5 ml), suivie d'une solution saturée d'acétate de sodium dans l'éthanol. Après lavage à l'éthanol, dialyse et lyophilisation, on obtient l'héparine O-hexanoylée (2,5 g).

***EXEMPLE 7 : préparation d'héparine o−octanoylée (IC 1942)***

A une solution, refroidie à 0° C, d'héparinate de tributylammonium (4 g) obtenu comme décrit dans l'exemple 2 dans le diméthylformamide (50 ml), on ajoute de la diméthylaminopyridine (0,25 g), de l'anhydride octanoïque (12,1 ml) et de la tributylamine (9,7 ml). Après 24 heures à température ambiante, de l'eau (1,5 ml) est ajoutée suivie, après 30 minutes, d'une solution saturée d'acétate de sodium dans l'éthanol. Après dialyse contre une solution à 20 % d'éthanol, puis contre l'eau distillée et ultrafiltration, le produit est soumis à un échange de cations par passage sur Dowex 50 $H^+$, suivi de neutralisation par la soude. Après lyophilisation, on obtient l'héparine 0-octanoylée (2,5 g).

***EXEMPLE 8 : PREPARATION D'HEPARINE O−DECANOYLEE (IC 1943)***

***A) UTILISATION DU SEL DE TETRABUTYLAMMONIUM DE L'HEPARINE:***

A une solution du sel de tétrabutylammonium de l'héparine (O,55 g) obtenu comme décrit dans l'exemple 1 dans le diméthylformamide (5 ml), on ajoute à 0° C de l'anhydride caprique (1 ml ; 6 mmoles) de la triéthylamine (0,84 ml, 6 mmoles) et de la diméthylaminopyridine. Après 24 heures à température ambiante, de l'eau (5 ml) est ajoutée, puis le mélange réactionnel est dialysé pendant 72 heures contre une solution de bicarbonate à 5 %, puis contre de l'eau distillée. Après échange sur Dowex 50 $H^+$, neutralisation par la soude et lyophilisation, on obtient l'héparine O-décanoylée sous forme de sel de sodium (0,30 g).

Le spectre RMN du proton (TSP, standard interne) présente des signaux à 0,8 ; 1,2 ; 1,5 et 2,4 ppm, caractéristiques des $CH_3$ et $CH_2$ de $CH_3$-$(CH_2)_8$-CO-O-

***B) UTILISATION DU SEL DE TRIBUTYLAMMONIUM DE L'HEPARINE***

A une solution, refroidie à 0° C, d'héparinate de tributylammonium (4 g) obtenu comme décrit dans l'exemple 2 dans le diméthylformamide (50 ml), on ajoute de la diméthylaminopyridine (0,25 g), de l'anhydride décanoïque (13,3 g dissous dans 20 ml de diméthylformamide), et de la tributylamine (9,7 ml). Après 24 heures à température ambiente, de l'eau (1,5 ml) est ajoutée, suivie d'une solution saturée d'acétate de sodium dans l'éthanol. Le précipité est dissous dans du diméthylsulfoxyde puis dialysé contre de l'eau, du bicarbonate de sodium et à nouveau de l'eau.
Après lyophilisation, on obtient l'héparine O-décanoylée (2,38 g).

***EXEMPLE 9 : PREPARATION D'HEPARINE O−OLEOYLEE (IC 2013)***

Le sel de tributylammonium de l'héparine (3 g) et de la N,N-diméthylaminopyridine (244 mg) sont dissous dans du diméthylformamide anhydre (50 ml). Après refroidissement à 0°C, on ajoute, goutte à goutte, de l'anhydride oléique (21 g) synthétisé selon Plusquellec et al, Tetrahedron, 1988, 44, 2471-2476, en solution dans le dichlorométhane (20 ml), puis de la tributylamine (9,5 ml). Après 24 heures de réaction et refroidissement à 0°C, on introduit du bicarbonate de sodium à 5% (10ml) puis, une heure après, une solution alcoolique saturée d'acétate de sodium. Après lavage à l'éthanol absolu, solubilisation dans un mélange diméthylsulfoxyde-eau (4/1; v/v, 750 ml), on dialyse contre de l'éthanol aqueux à 10% pendant 2 jours, puis de l'eau pendant 3 jours. Le sel de sodium de l'héparine oléoylée est isolé, après lyophilisation et précipitation dans l'éther éthylique du lyophilisat resolubilisé dans le DMF (2,77 g). Teneur en acide oléique : 1,44μmole/mg (dosage selon Ducombe W. et al, Biochemical Journal, 1963, 88, 7).

***EXEMPLE 10 : PREPARATION D'HEPARINE O−BENZOYLEE (IC 1944)***

Le sel de tétrabutylammonium de l'héparine, obtenu comme décrit dans l'exemple 2, est benzoylé par l'anhydride benzoïque dans les conditions décrites précédemment pour la préparation de l'héparine O-décanoylée (exemple 8).

Le spectre RMN du carbone du produit obtenu présente des signaux à 131, 132 et 136 ppm caractéristiques des groupements benzoyles.

Le produit est dépourvu d'activité anticoagulante in vitro.

***EXEMPLE 11 : PREPARATION D'HEPARINE O-3-CYCLOPENTYL PROPIONYLEE (IC 2014)***

L'anhydride 3-cyclopentylpropionique est préparé selon la méthode de Plusquellec et al, Tetrahedron, 1988, 44, 2471-2476. Celui-ci est obtenu après addition de l'acide (23 ml, 150 mmoles), en solution dans le dichlorométhane (400 ml), au mélange, agité et refroidi à -10°C, contenant du bromure de tétrabutylammonium (15 mmoles), de la soude 20% (60 ml) et du dichlorométhane (80 ml), et ceci après décantation, lavages au bicarbonate de sodium 5% et à l'eau, et concentration en une huile (96%).
Fréquences caractéristiques en infra-rouge : 1800, 1745, 1040 $cm^{-1}$.

Le sel de tributylammonium de l'héparine (4g) et de la N,N-diméthylaminopyridine (253 mg) sont dissous dans le diméthylformamide anhydre (40 ml). Après refroidissement à 0°C on ajoute, goutte à goutte, de l'anhydride-3-cyclopentylpropionique (11g), puis de la tributylamine (9,8 ml). Après 24 heures de réaction à température ambiante, puis refroidissement 0°C, de l'eau (1 ml) est ajoutée puis une heure après, une solution alcoolique saturée d'acétate de sodium. Après lavage à l'éthanol absolu, le précipité est dialysé contre du bicarbonate de sodium à 5% pendant 24 heures, puis contre de l'eau pendant 3 jours. Après lyophilisation on obtient l'héparine O-3-cyclopentylpropionylée sous forme de son sel de sodium (2,64g).

Le spectre de RMN du carbone dans $D_2O$ (méthanol à 51,6 ppm, standard interne) présente les signaux caractéristiques à 27,4 ppm; 33,1 ppm; 34,6 ppm; 35,9 ppm et 41,7 ppm du groupement O-cyclopentylpropionyle.

Rapport sulfate/Carboxyle : 2,2

***EXEMPLE 12 : PREPARATION D'UNE HEPARINE DE FAIBLE MASSE MOLECULAIRE (PM MOYEN ~ 4.500 DALTONS, INTERVALLE DE PM ~ 1.800-8.000 DALTONS) O-ACETYLEE (IC 1945)***

Cette héparine de faible masse moléculaire a été obtenue par dépolymérisation nitreuse partielle et fractionnement alcoolique comme décrit dans le brevet EP 181 252 et est dénommée ci-après CY 216.

*A) Utilisation du sel de tetrabutylammonium du CY 216*

Le sel de sodium du CY 216 (1 g) est converti en sel de tétrabutylammonium par passage au travers d'une colonne de résine Dowex 50 $H^+$ suivi de neutralisation par l'hydroxyde de tétrabutylammonium.

Le sel ainsi obtenu (1,7 g) est séché sous vide pendant trois heures à 50° C puis dissous dans du diméthylformamide anhydre (10 ml). Après refroidissment à 0° C, de l'anhydride acétique (1,7 ml) est ajouté goutte à goutte, suivi de triéthylamine (2,4 ml) et de diméthylaminopyridine (102 mg). Après 20 heures de réaction, le produit est chromatographié sur une colonne de Séphadex G-25 éluée par l'eau. On obtient, après conversion en sel de sodium et lyophilisation, le CY 216 O-acétylé (O,89 g).

Son spectre de RMN du carbone (méthanol 51,6 ppm, standard interne) présente un signal à 23 ppm caractéristique des acétates.

Le signal du $CH_3$ de $CH_3$-CO-NH-, à ~ 24,5 ppm, est identique à celui du produit de départ.

Le rapport sulfate /carboxyle est de 2.09 meq/g (produit de départ : 2,05 meq/g)

- Titre APTT : 18 ui/mg
- Titre anti-Xa : 205 u/mg (Dosage de Yin et al., J. Lab. Clin. Med., 1973, 81, 298-310)

*B) Utilisation du sel de tributylammonium du CY 216*

Le sel de sodium du CY 216 est converti en sel de tributylammonium par passage au travers d'une colonne de résine Dowex 50 $H^-$, suivi de neutralisation par la tributylamine, comme décrit pour l'héparine. Le sel de tributylammonium du CY 216 est obtenu après lavage à l'éther, lyophilisation et séchage à l'étuve sous vide.

Le sel ci-dessus (4 g) et de la N,N-diméthylaminopyridine (288 mg) sont dissous dans le diméthylformamide. Après refroidissement à 0°C, de l'anhydride acétique (4,4 ml) est ajouté goutte à goutte, suivi de tributylamine (11,2 ml). Après 24 heures à température ambiante et refroidissement à 0°C de l'eau est ajoutée (1,7 ml) suivie, après 1 heure, d'une solution alcoolique saturée d'acétate de sodium. Le précipité est lavé à l'éthanol, solubilisé dans l'eau apyrogène, dialysé pendant 36 heures contre du bicarbonate à 5%, puis contre de l'eau pendant 3 jours. On obtient, après lyophylisation, le CY 216 acétylé sous forme sel de sodium (1,4 g).

Le spectre RMN du carbone dans $D_2O$ (méthanol à 51,6 ppm comme standard interne) présente à 23 ppm le signal caractéristique du groupement acétylé.

Rapport sulfate/carboxyle : 2,07.

***EXEMPLE 13 : PREPARATION D'UNE HEPARINE DE FAIBLE MASSE MOLECULAIRE (PM MOYEN 4.500 DALTONS, INTERVALLE DE PM ~ 1.800−8.000 DALTONS) O−BUTYRYLEE (IC 1957 )***

Le sel de tributylammonium du CY 216 (4 g) obtenu comme décrit dans l'exemple 12 et de la N,N-diméthylaminopyridine (288 g) sont dissous dans du diméthylformamide anhydre (40 ml); après refroidissement à 0 ° C de l'anhydride butyrique (7,68 ml) est ajouté, goutte à goutte, suivi de tributylamine (11,2 ml). Après 24 heures de réaction à température ambiante, puis refroidissement à 0 ° C, de l'eau est ajoutée (1,7 ml) suivie, une heure après, d'une solution alcoolique saturée d'acétate de sodium. Après lavage à l'éthanol, solubilisation dans l'eau apyrogène, dialyse contre du bicarbonate de sodium à 5% pendant 36 heures, puis contre l'eau pendant 3 jours, le CY 216-O-butyrylé est isolé sous forme de sel de sodium après lyophylisation (2,14 g).

Le spectre RMN du carbone dans $D_2O$ (méthanol à 51,6 ppm, standard interne) présente à 15,6 ppm; 20,5 ppm et à 39,4 ppm les signaux caractéristiques du groupement O-butyryle.

Rapport sulfate/carboxyle : 2,08.

***EXEMPLE 14 : PREPARATION D'UNE HEPARINE DE FAIBLE MASSE MOLECULAIRE (PM MOYEN ~ 4.500 DALTONS, INTERVALLE DE PM ≃ 1.800−8.000 DALTONS) O−HEXANOYLEE (IC 1958)***

Le sel de tributylammonium du CY 216 (4 g) obtenu comme décrit dans l'exemple 12 et de la N,N-diméthylaminopyridine (288 g) sont dissous dans du diméthylformamide (40 ml). Après refroidissement à 0 ° C, on ajoute, goutte à goutte de l'anhydride caproïque (10,8 ml) et de la tributylamine (11,2 ml). Après 24 heures de réaction à température ambiante, puis refroidissement à 0 ° C, de l'eau est ajoutée (1,7 ml) suivie, une heure après, d'une solution alcoolique saturée d'acétate de sodium. Après lavage à l'éthanol absolu, solubilisation dans l'eau apyrogène, dialyse contre du bicarbonate de sodium à 5% pendant 36 heures, puis contre l'eau pendant 3 jours, le CY 216-O-caproylé est isolé sous forme sel de sodium après lyophylisation (2,5 g).

Le spectre RMN du carbone dans $D_2O$ (méthanol à 51,6 ppm, standard interne) présente à 15,9 ppm; 24,2 ppm; 26,4 ppm; 33,1 ppm et 36,4 ppm les signaux caractéristiques du groupement caproyle.

Rapport sulfate/carboxyle : 2,08.

***EXEMPLE 15 : PREPARATION D'UNE HEPARINE DE FAIBLE MASSE MOLECULAIRE (PM MOYEN ~ 4.500 DALTONS, INTERVALLE DE PM ~ 1.800−8.000 DALTONS) O−OCTANOYLEE (IC 1959)***

Le sel de tributylammonium du CY 216 (4 g) obtenu comme décrit dans l'exemple 12 et de la N,N-diméthylaminopyridine (288 g) sont dissous dans du diméthylformamide (40 ml). Après refroidissement à 0 ° C, on ajoute, goutte à goutte de l'anhydride caprylique (14 ml) puis de la tributylamine (11,2 ml). Après 24 heures de réaction à température ambiante, et refroidissement à 0 ° C, de l'eau est ajoutée (1,7 ml) suivie, une heure après, d'une solution alcoolique saturée d'acétate de sodium. Après lavage à l'éthanol absolu, solubilisation dans l'eau apyrogène, dialyse contre du bicarbonate de sodium à 5% pendant 36 heures, puis contre l'eau pendant 3 jours, on obtient, après lyophylisation, le CY 216-O-octanoylé sous forme sel de sodium (1,76 g).

Le spectre RMN du carbone dans $d^6$ DMSO (méthanol à 51,6 ppm, standard interne) présente des signaux caractéristiques à 16,8 ppm; 25,0 ppm; 27,3 ppm; 30,9 ppm, 31,4, 34,1 et 36,4 ppm du groupement capryloyle.

Rapport sulfate/carboxyle : 2,07.

***EXEMPLE 16 : PREPARATION D'UNE HEPARINE DE FAIBLE MASSE MOLECULAIRE (PM MOYEN ~ 4.500 DALTONS, INTERVALLE DE PM ~ 1.800−8.000 DALTONS) O−DECANOYLEE (IC 1960)***

Le sel de tributylammonium du CY 216 (4 g) obtenu comme décrit dans l'exemple 12 et de la N,N-diméthylaminopyridine (288 g) sont dissous dans du diméthylformamide (40 ml). Après refroidissement à 0 ° C, on ajoute, goutte à goutte de l'anhydride décanoïque (15,3 ml) en solution dans du diméthylformamide anhydre (10 ml), puis de la tributylamine (11,2 ml). Après 24 heures de réaction à température ambiante, et refroidissement à 0 ° C, de l'eau est ajoutée (1,7 ml) suivie, une heure après, d'une solution alcoolique saturée d'acétate de sodium. Après lavage à l'éthanol absolu et remise en suspension dans l'eau apyrogène, on dialyse contre du bicarbonate de sodium à 5% pendant 2 jours, contre du NaCl 10%

pendant 2 jours, puis contre de l'eau pendant 5 jours. Le CY 216-O-décanoylé est isolé sous forme sel de sodium après lyophylisation (2,76 g).

Le spectre RMN du carbone dans $D_2O$ (méthanol à 51,6 ppm, standard interne) présente des signaux caractéristiques à 16,4 ppm; 22,3 ppm; 25,1 ppm; 29,2 ppm; 31,9, 34,4 et 36,5 ppm du groupement décanoyle.

Rapport sulfate/carboxyle : 2,13.

***EXEMPLE 17 : PREPARATION D'UNE HEPARINE DE FAIBLE MASSE MOLECULAIRE (PM MOYEN ~ 2.500 DALTONS, INTERVALLE DE PM ~ 1.500−8.000 DALTONS) O−ACETYLEE (IC 1946)***

Cette héparine de faible masse moléculaire a été obtenue par dépolymérisation nitreuse partielle selon le procédé décrit dans le brevet EP 37 319 et est dénommée ci-après CY 222.

Le sel de sodium du CY 222 est converti en sel de tributylammonium par passage au travers d'une colonne de résine Dowex 50 $H^+$, suivi de neutralisation par la tributylamine.

Le sel obtenu après lyophilisation (1,5 g) est dissous dans du diméthylformamide (5 ml) puis, après refroidissement à 0° C, de l'anhydride acétique (1,35 ml) est ajouté goutte à goutte, suivi de triéthylamine (2 ml) et de diméthylaminopyridine (85 mg). Après 18 heures de réaction, de l'eau est ajoutée (20 ml) puis le mélange est dialysé pendant 3 hours contre de l'eau distillée. Après conversion en sel de sodium et lyophilisation, on obtient le CY 222 O-acétylé (0,86 g).

Le produit présente un rapport sulfate/carboxyle de 1,98 meq/g (produit de départ : 1,97 meq/g).

Le spectre de RMN du carbone (méthanol 51,6 ppm, standard interne) du produit contient un signal à 23 ppm caractéristique des O-acétyl. La comparaison des intensités des signaux des N-acétyl (à ~ 24,5 ppm) entre le produit de départ et le produit d'arrivée démontre que l'acétylation a été sélective.

- Titre APTT : 8 ui/mg
- Titre anti-Xa : 191 u/mg (Dosage de Yin et al., J. Lab. Clin. Med., 1973, 81, 298-310)

***EXEMPLE 18 : A) PREPARATION D'UN FRAGMENT D'HEPARINE DEPOURVU D'AFFINITE POUR L'ANTITHROMBINE III (IC 1772)***

*1/ Coupure des chaînes d'héparine à l'aide d'acide* periodique :

10 g d'héparine injectable de mucus de porc, sous forme de sel de sodium, titrant 157 ui/mg dans le dosage Codex et 155 u/mg dans le dosage anti-facteur Xa de Yin et al. sont mis en solution dans 250 ml d'eau déminéralisée à 4° C. Le pH de la solution est ajusté à 5,0 par de l'acide chlorhydrique concentré. 10 g de métaperiodate de sodium ($NaIO_4$, PM : 213,89) en solution dans 250 ml d'eau déminéralisée à 4° C sont ajoutés sous agitation modérée. Le pH de l'ensemble est ajusté à 5,0 par de l'acide chlorhydrique concentré. La solution est abandonnée 24 heures, à l'obscurité, en chambre froide à + 4° C.

*2/ Elimination du periodate résiduel* :

On répartit ensuite la solution réactionnelle dans trois boyaux de dialyse NOJAX 40$^R$, (porosité de 3 à 4.000 Da) et on la soumet à une dialyse 15 heures contre de l'eau déminéralisée courante.

*3/ Dépolymérisation en milieu basique* :

Aux 780 ml de solution obtenue après dialyse, sont ajoutés 16 ml de soude 10 N, et l'ensemble est soumis à agitation 3 heures à température ambiante (de l'ordre de 18-21° C).

*4/ Réduction* :

500 mg de borohydrure de sodium $NaBH_4$, PM : 37,83) sont alors ajoutés et la solution est agitée de nouveau 4 heures à température ambiante. Son pH est ensuite amené à 4 à l'aide d'acide chlorhydrique concentré. Après 15 minutes d'agitation, le pH est ajusté à 7 à l'aide de soude concentrée.

Aux 820 ml de solution ainsi obtenue sont ajoutés 16,4 g de NaCl puis 1270 ml d'éthanol. L'ensemble est laissé au repos 3 heures, puis centrifugé à 2.500 t/minute pendant 20 minutes. Le précipité est recueilli, remis en suspension dans 200 ml d'éthanol pur, broyé à l'Ultra-Turrax$^R$ et finalement récupéré par filtration sur buchner fritté. Il est alors séché sous vide à 40° C pendant 5 heures.

On récupère ainsi 8,9 g de produit.

5/ Fractionnement alcoolique :

Ces 8,9 g sont dissous dans environ 120 ml d'eau déminéralisée à température ambiante. On ajoute 1,78 g de NaCl et le pH de la solution est abaissé à 3,5 à l'aide d'acide chlorhydrique. Le volume de la solution est ajusté à 178 ml à l'aide d'eau déminéralisée. 151 ml d'éthanol pur sont ajoutés sous agitation. L'agitation est maintenue 15 minutes après la fin de l'addition, puis l'ensemble est laissé au repos 10 heures, à température ambiante.

Le précipité formé est recueilli par centrifugation 20 minutes à 2.500 t/minute. Il est remis en suspension dans 150 ml d'éthanol pur, broyé à l'Ultra-Turrax[R], récupéré par filtration sur buchner fritté, lavé par 300 ml d'éthanol pur et finalement séché sous vide à 40 ° C pendant 24 heures.

On récupère ainsi sous forme de poudre blanche 5 grammes du produit IC 1772, ayant les caractéristiques suivantes :

| | |
|---|---|
| - $SO_3^-$ | 3,55 meq/g |
| - $COO^-$ | 1,54 meq/g |
| - S + C | 5,09 meq/g |
| - S/C | 2,31 meq/g |

Il est pratiquement dépourvu de N-acétyl glucosamine (absence de signal à ~ 24,5 ppm sur le spectre du carbone).

| | |
|---|---|
| - Titre Codex | 11 ui/mg |
| - Titre APTT | 9 ui/mg |
| - Titre anti-Xa | 12 ui/mg |

***B/ PREPARATION D'UN FRAGMENT D'HEPARINE DEPOURVU D'AFFINITE POUR L'ANTITHROMBINE III O−ACETYLE (IC 1924)***

Le produit obtenu à l'étape ***A/*** est converti en sel de tétrabutylammonium par passage sur une résine Dowex 50 $H^+$, suivi de neutralisation à l'aide d'hydroxyde de tétrabutylammonium. A partir de 9,5 g de sel de sodium, on obtient 18 g de sel de tétrabutylammonium.

A une solution de 6 g du sel ainsi obtenu, dans le diméthylformamide (55 ml), on ajoute, après refroidissement à 0 ° C, de l'anhydride acétique (6,2 ml; 65,6 mmoles) puis de la triéthylamine (9 ml; 65,5 mmoles) et de la diméthylaminopyridine (403 mg; 3,3 mmoles). Après 24 heures, une solution saturée d'acétate de sodium dans l'éthanol est ajoutée (250 ml). Après centrifugation et lavage du précipité à l'éthanol, le solide est dessalé sur Séphadex G-25, puis soumis à un échange d'ions par passage sur Dowex $50H^+$, suivi de neutralisation par la soude. Après lyophilisation, on obtient le produit IC 1924 (3 g).

Ce produit présente les caractéristiques suivantes :

- rapport sulfate/carboxyle : 2,28 meq/g

Le spectre de RMN du carbone (méthanol 51,6 ppm, standard interne) montre clairement que le produit a été O-acylé. Le signal caractéristique du $C_2$ de la N-acétyl-glucosamine, aux environs de 56 ppm, est tout comme dans le produit de départ, absent du spectre.

***EXEMPLE 19 : PREPARATION D'UN FRAGMENT D'HEPARINE DEPOURVU D'AFFINITE POUR L'ANTITH−ROMBINE III O−BUTYRYLE (IC 1925)***

6g du sel de tétrabutylammonium de IC 1772, obtenu comme décrit dans l'exemple 18, sont butyrylés par l'anhydride butyrique dans les conditions décrites pour l'acétylation. On obtient le produit IC 1925 (2,96 g) qui présente les caractéristiques suivantes :

- rapport sulfate/carboxyle : 2,31 meq/g

Le spectre $^{13}C$-RMN (méthanol 51,6 ppm, standard interne) contient les signaux caractéristiques du groupe butyle à 15,6, 20,4 et 38,4 ppm.

***EXEMPLE 20 : PREPARATION D'UN FRAGMENT D'HEPARINE DEPOURVU D'AFFINITE POUR L'ANTITHROMBINE III O-HEXANOYLE (IC 1926)***

6g du sel de tétrabutylammonium de IC 1772, obtenu comme décrit dans l'exemple 12, sont traités par l'anhydride hexanoïque de la même façon que pour l'acétylation. On obtient le produit IC 1926 (3g) qui présente les caractéristiques suivantes :

- rapport sulfate/carboxyle : 2,20 meq/g

Le spectre de RMN du carbone (méthanol 51,6 ppm, standard interne) présente les signaux caractéristiques des $CH_3$ et $CH_2$ du groupe hexyl à 15,5, 23,9, 26,2, 32,7 et 36,1 ppm.

Le spectre de RMN du proton indique la présence d'environ un groupe hexyl par unité disaccharidique.

***EXEMPLE 21 : PREPARATION DE MELANGE DE FRAGMENTS DEPOURVUS D'AFFINITE POUR L'ANTITHROMBINE III, HOMOGENES AU REGARD DE LEUR MASSE MOLECULAIRE ET O-BUTYRYLES***

Le mélange de fragments dépourvus d'activité anticoagulante décrit à l'exemple 12 est fractionné, en ses différents constituants, par gel filtration. On obtient ainsi des fractions homogènes en masse moléculaire dont les masses sont de 7700, 6500, 5800, 5300, 4980, 4400, 3900, 3400, 2600, 1860 et 1210.

**Exemple d'estérification d'une fraction :**

La fraction de masse 2600 (0,20g) est transformée en sel de tributylammonium, puis lyophilisée et séchée (0,34g). Ce produit est ensuite dissous dans du DMF (2 ml) puis de la diméthylaminopyridine (18 mg), de l'anhydride butyrique (0,49 ml) et de la tributylamine (0,7ml) sont ajoutés successivement, après refroidissement à 0 °C. Après 24 heures à température ambiante on ajoute du bicarbonate de sodium (1 ml d'une solution à 5%) puis, 2 heures plus tard, le mélange est chromatographié sur une colonne de Sephadex G25 (1 litre) élué par du chlorure de sodium 0.2M. Le produit est lyophilisé après dessalage donnant une poudre beige clair (0,24 g).

Les autres fractions sont traitées de la même façon, donnant les produits correspondants dont l'analyse IR révèle la présence d'un bande ester à 1734 $cm^{-1}$. Le taux de sulfatation des produits (raport sulfate sur carboxyle) est inchangé après acylation.

**Détermination du taux d'acylation :**

Celui-ci est déterminé par chromatographie en phase gazeuse, après butanolyse des produits par un mélange butano-acide sulfurique, suivi d'extraction au chloroforme des esters butyliques et élimination du butanol en excès par lavage à l'eau.

***EXEMPLE 22 : PREPARATION DE DERMATANE SULFATE O-ACETYLE (IC 1947)***

A une solution, refroidie à 0 °C, de sel de tétrabutylammonium de dermatane sulfate (0,91 g), obtenu dans les mêmes conditions que celles décrites pour l'obtention d'héparinate de tétrabutylammonium dans l'exemple 1, dans le diméthylformamide (20 ml), on ajoute, goutte à goutte, de l'anhydride acétique (1,35 ml ; 14,2 mmoles) puis de la triéthylamine (1,97 ml ; 14,2 mmoles) et de la diméthylaminopyridine (0,7 mmole). Après 24 heures à température ambiante, on ajoute de l'eau (40 ml, puis dialyse pendant 72 heures. Le sel de sodium est obtenu par passage au travers d'une colonne Dowex 50 $H^+$ , à 0 °C, suivie de neutralisation par la soude. On obtient, après lyophilisation, une poudre beige (0,52 g).

Le dermatane sulfate O-acétylé présente les caractéristiques suivantes :

- rapport sulfate/carboxyle : 0,99 meq/g (produit de départ : 1,05 meq/g)
- spectre $^{13}C$-RMN (méthanol 51,6 ppm, standard interne)
  . signal à 25,2 ppm $CH_3$ de $CH_3$-CO-NH- (identique au produit de départ)
  . signal à 23,0 ppm $CH_3$ de $CH_3$-CO-O

***EXEMPLE 23 : PREPARATION DE DERMATANE SULFATE O-SUCCINYLE (IC 2020)***

Le sel de tétrabutylammonium du dermatane sulfate (1g) et de la N,N-diméthylaminiopyridine (110 mg) sont dissous dans du diméthylformamide anhydre. Après addition d'anhydride succinique (396 mg) et de tributylamine (0,94 ml), le milieu est incubé en conditions anhydres pendant 2 heures à 60 °C. Après refroidissement et addition d'eau (2 ml), on effectue une précipitation dans une solution alcoolique glacée

saturée en acétate de sodium. Le précipité est lavé à l'éthanol, solubilisé dans l'eau apyrogène, puis dialysé contre du bicarbonate de sodium à 5% pendant 36 heures, puis contre de l'eau pendant 3 jours. On obtient, après lyophylisation, le dermatane sulfate O-succinylé sous forme de sel de sodium (0,83 mg).

Le spectre infra-rouge (KBr) présente à 1730 $cm^{-1}$ et 1420 $cm^{-1}$ les fréquences caractéristiques (nombre d'onde) du groupement carboxylique.

Le spectre RMN du carbone dans $D_2O$ (méthanol à 51,6 ppm comme standard interne) présente à 33; 34,5 et 183,6 ppm les signaux caractéristiques du groupement succinyle, et à 25,3 ppm le signal caractéristique du méthyle du groupement acétamido, identique au produit de départ.

Rapport sulfate/carboxyle : 0,51.

***EXEMPLE 24 : PREPARATION D'HEPARINE O–BUTYRYLEE, PREALABLEMENT N–DESULFATEE ET N–ACETYLEE (IC 1948)***

L'héparine est N-désulfatée puis N-acétylée selon la technique décrite par Nagasawa et Inoue (Methods Carbohydr. Chem. Vol. VIII, p.291-294).

Le sel de tributylammonium (1 g) est obtenu par passage sur une résine Dowex 50 $H^+$, suivi de neutralisation par une solution de tributylamine dans l'éthanol.

Après lyophilisation et séchage, ce sel est dissous dans le diméthylformamide (10 ml) puis, après refroidissement à 0 ° C, de l'anhydride butyrique (2 ml), de la tributylamine (2 ml) et de la diméthylaminopyridine (65 mg) sont ajoutés. Après 24 heures, de l'eau (5 ml) est ajoutée, puis le mélange est dialysé contre une solution à 5% de bicarbonate de sodium, puis contre de l'eau. Après passage sur Dowex 50 $H^+$, suivi de neutralisation par la soude, on obtient le sel de sodium de l'héparine N-acétylée O-butyrylée.

Le spectre de RMN du carbone (méthanol 51,6 ppm, standard interne) présente un signal à 24,6 ppm caractéristique du N-acétyl et des signaux à 16, 20 et 38 ppm caractéristiques des esters butyriques.

L'expérience peut être répétée avec une héparine partiellement N-désulfatée N-acétylée et conduire à un produit partiellement N-désulfaté N-acétylé et O-butyrylé.

***EXEMPLE 25 : PREPARATION DE DERMATANE SULFATE PERACETYLE (IC 1950)***

Le sel de tétrabutylammonium du dermatane sulfate (0,8 g) dissous dans du diméthylformamide (20 ml) est acétylé par addition de diméthylaminopyridine (76 mg), d'anhydride acétique (1,2 ml) et de triéthymaine (1,7 ml). Le mélange est chauffé à 80 ° C pendant 1 heure.

Après retour à température ambiante, de l'eau (0,45 ml) est ajoutée, suivie d'une solution 0,3 M d'acétate de sodium dans l'éthanol (100 ml). Après centrifugation, le précipité est dissous dans l'eau puis dialysé contre l'eau distillée. Le sel de sodium est obtenu par échange sur une colonne Dowex 50 H +, suivi de neutralisation par la soude. On obtient, après lyophilisation, le dermatane sulfate peracétylé (0,51 g).

Ce produit présente un rapport sulfate/carboxyle de 1,07 meq/g (produit de départ : 1,05 meq/g) et contient environ trois groupes acétyles par unité disaccharidique.

***EXEMPLE 26 : PREPARATION D'HEPARINE BENZYLESTER O–ACETYLEE (IC 1949)***

A une solution d'héparinate de tétrabutylammonium (1 g) dans le diméthylformamide (10 ml), on ajoute du bromure de benzyle (0,17 ml). Après 24 heures à température ambiante, on ajoute de l'acétate de tétrabutylammonium (220 mg). Après 24 heures, on procède à l'acétylation du benzylester formé. Pour cela, de la diméthylaminopyridine est introduite (57 mg), suivie de triéthylamine (1,3 ml) et d'anhydride acétique (0,9 ml).

Après retour à température ambiante, le mélange réactionnel est agité pendant 24 heures. De l'eau est alors ajoutée, puis le produit est précipité par une solution saturée d'acétate de sodium dans l'éthanol. Après dialyse contre de l'eau distillée, passage sur Dowex 50 H +, neutralisation par la soude et lyophylisation, on obtient le sel de sodium de l'héparine benzylester O-acétylée (0,57 g).

Le produit présente un rapport sulfate/carboxyle de 3,6 meq/g (produit de départ non benzylé : 2,20 meq/g).

Le spectre du carbone (méthanol 51,6 ppm, standard interne) présente des signaux à 23,3 ppm (O-acétyle) et à 131,6 ppm (benzyle).

Le signal du $CH_3$ de $CH_3$-CO-NH, à ~ 24,5 ppm, est identique à celui du produit de départ.

***EXEMPLE 27 : PREPARATION DE DERMATANE SULFATE BENZYL ESTER O−ACETYLE (IC 1953)***

Le sel de tétrabutylammonium du dermatane sulfate (1 g) est dissous dans du diméthylformamide anhydre (15ml). A cette solution, refroidie à 0 °C, on ajoute du bromure de benzyle (0,25 ml), puis on laisse pendant 24 heures à température ambiante.

De l'acétate de tétrabutylammonium (0,32 g) est alors ajouté, puis après 24 heures à température ambiante, on procède à l'acétylation.

De l'anhydride acétique (1,5 ml) est introduit, suivi de triéthylamine (2,2 ml) et de diméthylaminopyridine (96 mg). Après 24 heures, de l'eau (0,6 ml) est ajoutée, puis le produit est précipité par addition d'une solution éthanolique saturée d'acétate de sodium.

Le produit est dialysé contre du chlorure de sodium à 10%, puis de l'eau.

On obtient, après lyophilisation, l'ester benzylique du dermatane sulfate O-acétylé (0,63 g).

***EXEMPLE 28 : PREPARATION DU DERMATANE SULFATE O−BUTYRYLE (IC 2018)***

Le sel de tributylammonium du dermatane sulfate (2g), obtenu dans les mêmes conditions que celles décrites pour l'obtention d'héparinate de tributylammonium dans l'exemple 2, et de la N,N-diméthylaminopyridine (220 mg) sont dissous dans le diméthylformamide anhydre (25 ml). Après refroidissement à 0 °C, on ajoute, goutte à goutte, de l'anhydride butyrique (5,9 ml) puis de la tributylamine (8,6 ml). Après 24 heures d'incubation, et refroidissement à 0 °C de l'eau est ajoutée (1 ml); précipitation dans une solution alcoolique glacée, saturée en acétate de sodium. Le précipité est lavé à l'éthanol, solubilisé dans l'eau apyrogène et dialysé contre du bicarbonate de sodium à 5% pendant 36 heures, puis contre de l'eau pendant 3 jours. On obtient, après lyophilisation, le dermatane sulfate O-butyrylé sel de sodium (1,3 g).

Le spectre RMN du carbone dans $D_2O$ (méthanol à 51,6 ppm comme standard interne) présente à 15,6; 20,5 et 38,4 ppm les signaux caractéristiques du groupement butyryle, et à 25,5 ppm le signal caractéristique du méthyle du groupement acétamido, identique au produit de départ.

Rapport sulfate/carboxyle : 1,05.

***EXEMPLE 29 : PREPARATION DU DERMATANE SULFATE O−HEXANOYLE (IC 2019)***

Le sel de tributylammonium du dermatane sulfate (2 g) et de la N,N-diméthylaminopyridine (220 mg) sont dissous dans du diméthylformamide anhydre (25 ml). Après refroidissement à 0 °C, on ajoute, goutte à goutte, de l'anhydride hexanoïque (9,4 ml) puis de la tributylamine (8,6 ml). Après 24 heures à température ambiante et refroidissement à 0 °C, addition d'eau (1 ml), on effectue une précipitation dans une solution alcoolique glacée saturée en acétate de sodium. Le précipité est lavé par l'éthanol absolu, solubilisé dans l'eau apyrogène et dialysé contre du bicarbonate de sodium à 5% pendant 36 heures, puis contre de l'eau pendant 3 jours. On obtient, après lyophilisation, le dermatane sulfate O-hexanoylé sel de sodium (1 g).

Le spectre RMN du carbone dans $D_2O$ (méthanol à 15,6 ppm comme standard interne) présente à 15,9; 24,2; 26,5; 33,1 et 36,4 ppm les signaux caractéristiques du groupement O-hexanoylé et à 25,2 ppm le signal caractéristique du méthyle du groupement acétamido, identique au produit de départ.

Rapport sulfate/carboxyle : 1,02.

***EXEMPLE 30 : MISE EN EVIDENCE DE L'O−ACETYLATION SELECTIVE PAR LE PROCEDE DE L'INVENTION ET COMPARAISON AVEC D'AUTRES PROCEDES D'ACYLATION***

Le procédé de la présente invention a été comparé aux procédés des brevets FR 2100735 et EP 256880. En particulier, les caractéristiques de l'ester acétique de l'héparine préparé selon le procédé de l'invention (IC 1938, exemple 1) ont été comparées à celles de l'ester acétique de l'héparine obtenu en appliquant des conditions opératoires décrites dans le brevet FR 2100735 (produit A) et aux esters acétiques de l'héparine obtenus selon les modes opératoires des exemple 3 et 4 du brevet EP 256880 (produits B et C).

**(a) PREPARATION DU PRODUIT A**

Dans une solution d'héparinate de tétrabutylammonium (1 g) dissous dans du diméthylformamide anhydre (10ml), on introduit du dichlorohexylcarbodiimide (4,2 g) dissous dans du diméthylformamide (15 ml), puis de l'acide acétique (1,16 ml) dissous dans du diméthylformamide (25 ml) est ajouté goutte à goutte en 45 minutes à +4 °C.

Après 24 heures à température ambiante, le mélange réactionnel est filtré et concentré sous vide. Le résidu est remis en suspension dans l'éther. Après filtration et lavage, le précipité est dialysé contre de l'eau distillée. Le sel de sodium est obtenu par passage sur une colonne de résine Dowex H+, puis neutralisation par la soude. On obtient 0,493 g de produit A.

Cette préparation a également été réalisée pendant 48 heures à +4°C.

**(b) PREPARATION DES PRODUITS B ET C**

Les produits B et C ont été préparés par acétylation de l'héparine dans un mélange de formamide et de pyridine par du chlorure d'acétyle, en utilisant 2 ml de chlorure d'acétyle pour le produit B et 40 ml pour le produit C, dans les conditions décrites dans les exemples 3 et 4 du brevet EP 256880. L'ester acétique est ensuite repris dans l'eau et dialysé contre du chlorure de sodium.

**(c) RESULTATS**

Les caractéristiques des produits sont données dans le tableau 1. Les caractéristiques de l'héparine de départ utilisée dans chaque essai sont données à titre de référence.

TABLEAU 1

| Produit | Rapport Sulfate/Carboxyle (meq/g) | Titre APTT* ui/mg | Titre YW* u/mg |
|---|---|---|---|
| IC 1938 | 2,28 | 91 | 70 |
| Hép. départ | 2,20 | 160 | 160 |
| A | 3,40 | 42 | 41 |
| Hép. départ | 2,40 | 160 | 160 |
| B | 2,10 | 57 | 39 |
| C | 1,15 | <2 | 0,6 |
| Hép. départ | 2,40 | 160 | 160 |

* Les titres APTT et Yin et WEssler sont mesurés in vitro.

Les résultats montrent que, bien que les titres APTT et YW soient inférieurs pour tous les produits à ceux de l'héparine de départ, et ceci plus fortement pour les produits A, B et C, seul le produit IC 1938 conserve un rapport sulfate/carboxyle pratiquement identique à celui de l'héparine de départ. Ceci résulte du fait que le procédé de l'invention permet d'acyler sélectivement les fonctions hydroxyles sans altérer les groupements fonctionnels de l'héparine, ce qui est confirmé par l'analyse chimique des produits.

Les produits IC 1938, A, B et C ont été analysés en RMN du carbone (méthanol 51,6 ppm, standard interne). Les spectres de ces produits ainsi que celui de l'héparine de départ sont donnés comme suit :

Figure 1 : Héparine de départ
Figure 2 : IC 1938
Figure 3 : Produit A après 24 heures de réaction
Figure 4 : Produit A après 48 heures de réaction
Figure 5 : Produit B
Figure 6 : Produit C

Les résultats sont les suivants :

***1. Le produit IC 1938 présente dans le spectre du carbone (figure 2)*** **:**

- un signal à 23,4 ppm correspondant au $CH_3$ de $CH_3$-CO-O
- un signal à 24,4 ppm correspondant au $CH_3$ de $CH_3$-CO-NH,

identique au signal présent dans l'héparine de départ.

Ces signaux montrent que les groupements aminés et carboxyles ont été respectés et qu'il y a bien eu acétylation sélective au niveau des groupements hydroxyles.

***2. L'analyse du produit A en spectroscopie RMN du carbone montre*** que le produit obtenu en majorité, aussi bien après 24 heures qu'après 48 heures (figures 3 et 4), est un dérivé isourée de l'héparine, dont les fonctions carboxyles sont substituées par un groupement

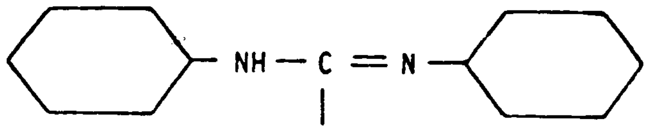

du fait de l'utilisation du dicycloheyxylcarbodiimide. En effet, on observe des signaux importants correspondant aux atomes de carbone du groupe ci-dessus vers 27, 24, 54 et 156 ppm, alors que le signal correspondant au $CH_3$ de $CH_3$ - CO - O vers 23 ppm est très faible.

Le procédé ne permet donct pas d'obtenir une O-acylation sélective et entraîne une altération des fonctions carboxyles, que reflète l'augmentation du rapport sulfate/carboxyle.

***3. Le produit B présente dans le spectre du carbone (figure 3)***:

- un signal à 23,1 ppm correspondant au $CH_3$ de $CH_3$ - CO
- O
- un signal à 24,8 ppm correspondant au $CH_3$ de $CH_3$ - CO
- NH,

nettement plus intense que ceux de l'héparine de départ et de IC 1938.

Ces signaux montrent qu'on observe non seulement une O-acétylation, mais également une forte N-acétylation. Le procédé d'acylation utilisé, non sélectif, entraîne une N-désulfatation partielle, suivie d'une acétylation des amines, ce qui entraîne également une diminution du rapport sulfate/carboxyle.

***4. Le produit C présente dans le spectre du carbone*** :

- un signal à 22 ppm correspondant au $CH_3$ de $CH_3$-CO-O
- un signal à 24 ppm correspondant au $CH_3$ de $CH_3$-CO-NH, nettement plus intense que ceux de l'héparine de départ et de IC 1938.

Comme pour le produit B, on observe à la fois une O- et une N-acétylation. La N-désulfatation, plus importante que pour le produit B, entraîne une forte diminution du rapport sulfate/carboxyle.

***ACTIVITEPHARMACOLOGIQUE DES PRODUITS DE L'INVENTION***

***A/ Activité anticoagulante in vitro*** :

**1. Evaluation du titre in vitro par rapport à une gamme-étalon :**

Cette mesure a été effectuée à l'aide du test "temps de céphaline kaolin sensibilisé" (Diagnostica Stago, Asnières, France), sur plasma humain. Les résultats sont donnés dans le Tableau 2.

TABLEAU 2

| Produit | Titre (ui/mg) |
|---|---|
| IC 1940 | 113 |
| IC 1941 | 28 |
| IC 1943 | 7 |
| Héparine | 160 |

IC 1940 = héparine O-butyrylée préparée comme décrit dans l'exemple 5
IC 1941 = héparine O-hexanoylée préparée comme décrit dans l'exemple 6
IC 1943 = héparine O-décanoylée préparée comme décrit dans l'exemple 8

Les résultats obtenus montrent que les produits sélectivement O-acylés de l'invention ont un titre in vitro inférieur à celui de l'héparine, leur activité anticoagulante décroissant lorsque la longueur de la chaîne acylante augmente.

**2. Mesure de l'activité anticoagulante in vitro des produits de l'invention sur sang humain :**

Les essais sont effectués à deux doses des produits de l'invention, 2 µg/ml et 4 µg/ml sur 5 ml de sang humain. Un essai témoin est également effectué pour chaque produit en remplaçant le produit à tester par du soluté isotonique de NaCl. Après 30 minutes d'incubation à température ambiante, le sang est centrifugé pendant 20 minutes à 3000 tours/minute. Le plasma déplaquetté est décanté pour permettre de réaliser les tests suivants :

- TCK (temps de Céphaline Kaolin) à l'aide du kit "temps de céphaline kaolin sensibilisé" (Diagnostica Stago, Asnières, France),
- Heptest$^R$ (Hemachem, St Louis, USA).

Les résultats sont donnés dans le Tableau 3. Chaque résultat correspond à la moyenne de trois essais.

TABLEAU 3

| Produit | Dose (µg/ml) | TCK (S) | HEPTEST (S) |
|---|---|---|---|
| Témoin | | 48,00 ± 2,70 | 21,50 ± 1,73 |
| IC 1940 | 2 | 208,75 ± 45,54 | 61,00 ± 8,52 |
| IC 1940 | 4 | 428,00 ± 103,43 | 101,00 ± 28,36 |
| Témoin | | 45,00 ± 5,00 | 23,66 ± 2,08 |
| IC 1941 | 2 | 53,00 ± 5,19 | 40,33 ± 7,63 |
| IC 1941 | 4 | 84,66 ± 15,01 | 61,33 ± 11,01 |
| Témoin | | 49,66 ± 8,50 | 23,00 ± 4,35 |
| IC 1943 | 2 | 50,00 ± 9,54 | 23,00 ± 4,58 |
| IC 1943 | 4 | 56,00 ± 10,00 | 24,00 ± 5,29 |

Les résultats montrent dans les deux méthodes un allongement du temps de coagulation pour les produits IC 1940 et IC 1941. Cet allongement est proportionnel à la dose.

En revanche, dans ces méthodes in vitro, le produit IC 1943 n'a montré qu'une très faible action sur l'allongement du temps de coagulation.

**3. Mesure de l'activité anticoagulante in vitro des produits de l'invention par thromboélastographie sur sang humain :**

Les produits de l'invention, aux doses de 2 µg/ml et 4 µg/ml sont mis en présence de 5 ml de sang humain comme décrit dans l'essai précédent, un tube témoin étant réalisé pour chaque produit en remplaçant le produit à tester par du soluté isotonique de NaCl.

Après les 30 minutes d'incubation à température ambiente, un tracé thromboélastographique est réalisé à l'aide d'un thromboélastographe Hellige sur 0,25 ml de sang recalcifié par 0,1 ml de $CaCl_2$ 0,058 M.

Les résultats sont donnés dans le Tableau 4.

TABLEAU 4

| Produit | Dose (µg/ml) | r * | k * | r + k * | amx * | IPT * |
|---|---|---|---|---|---|---|
| Témoin | | 13,00 ± 0,81 | 8,25 ± 1,25 | 21,25 ± 1,50 | 48,00 ± 2,44 | 11,25 ± 2,36 |
| IC 1940 | 2 | 33,50 ± 2,88 | 22,25 ± 4,03 | 55,75 ± 5,85 | 35,50 ± 3,69 | 2,57 ± 0,77 |
| IC 1940 | 4 | 78,75 ± 17,34 | 44,00 ± 8,68 | 122,75 ± 13,02 | 27,00 ± 16 | 0,82 ± 0,27 |
| Témoin | | 10,50 ± 0,70 | 4,50 ± 2,12 | 15,00 ± 2,82 | 57,00 ± 8,48 | 37,00 ± 28,28 |
| IC 1941 | 2 | 14,00 ± 3,00 | 10,33 ± 6,50 | 24,33 ± 6,00 | 51,66 ± 10,96 | 21,33 ± 19,85 |
| IC 1941 | 4 | 20,33 ± 3,51 | 15,00 ± 4,58 | 35,33 ± 5,03 | 40,33 ± 2,51 | 4,66 ± 2,08 |
| Témoin | | 13,33 ± 2,30 | 9,00 ± 2,64 | 22,33 ± 4,93 | 47,00 ± 4,58 | 10,33 ± 4,04 |
| IC 1943 | 2 | 12,66 ± 2,08 | 8,00 ± 2,64 | 20,66 ± 4,72 | 47,00 ± 5,29 | 12,00 ± 5,29 |
| IC 1943 | 4 | 12,66 ± 2,08 | 9,83 ± 3,01 | 22,50 ± 5,07 | 45,66 ± 5,77 | 9,33 ± 4,04 |

* r = temps de réaction
k = temps de coagulation correspondant à une amplitude du tracé de 20 mm
amx = amplitude maximale
IPT = indice de potentiel thrombodynamique

Les résultats montrent que IC 1940 et IC 1941 entraînent une augmentation du r + k, une diminution de l'amx et de l'IPT, ce qui correspond à une hypocoagulation accrue. Cette hypocoagulation augmente à la fois en fonction de la dose utilisée et de la longueur de la chaîne acylante.

IC 1943, dans ce test, ne provoque pas de modification sensible des paramètres.

***B/ ACTIVITE ANTICOAGULANTE IN VIVO*** :

**1. Mesure de l'activité anticoagulante in vivo des produits de l'invention chez le lapin (voie i.v.) :**

Les essais ont été réalisés sur des lapins néo-zélandais mâles. Un prélèvement sanguin est effectué au niveau de l'artère médiane de l'oreille avant l'injection du produit.

On injecte ensuite dans la veine marginale de l'oreille une solution du produit à tester de 25 mg de produit dans 5 ml de soluté isotonique de NaCl.

Le test du TCK et l'HEPTEST$^R$ sont réalisés sur le prélèvement sanguin effectué avant l'injection des produits, et sur des prélèvements effectués 6 h, 24 h, 48 h et 96 h après l'injection.

Les résultats sont donnés sur les figures 7 et 8. Ils montrent un net allongement dans le temps de l'activité anticoagulante, cet allongement augmentant avec la longueur de la chaîne acylante.

En effet, l'activité anticoagulante du produit IC 1940 est encore nette plus de 6 heures après l'injection, alors qu'elle est encore nette 24 heures après l'injection pour le produit IC 1941 et qu'elle se prolonge jusqu'à 96 heures pour le produit IC 1943.

**2. Mesure de l'activité anticoagulante in vivo des produits de l'invention par thromboélastographie :**

Les produits à tester sont injectés par voe i.v. comme décrit dans l'essai précédent (25 mg dans 5 ml de soluté isotonique NaCl). Un tracé thromboélastographique est réalisé à l'aide d'un thromboélastographe Hellige sur 0,25 ml des échantillons de sang prélevés avant l'injection et 6 h, 24 h, 48 h et 96 h après l'injection, des prélèvements supplémentaires étant effectués lorsque l'activité anticoagulante persiste au-delà de 96 heures.

Les résultats sont donnés dans les Tableaux 5, 6 et 7.

TABLEAU 5

| PRODUIT TESTE : IC 1940 | | | | | |
|---|---|---|---|---|---|
| | r | k | r + k | amx | IPT |
| Avant Injection | 13 | 6 | 19 | 67 | 33 |
| 6 h. après Injection | 91 | 60 | 151 | 56 | 2,1 |
| 24 h. après Injection | 12 | 5 | 17 | 67 | 40 |
| 96 h. après Injection | 8 | 3 | 11 | 70 | 77 |

TABLEAU 6

| PRODUIT TESTE : IC 1941 | | | | | |
|---|---|---|---|---|---|
| | r | k | r + k | amx | IPT |
| Avant Injection | 13 | 5 | 18 | 67 | 40 |
| 6 h. après Injection | 135 | 31 | 166 | 45 | 2,6 |
| 24 h. après Injection | 19 | 12 | 31 | 58 | 11,5 |
| 48 h. après Injection | 22 | 10 | 32 | 72 | 25 |

TABLEAU 7

| PRODUIT TESTE : IC 1943 | | | | | |
|---|---|---|---|---|---|
| | r | k | r + k | amx | IPT |
| Avant Injection | 12 | 4 | 16 | 69 | 55 |
| 6 h. après Injection | 40 | 27 | 67 | > 40 | - |
| 24 h. après Injection | 13 | 7 | 20 | 72 | 36 |
| 48 h. après Injection | 53 | 34 | 87 | 54,5 | 4,7 |
| 72 h. après Injection | 112 | 185 | 297 | > 38 | - |
| 96 h. après Injection | 82 | 45 | 127 | 62 | 3,6 |
| 168 h. après Injection | 19 | 9 | 28 | 68 | 23 |
| 192 h. après Injection | 17 | 5 | 22 | 70 | 46 |

Les résultats obtenus montrent que pour tous les produits, on note une forte augmentation du r + k et une diminution de l'IPT à la 6e heure, ce qui indique un allongement de l'hypocoagulabilité. Celui-ci se prolonge jusqu'à au moins 24 heures après l'injection pour le produit IC 1941 et est encore mesurable 96 heures après l'injection pour le produit IC 1943.

On constate donc que tous les produits ont une forte activité anticoagulante in vivo, que cette activité est prolongée dans le temps et que IC 1943 qui n'avait été que peu ou pas actif dans les essais in vitro s'avère très actif et fortement retard dans les essais in vivo.

**3. L'activité anticoagulante des produits IC 1945, IC 1957 et IC 1958 préparés respectivement selon les exemples 12, 13 et 14** a été également testée in vivo chez le lapin.

Les résultats ont montré un pharmacocinétique allongée par voie intraveineuse et par voie sous-cutanée.

***C/ ACTIVITE DANS UN MODELE IN VITRO D'INHIBITION DES VIRUS HIV−1 et 2*** **:**

Les produits préparés selon l'invention ont été testés dans un modèle d'inhibition des virus HIV-1 et HIV-2 in vitro tel que décrit dans R. Pauwels et al, J. Virol. Methods, 1987, 16, 171-185.

Tous les produits testés se sont avérés actifs à des doses totalement dépourvues de cytotoxicité. En particulier, on a constaté que les produits IC 1925 et IC 1926 étaient plus actifs que le produit de départ.

***D/ ACTIVITE DANS UN MODEL IN VITRO D'INHIBITION DE LA FORMATION DE SYNCITIA***

Les syncitia sont des cellules géantes, à noyaux multiples, formées par la fusion de lymphocytes T4 sains avec les lymphocytes T4 infectés.

Les produits préparés selon l'invention ont été testés dans un modèle de co-culture de cellules MOLT4 (lymphocytes T4 sains) et de cellules HUT-78/HTLV III B (Lymphocytes T4 infectés de lignée humaine).

Tous les produits testés se sont avérés actifs dans ce modèle, à des doses totalement dépourvues de cytotoxicité. En particulier, les produits IC 1924, IC 1925 et IC 1926 ont été trouvés plus actifs que le produit de départ.

***E/ ACTIVITE DANS UN MODELE IN VITRO D'INHIBITION DE DIFFERENTS VIRUS ENVELOPPES***

L'activité des produits préparés selon l'invention vi-à-vis de l'inhibition de différents virus enveloppés à ADN ou à ARN, en particulier les virus suivants :

- HSV 1 et 2 (Herpes simplex virus 1 et 2, virus à ADN)
- VSV (vesicular stomatitis virus) Virus à ARN
- Sindbis virus Virus à ARN

Tous les produits testés se sont montrés actifs dans l'inhibtion de ces virus, à des doses dépourvues de cytotoxicité. En particulier, les produits IC 1924, IC 1925 et IC 1926 ont présenté une activité fortement accrue vis-à-vis du VSV et du Sindbis virus, en comparaison avec le produit de départ.

**F/ ACTIVITE DANS UN MODELE IN VIVO D'INHIBITION DE LA PROLIFERATION DES CELLULES MUSCULAIRES LISSES**

Les produits préparés selon l'invention ont été testés dans un modèle d'inhibition de la prolifération des cellules musculaires lisses in vivo chez le rat (modèle du catheter ballon tel que décrit dans A.W. Clowes et M.M. Clowes, Labor. Investig., 1985, 52 (6), 612-616.

Les produits testés se sont montrés actifs. En particulier, les produits IC 1924, IC 1925 et IC 1926 ont présenté une activité accrue par rapport au produit de départ.

**Revendications**

**Revendications pour les Etats contractants suivants : AT, BE, CH, DE, FR, GB, GR, IT, LI, LU, NL, SE**

**1.** Glycosaminoglycanes sélectivement O-acylés répondant à la formule (I) suivante :

**A-[G-U]$_n$-B** (I)

dans laquelle :

- G représente un groupe (a) de formule :

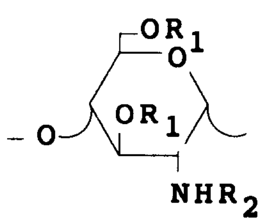

(a)

ou un groupe (a') de formule :

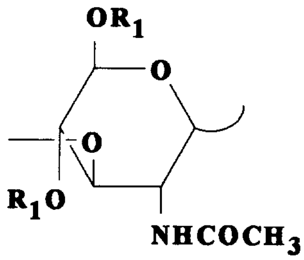

(a')

ou un groupe (b) de formule :

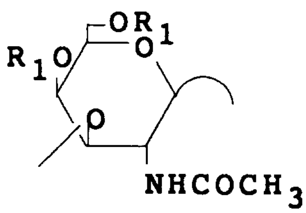

(b)

- U représente un groupe (c) de formule :

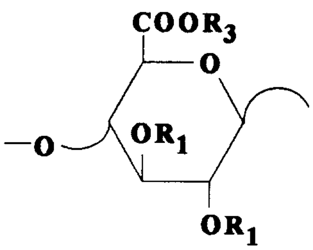

(c)

ou un groupe (d) de formule :

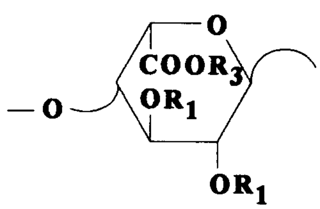

(d)

ou le résidu du groupe (c) ou du groupe (d) après oxydation periodique suivie d'une $\beta$-élimination ou d'une hydrolyse acide ;

- A représente un groupe $R_1$, un groupe $R_1$-(c), un groupe $R_1$-(d), ou un groupe (e) de formule :

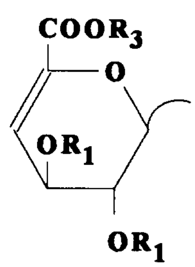

(e)

ou le résidu du groupe (c) ou du groupe (d) après oxydation periodique suivie d'une $\beta$-élimination ou d'une hydrolyse acide ;

- B représente un groupe O-$R_1$, un goupe (a)-O$R_1$, un groupe (a')-O$R_1$, un groupe (b)-O$R_1$ ou un groupe (f) de formule :

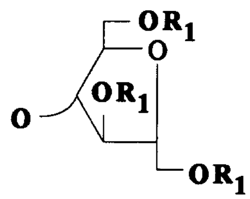

(f)

ou un groupe (g) de formule :

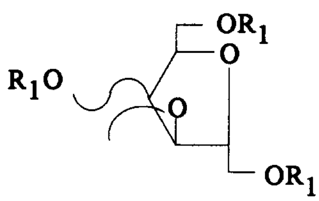

(g)

ou représente un groupe (a), un groupe (a'), ou un groupe (b) auxquels reste accroché un résidu de (c) ou de (d) tel que présent après oxydation periodique suivie d'une $\beta$-élimination ou d'une hydrolyse acide ;
- $R_1$ représente H, $SO_3^-$ , ou un radical acyle d'un acide carboxylique ou d'un acide dicarboxylique non $\alpha,\beta$-insaturé choisi parmi :
  - ♦ un groupe alcanoyle de 1 à 18 atomes de carbone ;
  - ♦ un groupe alcanoyle de 2 à 3 atomes de carbone substitué par :
    - un groupe cycloalkyle de 3 à 7 atomes de carbone,
    - un radical hydrocarboné aliphatique insaturé de 4 à 16 atomes de carbone ;
  - ♦ un groupe benzoyle éventuellement substitué par un ou plusieurs radicaux alkyles de 1 à 4 atomes de carbone, atomes d'halogène ou groupement $NO_2$ ou $OCH_3$ ;
  - ♦ un groupe cycloalkyle (3-7 C) carbonyle ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- n étant un nombre entier de 1 à 80,

$R_1$ étant acyle dans la proportion d'au moins 0,1 à 3 groupes acyles par unité disaccharidique, et leurs sels pharmaceutiquement acceptables,
susceptibles d'être obtenus à partir d'un glycosaminoglycane choisi dans le groupe constitué par :
l'héparine, un mélange de fragments d'héparine ayant une masse moléculaire inférieure à 10.000 daltons, un mélange de fragments d'héparine ayant une masse moléculaire moyenne se situant entre 2.000 et 7.000 daltons, un mélange de fragments d'héparine ayant une masse moléculaire moyenne d'environ 4.500 daltons, un mélange de fragments d'héparine ayant une masse moléculaire moyenne d'environ 2.500 daltons, ou un mélange de fragments d'héparine de même masse moléculaire, une fraction ou un fragment d'héparine dépourvus de site de fixation à l'antithrombine III,
le dermatane sulfate et ses fragments,
que :

(1) on transforme en un sel d'amine tertiaire ou en un sel d'ammonium quaternaire soluble dans un solvant organique aprotique polaire ;
(2) on traite ce sel par un anhydride de formule :

$R_1$-O-$R_1$

dans laquelle $R_1$ représente un radical acyle, tel qu'indiqué précédemment, dans ledit solvant organique aprotique polaire, en présence de quantités catalytiques de pyridine ou d'une dialkylaminopyridine et d'un accepteur de protons à une température située entre 0 ° C et 100 ° C ;

(3) on précipite le produit ainsi obtenu par action d'une solution d'acétate de sodium dans l'éthanol et,

(4) on isole le glycosaminoglycane sélectivement O-acylé par dissolution dans l'eau du précipité ainsi obtenu et par dialyse, et l'on transforme éventuellement le sel de sodium du glycosaminoglycane sélectivement O-acylé ainsi obtenu en un autre sel pharmaceutiquement acceptable.

**2.** Glycosaminoglycanes sélectivement O-acylés selon la revendication 1, caractérisés en ce qu'ils répondent à la formule II suivante :

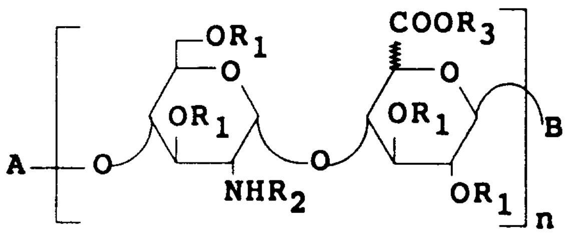

(II)

dans laquelle :

- A a les significations de la revendication 1 ;
- $R_1$ représente H et/ou $SO_3^-$ et/ou un radical acyle, tel que défini dans la formule (I) ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- B représente (a)-$OR_1$ ou (f), (a) et (f) étant tels que définis dans la revendication 1, ou $OR_1$, ou un groupe (a) auquel reste accroché un résidu de (c) ou de (d) tel que présent après oxydation periodique suivie d'une $\beta$-élimination ou d'une hydrolyse acide ;
- n est un nombre entier de 1 à 80, sous réserve que lorsque A représente $R_1$, un groupe $R_1$-(c) ou un groupe $R_1$-(d) et que B représente O-$R_1$, un groupe (a)-$OR_1$, ou un groupe (b)-$OR_1$, n soit un nombre entier de 1 à 16.

**3.** Glycosaminoglycanes sélectivement O-acylés selon la revendication 1, caractérisés en ce qu'ils répondent à la formule II suivante :

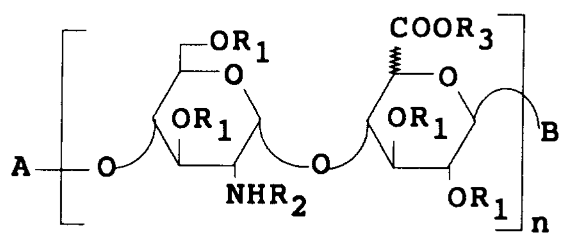

(II)

dans laquelle :

- A et B ont les significations de la revendication 1 ;
- $R_1$ représente H et/ou $SO_3^-$ et/ou un radical acyle d'un acide carboxylique ou dicarboxylique non $\alpha,\beta$-insaturé choisi parmi :
  - ♦ un groupe alcanoyle de 4 à 18 atomes de carbone ;
  - ♦ un groupe alcanoyle de 2 à 3 atomes de carbone substitué par :
    - un groupe cycloalkyle de 3 à 7 atomes de carbone,
    - un radical hydrocarboné aliphatique insaturé de 4 à 16 atomes de carbone ;
  - ♦ un groupe benzoyle éventuellement substitué par un ou plusieurs radicaux alkyles de 1 à 4 atomes de carbone, atomes d'halogène ou groupement $NO_2$ ou $OCH_3$ ;

♦ un groupe cycloalkyle (3-7 C) carbonyle ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- n est un nombre entier de 1 à 80,

$R_1$ étant acyle dans la proportion d'au moins 0,5 à 2 groupes acyles par unité disaccharidique.

4. Glycosaminoglycanes sélectivement O-acylés selon les revendications 1 ou 2, caractérisés en ce que le glycosaminoglycane est choisi dans le groupe constitué par un mélange de fragments d'héparine ayant une masse moléculaire inférieure à 10.000 daltons, un mélange de fragments d'héparine ayant une masse moléculaire moyenne se situant entre 2.000 et 7.000 daltons, un mélange de fragments d'héparine ayant une masse moléculaire moyenne d'environ 4.500 daltons, un mélange de fragments d'héparine ayant une masse moléculaire moyenne d'environ 2.500 daltons, un mélange de fragments d'héparine de même masse moléculaire, le groupe acyle étant dans une proportion de 0,5 à 2 groupes par unités disaccharidique.

5. Glycosaminoglycanes sélectivement O-acylés selon la revendication 3, caractérisés en ce que le glycosaminoglycane est l'héparine, le groupe acyle étant dans une proportion d'au moins 0,5 à 2 groupes par unité disaccharidique.

6. Glycosaminoglycanes sélectivement O-acylés selon la revendication 5, caractérisés en ce que le groupe acyle est dans une proportion de 1 groupe acyle par unité disaccharidique.

7. Glycosaminoglycanes sélectivement O-acylés selon l'une quelconque des revendications 1 à 4, caractérisés en ce qu'ils répondent à la formule III suivante :

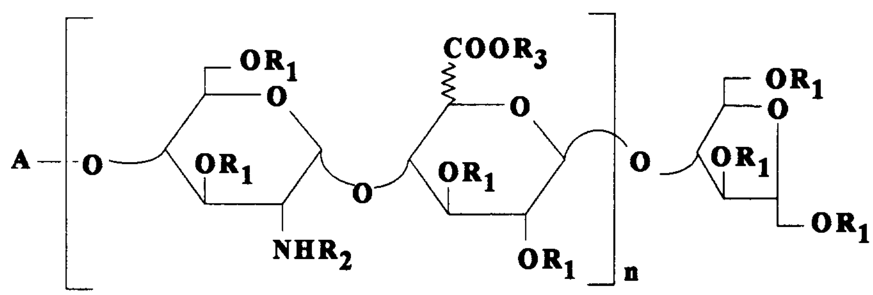

(III)

dans laquelle
- A représente $R_1$, ou $R_1$-(c) ou $R_1$-(d), tels que définis dans la revendication 1 ;
- $R_1$ représente H et/ou $SO_3^-$ et/ou un radical acyle, tel que défini dans la revendication 1 ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène et/ou un radical alkyle de 1 à 10 atomes de carbone, un radical phényl-alkyle de 7 à 12 atomes de carbone, ou un cation métallique alcalin ou alkalino-terreux ;
- n est un nombre entier de 3 à 12.

8. Glycosaminoglycanes sélectivement O-acylés selon l'une quelconque des revendications 1 à 4 caractérisés en ce qu'ils répondent à la formule IV suivante :

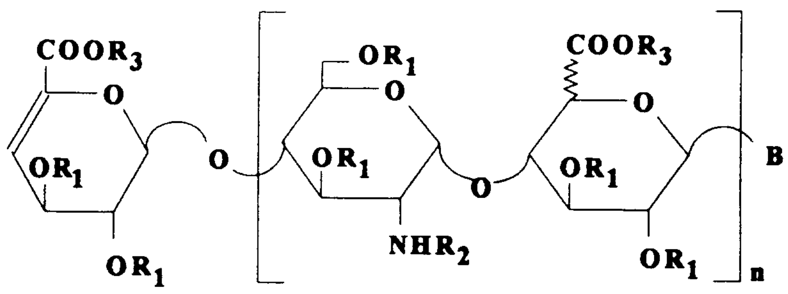

(IV)

dans laquelle :

- $R_1$ représente H et/ou $SO_3^-$ et/ou un radical acyle, tel que défini dans la revendication 1 ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène et/ou un radical alkyle de 1 à 10 atomes de carbone, un radical phényl-alkyle de 7 à 12 atomes de carbone, ou un cation métallique alcalin ou alkalino-terreux ;
- B représente (a)-$OR_1$, tels que défini dans la revendication 1, ou $OR_1$ ;
- n est un nombre entier de 2 à 20.

**9.** Glycosaminoglycanes sélectivement O-acylés selon les revendications 1 ou 2, caractérisés en ce que le glycosaminoglycane choisi est de l'héparine, une fraction ou un fragment d'héparine dépourvus de site de fixation à l'antithrombine III.

**10.** Glycosaminoglycanes sélectivement O-acylés selon l'une quelconque des revendications 1, 2, 3 et 9, caractérisés en ce qu'ils répondent à la formule V suivante :

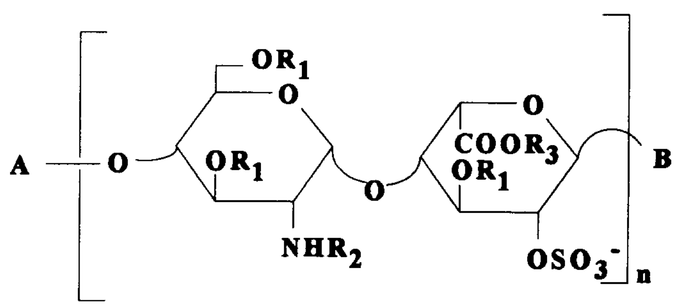

(V)

dans laquelle :

- A représente $R_1$-(c), $R_1$-(d), ou le résidu de (c) ou de (d) après oxydation periodique suivie d'une $\beta$-élimination ou d'une hydrolyse acide ;
- B représente un groupe (a)-$OR_1$, un groupe (a) auquel reste accroché un résidu de (c) ou de (d) tel que présent après oxydation periodique suivie d'une $\beta$-élimination ou d'une hydrolyse acide ;
- $R_1$ a les significations données dans la revendication 1 ;
- $R_2$ représente $SO_3^-$ ou un radical acétyle, la proportion de $SO_3^-$ étant d'environ 90 % ;
- $R_3$ représente un atome d'hydrogène ou un cation métallique alcalin ou alkalino-terreux ;
- n est un nombre entier de 1 à 80.

**11.** Glycosaminoglycanes sélectivement O-acylés selon l'une quelconque des revendications 1, 2, 3 et 9, caractérisés en ce qu'ils répondent à la formule VI suivante :

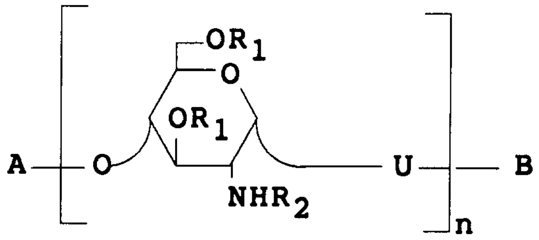

(VI)

dans laquelle :

- A représente $R_1$, $R_1$-(c), $R_1$-(d), ou le résidu de (c) ou de (d) après oxydation periodique suivie d'une $\beta$-élimination ;
- U représente :

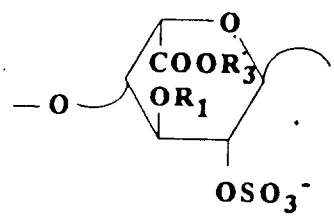

où à raison d'un motif pour deux chaînes au moins, un acide uronique (D-glucuronique ou L-iduronique) non sulfaté ouvert entre les carbones en positions 2 et 3, de formule :

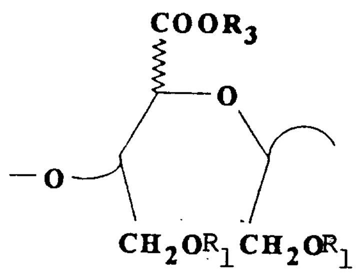

- B représente (a)-$OR_1$, ou $OR_1$ ou un groupe (a) auquel reste accroché un résidu de (c) ou de (d) tel que présent après oxydation periodique suivie d'une $\beta$-élimination ;
- $R_1$ a les significations données dans la revendication 1 ;
- $R_2$ représente $SO_3^-$ ou un radical acétyle, la proportion de $SO_3^-$ étant d'environ 90 % ;
- $R_3$ représente un atome d'hydrogène ou un cation métallique alcalin ou alkalino-terreux ;
- n est un nombre entier de 2 à 18.

**12.** Glycosaminoglycanes sélectivement O-acylés selon l'une quelconque des revendications 1, 2, 3 et 9, caractérisés en ce qu'ils répondent à la formule VI dans laquelle :

- n est un nombre entier de 7 à 15 pour les espèces majoritaires,
- $R_1$ est un radical alcanoyle de 2 à 10 atomes de carbone.

**13.** Glycosaminoglycanes sélectivement O-acylés selon les revendications 11 et 12, caractérisés en ce qu'ils sont constitués d'un mélange de fragments de même masse moléculaire, dans lesquels n est un nombre entier de 2 à 12, et $R_1$ est un radical alcanoyle de 4 à 10 atomes de carbone.

**14.** Glycosaminoglycanes sélectivement O-acylés selon l'une quelconque des revendications 1, 2, 3 et 9, caractérisés en ce qu'ils répondent à la formule VII suivante :

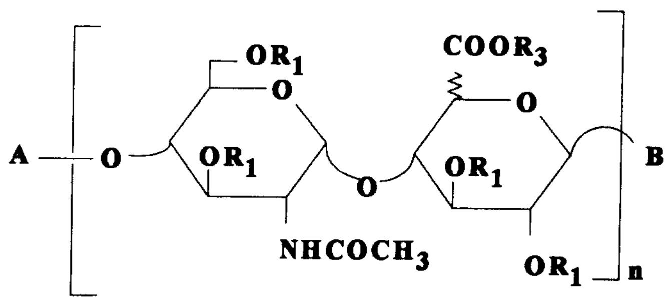

(VII)

dans laquelle :

- A représente $R_1$, $R_1$-(c), ou $R_1$-(d), tels que définis dans la formule (I) ;
- $R_1$ représente un radical alcanoyle de 2 à 18 atomes de carbone ;
- $R_3$ représente un atome d'hydrogène ou un cation métallique alcalin ou alkalino-terreux ;
- B représente (a)-$OR_1$ tel que défini dans la formule (I), ou $OR_1$ ;
- n est un nombre entier de 1 à 80.

**15.** Glycosaminoglycanes sélectivement O-acylés selon la revendication 1, caractérisés en ce qu'ils répondent à la formule VIII suivante :

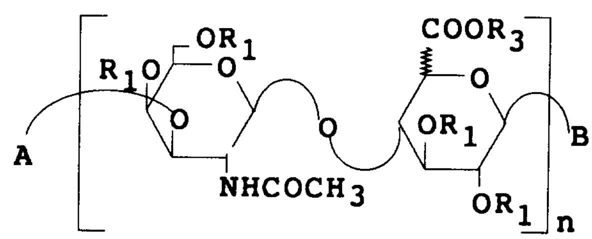

(VIII)

dans laquelle :

- A a les significations de la revendication 1 ;
- $R_1$ représente H et/ou $SO_3^-$ et/ou un radical acyle, tel que défini dans la revendication 1 ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- B représente (b)-$OR_1$, ou (g), tels que définis dans la formule (I), ou $OR_1$, ou un groupe (b) auquel reste accroché un résidu de (c) ou de (d) tel que présent après oxydation periodique suivie d'une $\beta$-élimination ou d'une hydrolyse acide ;
- n est un nombre entier de 1 à 80.

**16.** Glycosaminoglycanes sélectivement O-acylés selon les revendications 1 ou 15, caractérisés en ce que le glycosaminoglycane est choisi parmi le groupe constitué par le dermatane sulfate et ses fragments, le groupe acyle étant dans une proportion d'au moins 0,1 à 3 groupes par unité disaccharidique.

**17.** Glycosaminoglycanes sélectivement O-acylés selon l'une quelconque des revendications 1 à 16, dans lesquelles $R_1$ est un radical alcanoyle de 4 à 10 atomes de carbone.

**18.** Procédé de préparation de glycosaminoglycanes sélectivement O-acylés selon l'une quelconque des revendications 1 à 17 caractérisé en ce que :

(1) on transforme un glycosaminoglycane de formule IX :

$A^\circ$-[$G^\circ$-$U^\circ$]$_n$-$B^\circ$ (IX)

dans laquelle :

- G° représente un groupe (a)° de formule :

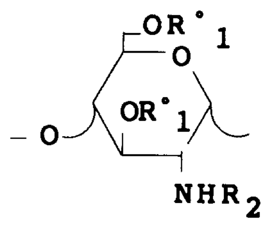

(a)°

ou un groupe (a')° de formule :

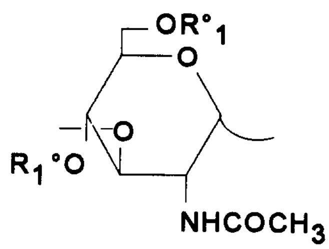

(a')°

ou un groupe (b)° de formule :

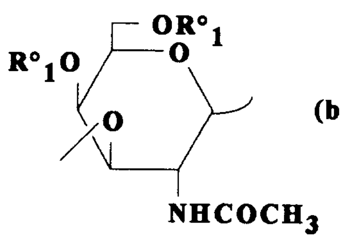

(b)°

- U° représente un groupe (c)° de formule :

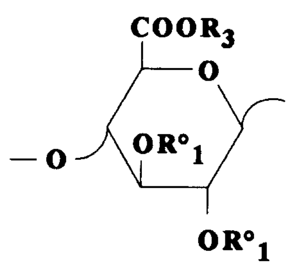

(c)°

ou un groupe (d)° de formule :

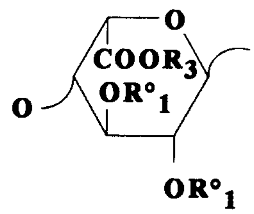

(d)°

ou le résidu du groupe (c)° ou du groupe (d)° après oxydation periodique suivie d'une $\beta$-élimination ou d'une hydrolyse acide ;

- A° représente un groupe $R°_1$, un groupe $R°_1$-(c)° un groupe $R°_1$-(d)°, ou un groupe (e)° de formule :

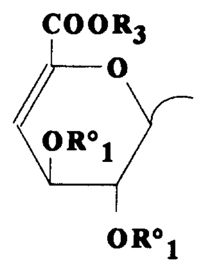

(e)°

ou le résidu du groupe (c)° ou du groupe (d)° après oxydation periodique suivie d'une $\beta$-élimination ou d'une hydrolyse acide ;

- B° représente un groupe $OR°_1$, un groupe (a)°-$OR°_1$, un groupe (a')°-$OR°_1$, un groupe (b)-°-$OR°_1$ ou un groupe (f)° de formule :

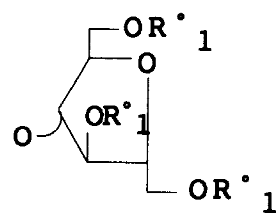

(f)°

ou un groupe (g)° de formule :

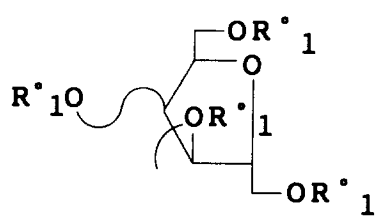

(g)°

ou représente les groupes (a)°, (a')°, (b)° auxquels reste accroché un résidu de (c)° ou de (d)° tel que présent après oxydation periodique suivie d'une $\beta$-élimination ou d'une hydrolyse acide ;

- $R_1$° représente H ou $SO_3^-$ ;
- $R_2$ représente $SO_3^-$ ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;

- n est un nombre entier de 1 à 80,

en un sel d'amine tertiaire ou en sel d'ammonium quaternaire dudit glycosaminoglycane soluble dans un solvant organique aprotique polaire ;

(2) on traite ce sel par un anhydride de formule :

$R_1$-O-$R_1$

dans laquelle $R_1$ représente un radical acyle, tel que défini dans la revendication 1, dans ledit solvant organique aprotique polaire, en présence de quantités catalytiques de pyridine ou d'une dialkylaminopyridine et d'un accepteur de protons à une température située entre 0 ° C et 100 ° C ;

(3) on précipite le produit ainsi obtenu par action d'une solution d'acétate de sodium dans l'éthanol et,

(4) on isole le glycosaminoglycane sélectivement O-acylé par dissolution dans l'eau du précipité ainsi obtenu et par dialyse, et l'on transforme éventuellement le sel de sodium du glycosaminoglycane sélectivement O-acylé ainsi obtenu, en un autre sel pharmaceutiquement acceptable.

**19.** Procédé selon la revendication 18, caractérisé en ce que le glycosaminoglycane mis en oeuvre dans l'étape (1) est choisi dans le groupe constitué par l'héparine, un mélange de fragments d'héparine ayant une masse moléculaire inférieure à 10.000 daltons, un mélange de fragments d'héparine ayant une masse moléculaire moyenne se situant entre 2.000 et 7.000 daltons, un mélange de fragments d'héparine ayant une masse moléculaire moyenne d'environ 4.500 daltons, un mélange de fragments d'héparine ayant une masse moléculaire d'environ 2.500 daltons ou un mélange de fragments d'héparine de même masse moléculaire.

**20.** Procédé selon la revendication 18, caractérisé en ce que le glycosaminoglycane mis en oeuvre dans l'étape (1) est de l'héparine ou une fraction ou un fragment d'héparine dépourvu de site de fixation à l'antithrombine III.

**21.** Procédé selon la revendication 18, caractérisé en ce que le glycosaminoglycane mis en oeuvre dans l'étape (1) est choisi parmi le groupe constitué par le dermatane sulfate et ses fragments.

**22.** Procédé selon l'une quelconque des revendications 18 à 21, caractérisé en ce que le sel du glycosaminoglycane de l'étape (1) est un sel de tributylamine ou un sel de tétrabutylammonium.

**23.** Procédé selon l'une quelconque des revendications 18 à 22, caractérisé en ce que, l'anhydride mis en oeuvre dans l'étape (2), est l'anhydride d'un acide alcanoïque contenant de 2 à 10 atomes de carbone.

**24.** Procédé selon l'une quelconque des revendications 18 à 23, caractérisé en ce que, l'étape (2) est conduite dans un solvant aprotique polaire choisi parmi le groupe constitué par le diméthylformamide, l'hexaméthylphosphorotriamide, la pyridine, ou un mélange de ces solvants entre eux ou avec le dichlorométhane.

**25.** Procédé selon l'une quelconque des revendications 18 à 24, caractérisé en ce que, comme accepteur de protons, lors de l'étape (2), l'on utilise une base choisie parmi le groupe constitué par la pyridine, la triéthylamine et la tributylamine.

**26.** Procédé selon l'une quelconque des revendications 18 à 25, caractérisé en ce que la dialyse de l'étape (4) est effectuée en présence d'une base faible.

**27.** Composition pharmaceutique caractérisée en ce qu'elle renferme à titre de substance active, une quantité efficace d'au moins un glycosaminoglycane sélectivement O-acylé selon l'une quelconque des revendications 1 à 17, en association avec un véhicule pharmaceutique.

**28.** Composition pharmaceutique selon la revendication 27 pour le traitement ou la prévention de maladies dues à des virus enveloppés.

**29.** Composition pharmaceutique selon la revendication 27, pour le traitement ou la prévention de maladies dues à des rétrovirus.

**Revendications pour l'Etat contractant suivant : ES**

**1.** Procédé de préparation de glycosaminoglycanes sélectivement O-acylés répondant à la formule (I) suivante :

**A-[G-U]$_n$-B** (I)

dans laquelle :

- G représente un groupe (a) de formule :

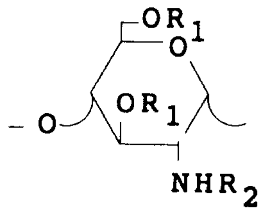

(a)

ou un groupe (a') de formule :

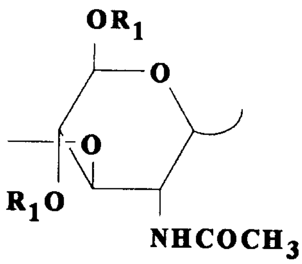

(a')

ou un groupe (b) de formule :

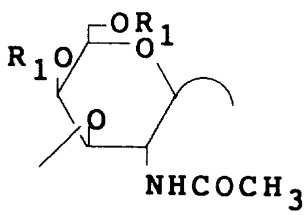

(b)

- U représente un groupe (c) de formule :

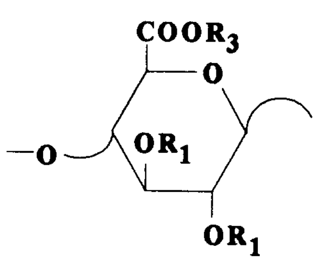

(c)

ou un groupe (d) de formule :

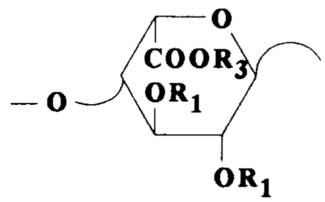

(d)

ou le résidu du groupe (c) ou du groupe (d) après oxydation periodique suivie d'une $\beta$-élimination ou d'une hydrolyse acide ;

- A représente un groupe $R_1$, un groupe $R_1$-(c), un groupe $R_1$-(d), ou un groupe (e) de formule :

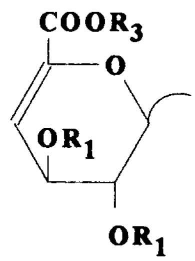

(e)

ou le résidu du groupe (c) ou du groupe (d) après oxydation periodique suivie d'une $\beta$-élimination ou d'une hydrolyse acide ;

- B représente un groupe O-$R_1$, un goupe (a)-O$R_1$, un groupe (a')-O$R_1$, un groupe (b)-O$R_1$ ou un groupe (f) de formule :

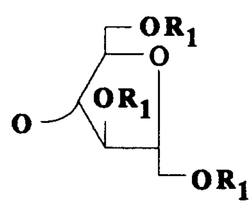

(f)

ou un groupe (g) de formule :

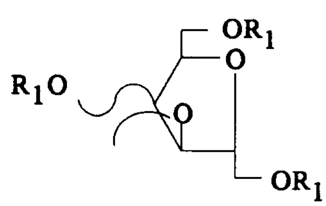

(g)

ou représente un groupe (a), un groupe (a'), ou un groupe (b) auxquels reste accroché un résidu de (c) ou de (d) tel que présent après oxydation periodique suivie d'une $\beta$-élimination ou d'une hydrolyse acide ;

- $R_1$ représente H, $SO_3^-$ , ou un radical acyle d'un acide carboxylique ou d'un acide dicarboxylique non $\alpha,\beta$-insaturé choisi parmi :
  - ♦ un groupe alcanoyle de 1 à 18 atomes de carbone ;
  - ♦ un groupe alcanoyle de 2 à 3 atomes de carbone substitué par :
    - • un groupe cycloalkyle de 3 à 7 atomes de carbone,
    - • un radical hydrocarboné aliphatique insaturé de 4 à 16 atomes de carbone ;
  - ♦ un groupe benzoyle éventuellement substitué par un ou plusieurs radicaux alkyles de 1 à 4 atomes de carbone, atomes d'halogène ou groupement $NO_2$ ou $OCH_3$ ;

♦ un groupe cycloalkyle (3-7 C) carbonyle ;

- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- n étant un nombre entier de 1 à 80,

$R_1$ étant acyle dans la proportion d'au moins 0,1 à 3 groupes acyles par unité disaccharidique, et leurs sels pharmaceutiquement acceptables,

caractérisé en ce que :

(1) on transforme un glycosaminoglycane choisi dans le groupe constitué par :

l'héparine, un mélange de fragments d'héparine ayant une masse moléculaire inférieure à 10.000 daltons, un mélange de fragments d'héparine ayant une masse moléculaire moyenne se situant entre 2.000 et 7.000 daltons, un mélange de fragments d'héparine ayant une masse moléculaire moyenne d'environ 4.500 daltons, un mélange de fragments d'héparine ayant une masse moléculaire moyenne d'environ 2.500 daltons, ou un mélange de fragments d'héparine de même masse moléculaire, une fraction ou un fragment d'héparine dépourvus de site de fixation à l'antithrombine III,

le dermatane sulfate et ses fragments,

en un sel d'amine tertiaire ou en un sel d'ammonium quaternaire soluble dans un solvant organique aprotique polaire ;

(2) on traite ce sel par un anhydride de formule :

$R_1$-O-$R_1$

dans laquelle $R_1$ représente un radical acyle, tel qu'indiqué précédemment, dans ledit solvant organique aprotique polaire, en présence de quantités catalytiques de pyridine ou d'une dialkylaminopyridine et d'un accepteur de protons à une température située entre 0°C et 100°C ;

(3) on précipite le produit ainsi obtenu par action d'une solution d'acétate de sodium dans l'éthanol et,

(4) on isole le glycosaminoglycane sélectivement O-acylé par dissolution dans l'eau du précipité ainsi obtenu et par dialyse, et l'on transforme éventuellement le sel de sodium du glycosaminoglycane sélectivement O-acylé ainsi obtenu en un autre sel pharmaceutiquement acceptable.

**2.** Procédé de préparation de glycosaminiglycanes sélectivement O-acylés selon la revendication 1, caractérisé en ce que l'on prépare des composés de formule II suivante :

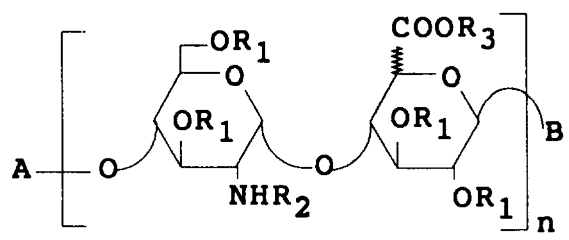

(II)

dans laquelle :

- A a les significations de la revendication 1 ;
- $R_1$ représente H et/ou $SO_3^-$ et/ou un radical acyle, tel que défini dans la formule (I) ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- B représente (a)-$OR_1$ ou (f), (a) et (f) étant tels que définis dans la revendication 1, ou $OR_1$, ou un groupe (a) auquel reste accroché un résidu de (c) ou de (d) tel que présent après oxydation periodique suivie d'une $\beta$-élimination ou d'une hydrolyse acide ;
- n est un nombre entier de 1 à 80, sous réserve que lorsque A représente $R_1$, un groupe $R_1$-(c) ou un groupe $R_1$-(d) et que B représente O-$R_1$, un groupe (a)-$OR_1$, ou un groupe (b)-$OR_1$, n soit un nombre entier de 1 à 16.

**3.** Procédé de préparation de glycosaminoglycanes sélectivement O-acylés selon la revendication 1, caractérisé en ce que l'on prépare des composés de formule II suivante :

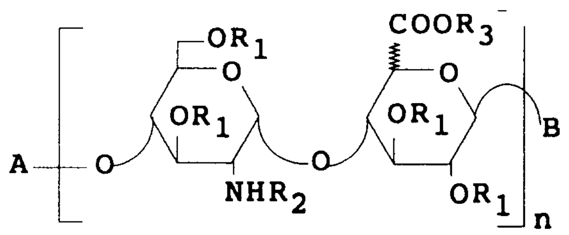

(II)

dans laquelle :

- A et B ont les significations de la revendication 1 ;
- $R_1$ représente H et/ou $SO_3^-$ et/ou un radical acyle d'un acide carboxylique ou dicarboxylique non $\alpha,\beta$-insaturé choisi parmi :
  - ♦ un groupe alcanoyle de 4 à 18 atomes de carbone ;
  - ♦ un groupe alcanoyle de 2 à 3 atomes de carbone substitué par :
    - un groupe cycloalkyle de 3 à 7 atomes de carbone,
    - un radical hydrocarboné aliphatique insaturé de 4 à 16 atomes de carbone ;
  - ♦ un groupe benzoyle éventuellement substitué par un ou plusieurs radicaux alkyles de 1 à 4 atomes de carbone, atomes d'halogène ou groupement $NO_2$ ou $OCH_3$ ;
  - ♦ un groupe cycloalkyle (3-7 C) carbonyle ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- n est un nombre entier de 1 à 80,

$R_1$ étant acyle dans la proportion d'au moins 0,5 à 2 groupes acyles par unité disaccharidique.

**4.** Procédé de préparation de glycosaminoglycanes sélectivement O-acylés selon les revendications 1 ou 2, caractérisé en ce que le glycosaminoglycane est choisi dans le groupe constitué par un mélange de fragments d'héparine ayant une masse moléculaire inférieure à 10.000 daltons, un mélange de fragments d'héparine ayant une masse moléculaire moyenne se situant entre 2.000 et 7.000 daltons, un mélange de fragments d'héparine ayant une masse moléculaire moyenne d'environ 4.500 daltons, un mélange de fragments d'héparine ayant une masse moléculaire moyenne d'environ 2.500 daltons, un mélange de fragments d'héparine de même masse moléculaire, le groupe acyle étant dans une proportion de 0,5 à 2 groupes par unité disaccharidique.

**5.** Procédé de préparation de glycosaminoglycanes sélectivement O-acylés selon la revendication 3, caractérisé en ce que le glycosaminiglycane est l'héparine, le groupe acyle étant dans une proportion d'au moins 0,5 à 2 groupes par unité disaccharidique.

**6.** Procédé de préparation de glycosaminoglycanes sélectivement O-acylés selon la revendication 5, caractérisé en ce que le groupe acyle est dans une proportion de 1 groupe acyle par unité disaccharidique.

**7.** Procédé de préparation de glycosaminoglycanes sélectivement O-acylés selon l'une quelconque des revendications 1 à 4, caractérisé en ce que l'on prépare des composés de formule III **suivante :**

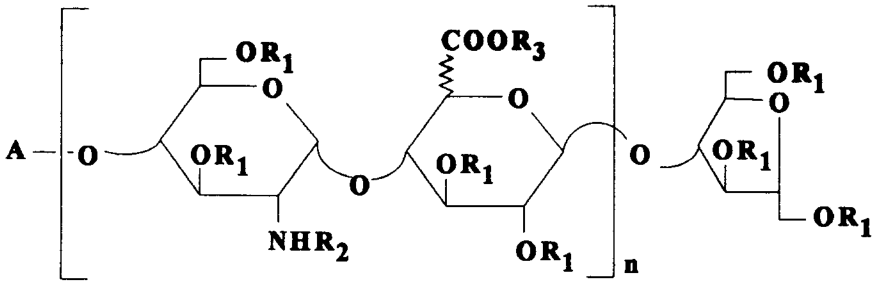

(III)

dans laquelle

- A représente $R_1$, ou $R_1$-(c) ou $R_1$-(d), tels que définis dans la revendication 1 ;
- $R_1$ représente H et/ou $SO_3^-$ et/ou un radical acyle, tel que défini dans la revendication 1 ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène et/ou un radical alkyle de 1 à 10 atomes de carbone, un radical phényl-alkyle de 7 à 12 atomes de carbone, ou un cation métallique alcalin ou alkalino-terreux ;
- n est un nombre entier de 3 à 12.

**8.** Procédé de préparation de glycosaminoglycanes sélectivement O-acylés selon l'une quelconque des revendications 1 à 4 caractérisé en ce que l'on prépare des composés de formule IV suivante :

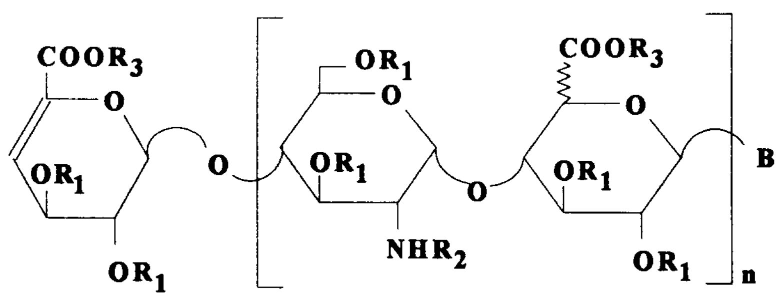

(IV)

dans laquelle :

- $R_1$ représente H et/ou $SO_3^-$ et/ou un radical acyle, tel que défini dans la revendication 1 ;
- $R_2$ représente $SO_3^-$ et/ou un radical acétyle, sous réserve que la proportion de N-acétyl glucosamine soit au plus égale à celle de l'héparine lorsque $R_1$ représente un radical acétyle ;
- $R_3$ représente un atome d'hydrogène et/ou un radical alkyle de 1 à 10 atomes de carbone, un radical phényl-alkyle de 7 à 12 atomes de carbone, ou un cation métallique alcalin ou alkalino-terreux ;
- B représente (a)-$OR_1$, tels que défini dans la revendication 1, ou $OR_1$ ;
- n est un nombre entier de 2 à 20.

**9.** Procédé de préparation de glycosaminoglycanes sélectivement O-acylés selon les revendications 1 ou 2, caractérisé en ce que le glycosaminoglycane choisi est l'héparine,
une fraction ou un fragment d'héparine dépourvus de site de fixation à l'antithrombine III.

**10.** Procédé de préparation de glycosaminiglycanes sélectivement O-acylés selon l'une quelconque des revendications 1, 2, 3 et 9, caractérisé en ce que l'on prépare des composés de formule V suivante :

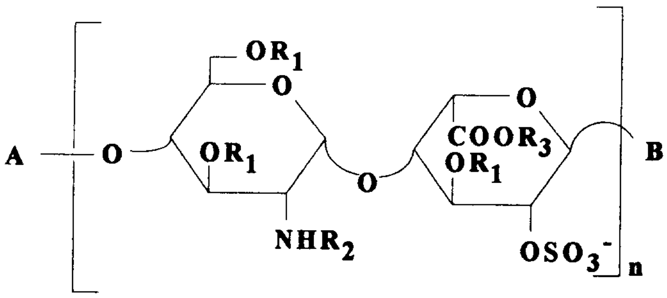

(V)

dans laquelle :

- A représente $R_1$-(c), $R_1$-(d), ou le résidu de (c) ou de (d) après oxydation periodique suivie d'une $\beta$-élimination ou d'une hydrolyse acide ;
- B représente un groupe (a)-$OR_1$, un groupe (a) auquel reste accroché un résidu de (c) ou de (d) tel que présent après oxydation periodique suivie d'une $\beta$-élimination ou d'une hydrolyse acide ;
- $R_1$ a les significations données dans la revendication 1 ;
- $R_2$ représente $SO_3^-$ ou un radical acétyle, la proportion de $SO_3^-$ étant d'environ 90 % ;
- $R_3$ représente un atome d'hydrogène ou un cation métallique alcalin ou alkalino-terreux ;
- n est un nombre entier de 1 à 80.

**11.** Procédé de préparation de glycosaminoglycanes sélectivement O-acylés selon l'une quelconque des revendications 1, 2, 3 et 9, caractérisé en ce que l'on prépare des composés de formule VI suivante :

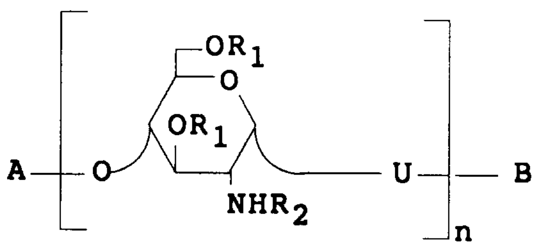

(VI)

dans laquelle :

- A représente $R_1$, $R_1$-(c), $R_1$-(d), ou le résidu de (c) ou de (d) après oxydation periodique suivie d'une $\beta$-élimination ;
- U représente :

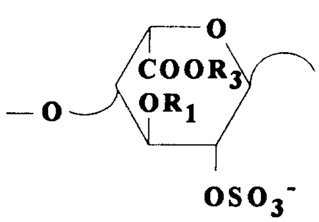

où à raison d'un motif pour deux chaînes au moins, un acide uronique (D-glucuronique ou L-iduronique) non sulfaté ouvert entre les carbones en positions 2 et 3, de formule :

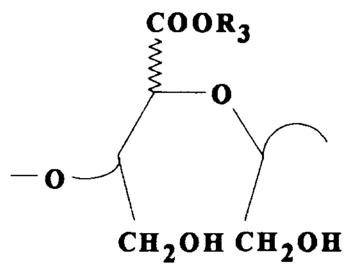

- B représente (a)-$OR_1$, ou $OR_1$ ou un groupe (a) auquel reste accroché un résidu de (c) ou de (d) tel que présent après oxydation periodique suivie d'une $\beta$-élimination ;
- $R_1$ a les significations données dans la revendication 1 ;
- $R_2$ représente $SO_3^-$ ou un radical acétyle, la proportion de $SO_3^-$ étant d'environ 90 % ;
- $R_3$ représente un atome d'hydrogène ou un cation métallique alcalin ou alkalino-terreux ;
- n est un nombre entier de 2 à 18.

**12.** Procédé de préparation de glycosaminoglycanes sélectivement O-acylés selon l'une quelconque des revendications 1,2, 3 et 9 caractérisé en ce que l'on prépare des composés de formule VI dans laquelle:
- n est un nombre entier de 7 à 15 pour les espèces majoritaires,
- $R_1$ est un radical alcanoyle de 2 à 10 atomes de carbone.

**13.** Procédé de préparation de glycosaminoglycanes sélectivement O-acylés selon les revendications 11 et 12, caractérisé en ce que l'on prépare des composés constitués d'un mélange de fragments de même masse moléculaire, dans lesquels n est un nombre entier de 2 à 12, et $R_1$ est un radical alcanoyle de 4 à 10 atomes de carbone.

**14.** Procédé de préparation de glycosaminiglycanes sélectivement O-acylés selon l'une quelconque des revendications 1,2, 3 et 9, caractérisé en ce que l'on prépare des composés de formule VII suivante :

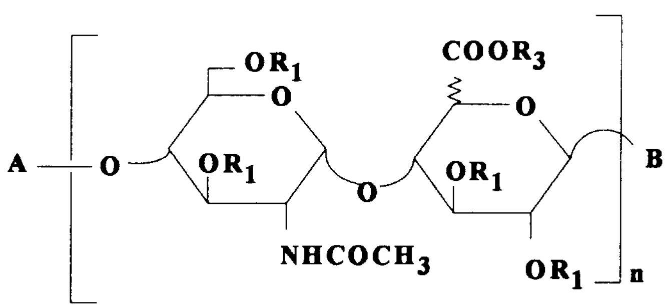

(VII)

dans laquelle :
- A représente $R_1$, $R_1$-(c), ou $R_1$-(d), tels que définis dans la formule (I) ;
- $R_1$ représente un radical alcanoyle de 2 à 18 atomes de carbone ;
- $R_3$ représente un atome d'hydrogène ou un cation métallique alcalin ou alkalino-terreux ;
- B représente (a)-$OR_1$ tel que défini dans la formule (I), ou $OR_1$ ;
- n est un nombre entier de 1 à 80.

**15.** Procédé de préparation de glycosaminoglycanes sélectivement O-acylés selon la revendication 1, caractérisé en ce que l'on prépare des composés de formule VIII suivante :

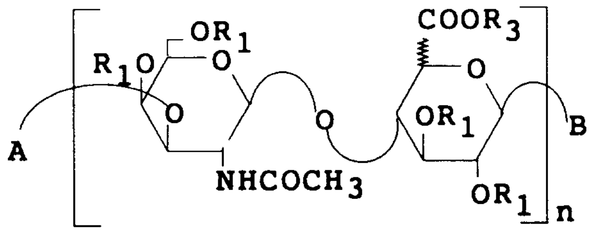

(VIII)

dans laquelle :

- A a les significations de la revendication 1 ;
- $R_1$ représente H et/ou $SO_3^-$ et/ou un radical acyle, tel que défini dans la revendication 1 ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- B représente (b)-$OR_1$, ou (g), tels que définis dans la formule (I), ou $OR_1$, ou un groupe (b) auquel reste accroché un résidu de (c) ou de (d) tel que présent après oxydation periodique suivie d'une $\beta$-élimination ou d'une hydrolyse acide ;
- n est un nombre entier de 1 à 80.

**16.** Procédé de préparation de glycosaminoglycanes sélectivement O-acylés selon les revendications 1 ou 15,caractérisé en ce que le glycosaminoglycane est choisi parmi le groupe constitué par le dermatane sulfate et-ses fragments, le groupe acyle étant dans une proportion d'au moins 0,1 à 3 groupes par unité disaccharidique.

**17.** Procédé de préparation de glycosaminoglycanes sélectivement O-acylés selon l'une quelconque des revendications 1 à 16, dans lequel on prépare des glycosaminoglycanes où $R_1$ est un radical alcanoyle de 4 à 10 atomes de carbone.

**18.** Procédé de préparation de glycosaminoglycanes sélectivement O-acylés selon l'une quelconque des revendications 1 à 17 caractérisé en ce que :

(1) on transforme un glycosaminoglycane de formule IX :

$A°\text{-}[G°\text{-}U°]_n\text{-}B°$ (IX)

dans laquelle :

- G° représente un groupe (a)° de formule :

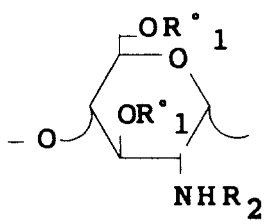

(a)°

ou un groupe (a')° de formule :

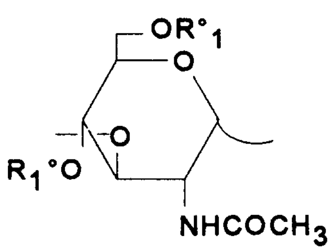

(a')°

ou un groupe (b)° de formule :

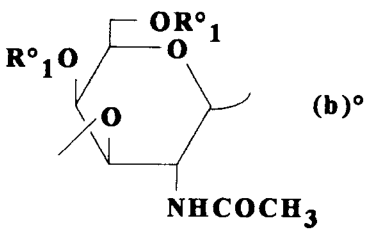

(b)°

- U° représente un groupe (c)° de formule :

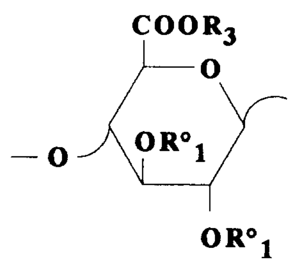

(c)°

ou un groupe (d)° de formule :

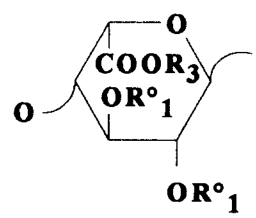

(d)°

ou le résidu du groupe (c)° ou du groupe (d)° après oxydation periodique suivie d'une β-élimination ou d'une hydrolyse acide ;

- A° représente un groupe R°$_1$, un groupe R°$_1$-(c)° un groupe R°$_1$-(d)°, ou un groupe (e)° de formule :

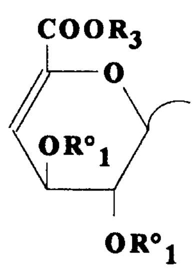

(e)°

ou le résidu du groupe (c)° ou du groupe (d)° après oxydation periodique suivie d'une β-élimination ou d'une hydrolyse acide ;

- B° représente un groupe OR°$_1$, un groupe (a)°-OR°$_1$, un groupe (a')°-OR°$_1$, un groupe (b)-°-OR°$_1$ ou un groupe (f)° de formule :

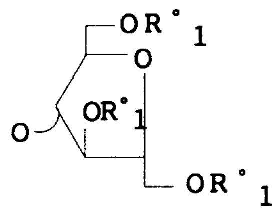

(f)°

ou un groupe (g)° de formule :

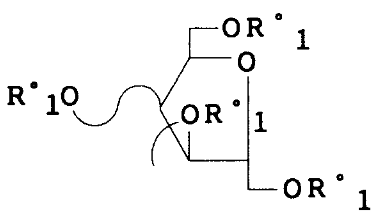

(g)°

ou représente les groupes (a)°, ( a')°, (b)° auxquels reste accroché un résidu de (c)° ou de (d)° tel que présent après oxydation periodique suivie d'une $\beta$-élimination ou d'une hydrolyse acide ;

- $R_1$° représente H ou $SO_3^-$ ;
- $R_2$ représente $SO_3^-$ ou un radical acétyle, sous réserve que la proportion de N-acétyl glucosamine soit au plus égale à celle de l'héparine lorsque $R_1$ représente un radical acétyle ;
- $R_3$ représente un atome d'hydrogène, un radical alkyle de 1 à 10 atomes de carbone, un radical phényl-alkyle de 7 à 12 atomes de carbone, ou un cation métallique alcalin ou alkalino-terreux ;
- n est un nombre entier de 1 à 80,

en un sel d'amine tertiaire ou en sel d'ammonium quaternaire dudit glycosaminoglycane soluble dans un solvant organique aprotique polaire ;
(2) on traite ce sel par un anhydride de formule :

$R_1$-O-$R_1$

dans laquelle $R_1$ représente un radical acyle, tel que défini dans la revendication 1, dans ledit solvant organique aprotique polaire, en présence de quantités catalytiques de pyridine ou d'une dialkylaminopyridine et d'un accepteur de protons à une température située entre 0°C et 100°C ;
(3) on précipite le produit ainsi obtenu par action d'une solution d'acétate de sodium dans l'éthanol et,
(4) on isole le glycosaminoglycane sélectivement O-acylé par dissolution dans l'eau du précipité ainsi obtenu et par dialyse, et l'on transforme éventuellement le sel de sodium du glycosaminoglycane sélectivement O-acylé ainsi obtenu, en un autre sel pharmaceutiquement acceptable.

**19.** Procédé selon la revendication 18, caractérisé en ce que le glycosaminoglycane mis en oeuvre dans l'étape (1) est choisi dans le groupe constitué par l'héparine, un mélange de fragments d'héparine ayant une masse moléculaire inférieure à 10.000 daltons, un mélange de fragments d'héparine ayant une masse moléculaire moyenne se situant entre 2.000 et 7.000 daltons, un mélange de fragments d'héparine ayant une masse moléculaire moyenne d'environ 4.500 daltons, un mélange de fragments d'héparine ayant une masse moléculaire d'environ 2.500 daltons ou un mélange de fragments d'héparine de même masse moléculaire.

**20.** Procédé selon la revendication 18, caractérisé en ce que le glycosaminoglycane mis en oeuvre dans l'étape (1) est de l'héparine ou une fraction ou un fragment d'héparine dépourvu de site de fixation à l'antithrombine III.

**21.** Procédé selon la revendication 18, caractérisé en ce que le glycosaminoglycane mis en oeuvre dans l'étape (1) est choisi parmi le groupe constitué par le dermatane sulfate et ses fragments.

**22.** Procédé selon l'une quelconque des revendications 18 à 21, caractérisé en ce que le sel du glycosaminoglycane de l'étape (1) est un sel de tributylamine ou un sel de tétrabutylammonium.

**23.** Procédé selon l'une quelconque des revendications 18 à 22, caractérisé en ce que, l'anhydride mis en oeuvre dans l'étape (2), est l'anhydride d'un acide alcanoïque contenant de 2 à 10 atomes de carbone.

**24.** Procédé selon l'une quelconque des revendications 18 à 23, caractérisé en ce que, l'étape (2) est conduite dans un solvant aprotique polaire choisi parmi le groupe constitué par le diméthylformamide, l'hexaméthylphosphorotriamide, la pyridine, ou un mélange de ces solvants entre eux ou avec le dichlorométhane.

**25.** Procédé selon l'une quelconque des revendications 18 à 24, caractérisé en ce que, comme accepteur de protons, lors de l'étape (2), l'on utilise une base choisie parmi le groupe constitué par la pyridine, la triéthylamine et la tributylamine.

**26.** Procédé selon l'une quelconque des revendications 18 à 25, caractérisé en ce que la dialyse de l'étape (4) est effectuée en présence d'une base faible.

**Claims**
**Claims for the following Contracting States : AT, BE, CH, DE, FR, GB, GR, IT, LI, LU, NL, SE**

**1.** Selectively O-acylated glycosaminoglycans according to the following formula (I):

$A\text{-}[G\text{-}U]_n\text{-}B$ (I)

in which:
- G represents a group (a) of formula:

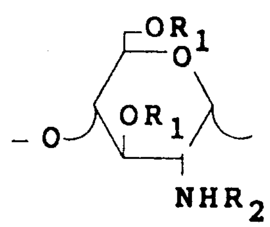

(a)

or a group (a') of formula:

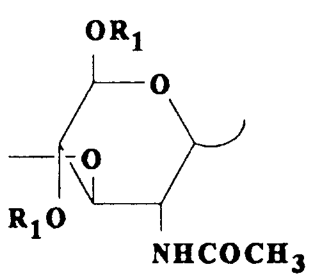

(a')

or a group (b) of formula:

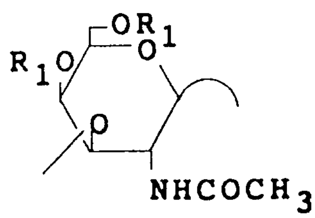

(b)

- U represents a group (c) of formula:

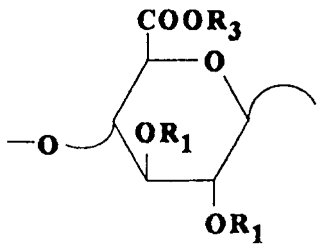

(c)

or a group (d) of formula:

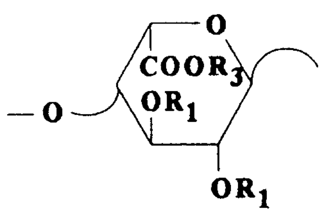

(d)

or the residue of the group (c) or group (d) after periodic oxidation, followed by a $\beta$-elimination or an acid hydrolysis;

- A represents a group $R_1$, a group $R_1$-(c), a group $R_1$-(d), or a group (e) of formula:

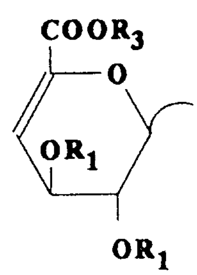

(e)

or the residue of the group (c) or of the group (d) after periodic oxidation followed by a $\beta$-elimination or an acid hydrolysis;

- B represents a group O-$R_1$, or a group (a)-$OR_1$, or a group (a')-$OR_1$, or a group (b) -$OR_1$ or a group (f) of formula:

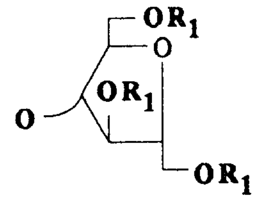

(f)

or a group (g) of formula:

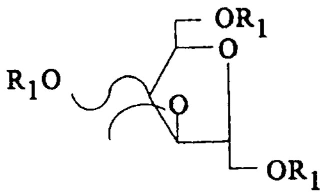

(g)

or represents a group (a), a group (a'), or a group (b) to which remains attached a residue of (c) or of (d) such that as that present after periodic oxidation, followed by a $\beta$-elimination of an acid hydrolysis;

- $R_1$ represents H, $SO_3^-$ or an acyl radical of a carboxylic acid or a non-$\alpha$, $\beta$- unsaturated dicarboxylic acid chosen from among:
  - an alkanoyl group of 1 to 18 carbon atoms;
  - an alkanoyl group of 2 to 3 carbon atoms substituted by
    - a cycloalkyl group of 3 to 7 carbon atoms,
    - an unsaturated aliphatic hydrocarbon radical of 4 to 16 carbon atoms;
  - a benzoyl group possibly substituted by one or more alkyl radicals of 1 to 4 carbon atoms, halogen atoms of $NO_2$ or $OCH_3$ groups;
  - a cycloalkyl (3-7 C) carbonyl;
- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7 to 12 carbon atoms, or an alkali or alkaline earth metal cation;
- n is an integer between 1 and 80;

$R_1$ being acyl in a proportion of 0.1 to 3 acyl groups per disaccharide unit, and their pharmaceutically acceptable salts,

able to be obtained from a glycosaminoglycan chosen from a group constituted by:

heparin, a mixture of heparin fragments having a molecular mass of less than 10,000 daltons, a mixture of heparin fragments having a mean molecular mass between 2,000 and 7,000 daltons, a mixture of heparin fragments having a mean molecular mass of about 4,500 dalton, a mixture of heparin fragments having a mean molecular mass of about 2,500 daltons, or a mixture of heparin fragments of the same molecular mass, a fraction or a fragment of heparin lacking the binding site for antithrombin III,

the dermatan sulphate and its fragments,

(1) is converted into a tertiary amine salt or a quaternary ammonium salt soluble of a polar aprotic organic solvent;

(2) this salt is treated by an anhydride of the formula:

$R_1$-O-$R_1$

in which $R_1$ represents an acyl radical, as previously indicated, in the said polar aprotic organic solvent, in the presence of catalytic amounts of pyridine or a dialkylaminopyridine and a proton acceptor a a temperature situated between 0 ° C and 100 ° C;

(3) the product thus obtained is precipitated by the action of a solution sodium acetate in ethanol and,

(4) the selectively O-acylated glycosaminoglycan is isolated by dissolving the precipitate thus obtained in water and by dialysis, and if required , the sodium salt of the selectively O-acylated glycosaminoglycan thus obtained is converted into another pharmaceutically acceptable salt.

**2.** Selectively O-acylated glycosaminoglycans according to claim 1, characterised in that they correspond to the following formula II:

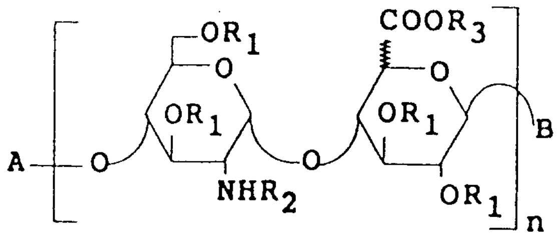

(II)

in which

- A has the meanings of claim 1;
- $R_1$ represents H and/or $SO_3^-$ and/or an acyl radical, such as that defined in claim 1;
- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7-12 carbon atoms, or an alkali or alkaline earth metal cation;
- B represents (a)-$OR_1$ or (f), (a) and (f) being such as defined in claim 1, or $OR_1$, or a group (a) to which remains attached a residue of (c) or of (d) such as that present after periodic oxidation followed by a $\beta$-elimination of an acid hydrolysis;
- n is an integer between 1 and 80, provided that when A represents $R_1$, a group $R_1$-(c) or a group $R_1$-(d) and when B represents O-$R_1$, a group (a)-$OR_1$, or a group (b)-$OR_1$, n is an integer between 1 and 16.

**3.** Selectively O-acylated glycosaminoglycans according to claim 1, characterised in that they correspond to the following formula (II):

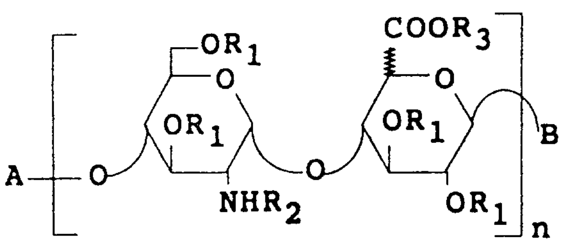

(II)

in which:

- A and B have their meanings of claim 1:
- $R_1$ represents H, $SO_3^-$ or an acyl radical of a carboxylic acid or a non-$\alpha$, $\beta$- unsaturated dicarboxylic acid chosen from among:
  - an alkanoyl group of 4 to 18 carbon atoms;
  - an alkanoyl group of 2 to 3 carbon atoms substituted by
    - a cycloalkyl group of 3 to 7 carbon atoms,
    - an unsaturated aliphatic hydrocarbon radical of 4 to 16 carbon atoms;
  - a benzoyl group possibly substituted by one or more alkyl radicals of 1 to 4 carbon atoms, halogen atoms of $NO_2$ or $OCH_3$ groups;
  - a cycloalkyl (3-7 C) carbonyl;
- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7 to 12 carbon atoms, or an alkali or alkaline earth metal cation;
- n is an integer between 1 and 80;

$R_1$ being acyl in a proportion of at least 0.5 to 2 acyl groups per disaccharide unit.

4. Selectively O-acylated glycosaminoglycans according to claims 1 or 2, characterised in that the glycosaminoglycan is chosen from a group constituted by: a mixture of heparin fragments having a molecular mass of less than 10,000 daltons, a mixture of heparin fragments having a mean molecular mass between 2,000 and 7,000 daltons, a mixture of heparin fragments having a mean molecular mass of about 4,500 dalton, a mixture of heparin fragments having a mean molecular mass of about 2,500 daltons, or a mixture of heparin fragments of the same molecular mass, the acyl group being in the proportion 0.5 to 2 groups per disaccharide unit.

5. Selectively O-acylated glycosaminoglycans according to claim 3, characterised in that the glycosaminoglycan is heparin, the acyl group being in the proportion of at least 0.5 to 2 groups per disaccharide unit.

6. Selectively O-acylated glycosaminoglycans according to claim 5, characterised in that the acyl group is in the proportion of 1 acyl group per disaccharide unit.

7. Selectively O-acylated glycosaminoglycans according to any of claims 1 to 4, characterised in that they corresponds to the following formula (III):

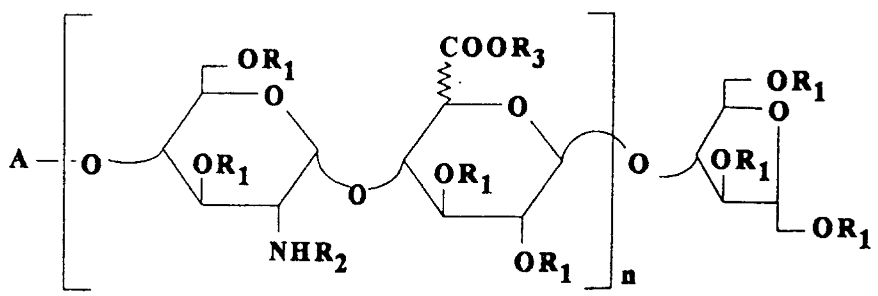

(III)

in which:

- A represents $R_1$, or $R_1$-(c) or $R_1$-(d), such as defined in claim 1;
- $R_1$ represents H and/or $SO_3^-$ and/or an acyl radical, such as that defined in claim 1;
- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom and/or an alkyl radical of 1 to 10 carbon atoms, a phenyl-alkyl radical of 7-12 carbon atoms, or an alkali or alkaline earth metal cation;
- n is an integer between 3 and 12.

8. Selectively O-acylated glycosaminoglycans according to claims 1 to 4 characterised in that they correspond to the following formula IV:

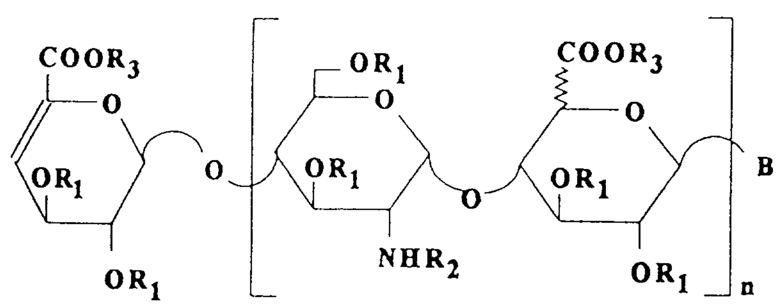

(IV)

in which:

- $R_1$ represents H and/or $SO_3^-$ and/or an acyl radical, such as that defined in claim 1;
- $R_2$ represents $SO_3^-$ and/or an acetyl radical, provided that the proportion of N-acetyl glucosamine does not exceed that in heparin when $R_1$ represents an acetyl radical;

- $R_3$ represents a hydrogen atom, an alkyl radical of 1 to 10 carbon atoms, a phenyl-alkyl radical of 7-12 carbon atoms, or an alkali or alkaline earth metal cation;
- B represents (a)-$OR_1$, such as defined in claim 1, or $OR_1$;
- n is an integer between 2 and 20.

**9.** Selectively O-acylated glycosaminoglycans according to claims 1 or 2, characterised in that the glycosaminoglycan chosen is heparin, a heparin fraction or fragment lacking the binding site for antithrombin III.

**10.** Selectively O-acylated glycosaminoglycans according to any of claims 1, 2, 3 and 9, characterised in that they correspond to the following formula V:

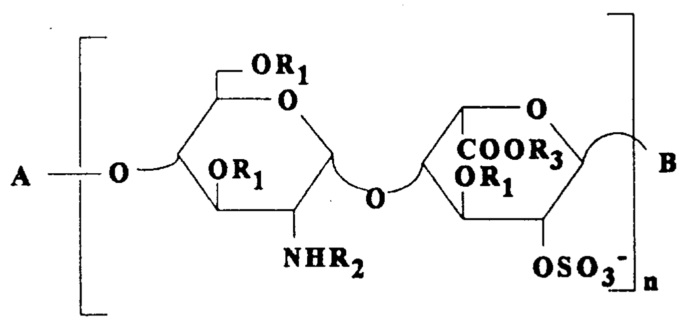

(V)

in which
- A represents $R_1$-(c), $R_1$-(d), or the residue of (c) or (d) after periodic oxidation followed by a $\beta$-elimination or an acid hydrolysis;
- B represents (a)-$OR_1$ or a group (a) to which remains attached a residue of (c) or of (d) such as that present after periodic oxidation followed by a $\beta$-elimination of an acid hydrolysis;
- $R_1$ has the meanings given in claim 1;
- $R_2$ represents $SO_3^-$ or an acetyl radical, the proportion of $SO_3^-$ being about 90%;
- $R_3$ represents a hydrogen atom or an alkali or alkaline earth metal cation;
- n is an integer between 1 and 80.

**11.** Selectively O-acylated glycosaminoglycans according to claims 1,2,3 and 9, characterised in that they correspond to the following formula VI:

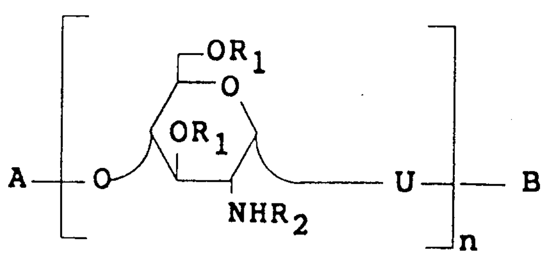

(VI)

in which:
- A represents $R_1$, or $R_1$-(c) or $R_1$-(d), or the residue of (c) or (d) after periodic oxidation followed by a $\beta$-elimination;

- U represents:

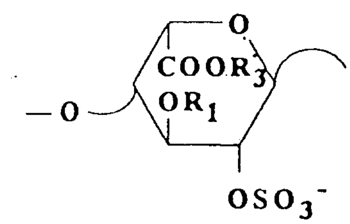

or in a ratio of one residue for at least two chains, a non-sulphated uronic acid (D-glucuronic or L-iduronic) open between the carbon atoms in positions 2 and 3, of formula:

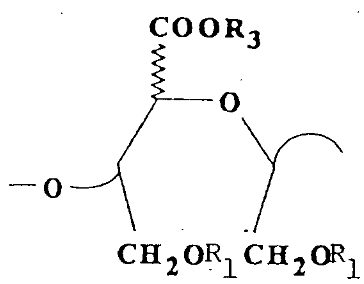

- B represents (a)-$OR_1$, or $OR_1$ or a group (a) to which remains attached a residue of (c) or of (d) such as that present after periodic oxidation followed by a $\beta$-elimination of an acid hydrolysis;
- $R_1$ has the meanings given in claim 1;
- $R_2$ represents $SO_3^-$ or an acetyl radical, the proportion of $SO_3^-$ being about 90%;
- $R_3$ represents a hydrogen atom or an alkali or alkaline earth metal cation;
- n is an integer between 2 and 18.

**12.** Selectively O-acylated glycosaminoglycans according to any of claims 1,2,3 and 9, characterised in that they correspond to formula VI in which:
- n is an integer between 7 and 15 for the major species,
- $R_1$ is an alkanoyl radical of 2 to 10 carbon atoms.

**13.** Selectively O-acylated glycosaminoglycans according to claims 11 and 12, characterised in that they comprise a mixture of fragments of the same molecular mass, in which n is an integer between 2 and 12, and $R_1$ is an alkanoyl radical of 4 to 10 carbon atoms.

**14.** Selectively O-acylated glycosaminoglycans according to any of claims 1,2,3 and 9, characterised in that they correspond to the following formula VII:

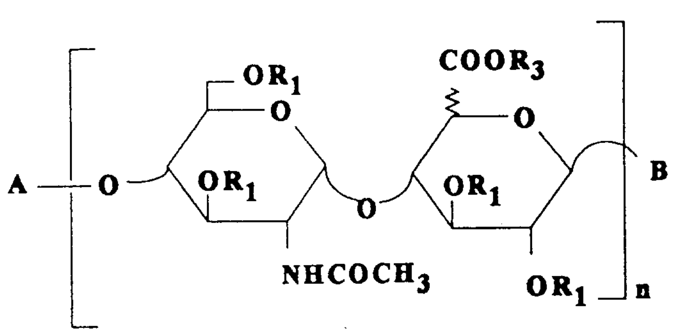

(VII)

in which:
- A represents $R_1$-(c), $R_1$-(d), such as defined in formula (I);
- $R_1$ represents an alkanoyl radical of 2 to 18 carbon atoms;

- $R_3$ represents a hydrogen atom or an alkali or alkaline earth metal cation;
- B represents (a)-$OR_1$ such as defined in formula (I), or $OR_1$
- n is an integer between 1 and 80.

**15.** Selectively O-acylated glycosaminoglycans according to claim 1 characterised in that they correspond to the following formula VIII:

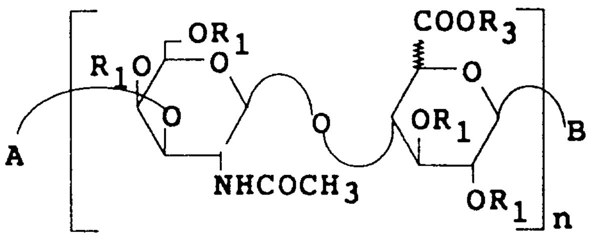

(VIII)

in which:
- A has the meanings of claim 1;
- $R_1$ represents H and/or $SO_3^-$ and/or an acyl radical, such as that defined in claim 1;
- $R_3$ represents a hydrogen atom, an alkyl radical of 1 to 10 carbon atoms, a phenyl-alkyl radical of 7-12 carbon atoms, or an alkali or alkaline earth metal cation;
- B represents (a)-$OR_1$, or (g), such as defined in formula (I), or $OR_1$, or a group (b) to which remains attached a residue of (c) or of (d) such as that present after periodic oxidation followed by a $\beta$-elimination of an acid hydrolysis;
- n is an integer between 1 and 80.

**16.** Selectively O-acylated glycosaminoglycans according to claims 1 or 15, characterised in that the glycosaminoglycan is chosen from among the group consisting of dermatan sulphate and its fragments, the acyl group being in the proportion of at least 01. to 3 groups per disaccharide unit.

**17.** Selectively O-acylated glycosaminoglycans according to any of claims 1 to 16, in which $R_1$ is an alkanoyl radical of 4 to 10 carbon atoms.

**18.** Process for the preparation of selectively O-acylated glycosaminoglycans according to claims 1 to 17 characterised in that:

(1) a glycosaminoglycan of formula IX is converted:

$A^\circ\text{-}[G^\circ\text{-}U^\circ]_n\text{-}B^\circ$ (IX)

in which:
- $G^\circ$ represents a group $(a)^\circ$ of formula:

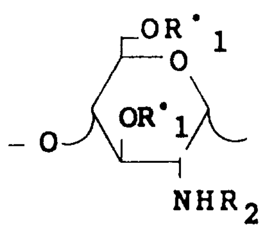

(a)°

or a group $(a')^\circ$ of formula:

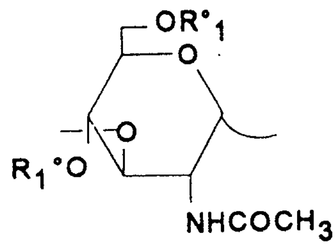

(a')°

or a group (b)° of formula:

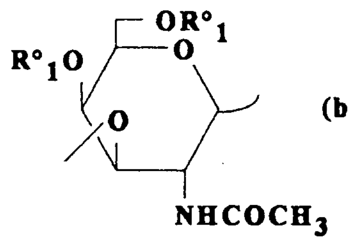

(b)°

- U° represents a group (c)° of formula:

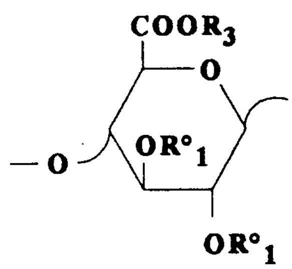

(c)°

or a group (d)° of formula:

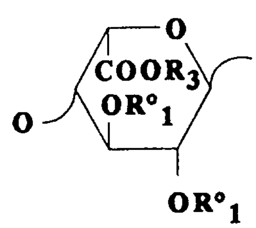

(d)°

or the residue of group (c)° or of group (d)° after periodic oxidation followed by a $\beta$-elimination or an acid hydrolysis;

- $A^\circ$ represents a group $R^\circ_1$, a group $R^\circ_1$-(c)°, a group $R^\circ_1$-(d)°, or a group (e)° of formula:

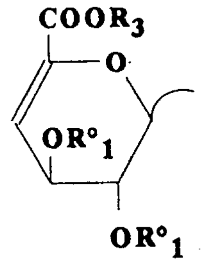

(e)°

or the residue of group (c)° or of group (d)° after periodic oxidation followed by a $\beta$-elimination or an acid hydrolysis;

- $B^\circ$ represents a group $OR^\circ_1$, a group (a)°-$OR^\circ_1$, a group (a')°-$OR^\circ_1$ or a group (f)° of formula:

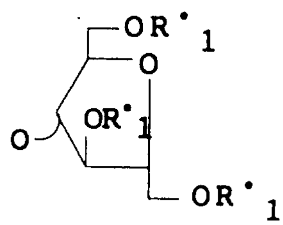

(f)°

or a group (g)° of formula:

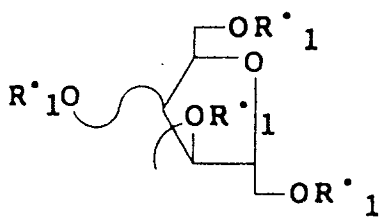

(g)°

or represents the groups (a)°, (a')°, (b)° to which remain attached a residue of (c)° or (d)° such as present after periodic oxidation followed by a $\beta$-elimination or an acid hydrolysis;

- $R_1$ ° represents H or $SO_3^-$ ;
- $R_2$ represents $SO_3^-$ or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7-12 carbon atoms, or an alkali or alkaline earth metal cation;
- n is an integer between 1 and 80, into a tertiary amine salt or a quaternary ammonium salt soluble of a polar aprotic organic solvent;

(2) this salt is treated by an anhydride of the formula:

$R_1$-O-$R_1$

in which $R_1$ represents an acyl radical, as such as that defined in claim 1, in the said polar aprotic organic solvent, in the presence of catalytic amounts of pyridine or a dialkylaminopyridine and a proton acceptor a a temperature situated between 0 °C and 100 °C;

(3) the product thus obtained is precipitated by the action of a solution sodium acetate in ethanol and,

(4) the selectively O-acylated glycosaminoglycan is isolated by dissolving the precipitate thus obtained in water and by dialysis, and if required , the sodium salt of the selectively O-acylated glycosaminoglycan thus obtained is converted into another pharmaceutically acceptable salt.

19. Process according to claim 18, characterised in that the glycosaminoglycan used in step (1) is chosen from the group constituted by heparin, a mixture of heparin fragments having a molecular mass of less than 10,000 daltons, a mixture of heparin fragments having a mean molecular mass between 2,000 and 7,000 daltons, a mixture of heparin fragments having a mean molecular mass of about 4,500 daltons, a mixture of heparin fragments having a mean molecular mass of about 2,500 daltons, or a mixture of heparin fragments of the same molecular mass.

20. Process according to claim 18, characterised in that the glycosaminoglycan used in step (1) is heparin or a fraction or fragment of heparin lacking the binding site for antithrombin III.

21. Process according to claim 18, characterised in that the glycosaminoglycan used in step (1) is chosen from the group constituted by dermatan sulphate and its fragments.

22. Process according to any of claims 18 or 21, characterised in that the glycosaminoglycan used in step (1) is a salt of tributylamine or tetrabutylammonium.

23. Process according to any of claims 18 to 22, characterised in that the anhydride used in step (2) is the anhydride of an alkanoic acid containing 2 to 10 carbon atoms.

24. Process according to claims 18 to 23, characterised in that the step (2) is conducted in a polar aprotic solvent chosen from among the group constituted by dimethylformamide, hexamethylphosphorotriamide, pyridine, or a mixture of these solvents with or without dichloromethane.

25. Process according to any of claims 18 to 24, characterised in that, as proton acceptors in step (2), a base is chosen from among the group constituted by pyridine, triethylamine and tributylamine.

26. Process according to any of claims 18 to 25, characterised in that the dialysis in step (4) is performed in the presence of a weak base.

27. Pharmaceutical composition characterised in that it contains as an active substance, an efficacious amount of at least one selectively O-acylated glycosaminoglycan according to any of claims 1 to 17, in combination with a pharmaceutical vehicle.

28. Pharmaceutical composition according to claim 27 for the treatment or prevention of illnesses caused by enveloped viruses.

29. Pharmaceutical composition according to claim 27, for the treatment or prevention of illnesses caused by retroviruses.

**Claims for the following Contracting State : ES**

1. Process for the preparation of selectively O-acylated glycosaminoglycans according to the following formula (I):

A-[G-U]$_n$-B (I)

in which:
- G represents a group (a) of formula:

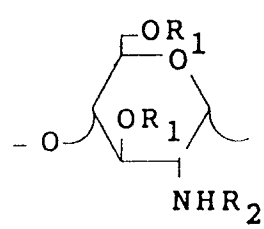

(a)

or a group (a') of formula:

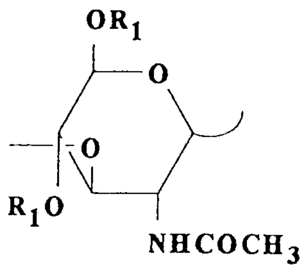

(a')

or a group (b) of formula:

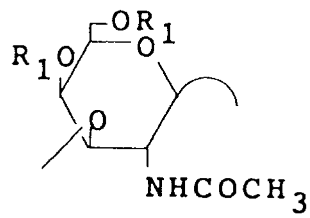

(b)

- U represents a group (c) of formula:

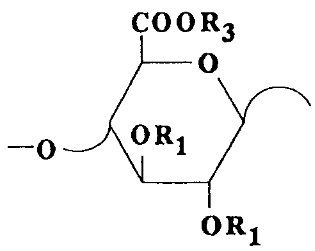

(c)

or a group (d) of formula:

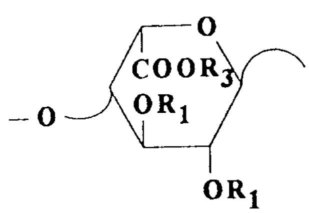

(d)

of the residue of the group (c) or group (d) after periodic oxidation, followed by a $\beta$-elimination or an acid hydrolysis;

- A represents a group $R_1$, a group $R_1$-(c), a group $R_1$-(d), or a group (e) of formula:

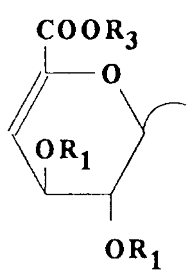

(e)

or the residue of the group (c) or of the group (d) after periodic oxidation followed by a $\beta$-elimination or an acid hydrolysis;

- B represents a group O-$R_1$, or a group (a)-$OR_1$, or a group (a')-$OR_1$, or a group (b) -$OR_1$ or a group (f) of formula:

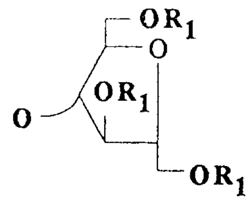

(f)

or a group (g) of formula:

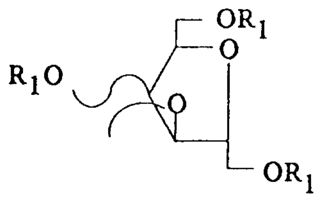

(g)

or represents a group (a), a group (a'), or a group (b) to which remains attached a residue of (c) or of (d) such that as that present after periodic oxidation, followed by a $\beta$-elimination of an acid hydrolysis;

- $R_1$ represents H, $SO_3^-$ or an acyl radical of a carboxylic acid or a non-$\alpha$, $\beta$- unsaturated dicarboxylic acid chosen from among:
  - an alkanoyl group of 1 to 18 carbon atoms;
  - an alkanoyl group of 2 to 3 carbon atoms substituted by
    - a cycloalkyl group of 3 to 7 carbon atoms,
    - an unsaturated aliphatic hydrocarbon radical of 4 to 16 carbon atoms;
  - a benzoyl group possibly substituted by one or more alkyl radicals of 1 to 4 carbon atoms, halogen atoms of $NO_2$ or $OCH_3$ groups;
  - a cycloalkyl (3-7 C) carbonyl;
- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7 to 12 carbon atoms, or an alkali or alkaline earth metal cation;
- n is an integer between 1 and 80;

$R_1$ being acyl in a proportion of 0.1 to 3 acyl groups per disaccharide unit, and their pharmaceutically acceptable salts,
characterised in that:

(1) a glycosaminoglycan chosen from a group constituted by:
heparin, a mixture of heparin fragments having a molecular mass of less than 10,000 daltons, a mixture of heparin fragments having a mean molecular mass between 2,000 and 7,000 daltons, a mixture of heparin fragments having a mean molecular mass of about 4,500 dalton, a mixture of heparin fragments having a mean molecular mass of about 2,500 daltons, or a mixture of heparin fragments of the same molecular mass, a fraction or a fragment of heparin lacking the binding site for antithrombin III,
the dermatan sulphate and its fragments,
is converted into a tertiary amine salt or a quaternary ammonium salt soluble of a polar aprotic organic solvent;

(2) this salt is treated by an anhydride of the formula:

$R_1$-O-$R_1$

in which $R_1$ represents an acyl radical, as previously indicated, in the said polar aprotic organic solvent, in the presence of catalytic amounts of pyridine or a dialkylaminopyridine and a proton acceptor a a temperature situated between 0 ° C and 100 ° C;

(3) the product thus obtained is precipitated by the action of a solution sodium acetate in ethanol and,

(4) the selectively O-acylated glycosaminoglycan is isolated by dissolving the precipitate thus obtained in water and by dialysis, and if required , the sodium salt of the selectively O-acylated glycosaminoglycan thus obtained is converted into another pharmaceutically acceptable salt.

**2.** Process for the preparation of selectively O-acylated glycosaminoglycans according to claim 1, characterised in that they correspond to the following formula II:

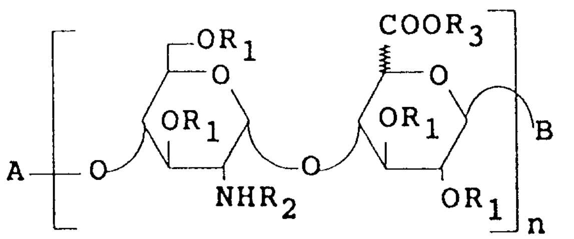

(II)

in which

- A has the meanings of claim 1;
- $R_1$ represents H and/or $SO_3^-$ and/or an acyl radical, such as that defined in claim 1;
- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7-12 carbon atoms, or an alkali or alkaline earth metal cation;
- B represents (a)-$OR_1$ or (f), (a) and (f) being such as defined in claim 1, or $OR_1$, or a group (a) to which remains attached a residue of (c) or of (d) such as that present after periodic oxidation followed by a $\beta$-elimination of an acid hydrolysis;
- n is an integer between 1 and 80, provided that when A represents $R_1$, a group $R_1$-(c) or a group $R_1$-(d) and when B represents O-$R_1$, a group (a)-$OR_1$, or a group (b)-$OR_1$, n is an integer between 1 and 16.

**3.** Process for the preparation of selectively O-acylated glycosaminoglycans according to claim 1, characterised in that they correspond to the following formula (II):

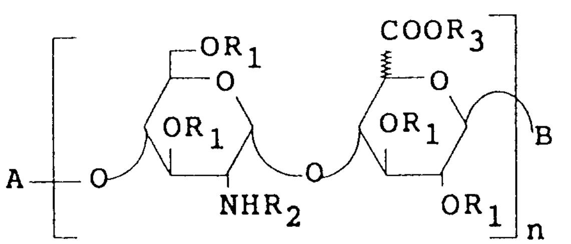

(II)

in which:

- A and B have their meanings of claim 1:
- $R_1$ represents H, $SO_3^-$ or an acyl radical of a carboxylic acid or a non-$\alpha$, $\beta$- unsaturated dicarboxylic acid chosen from among:
  - an alkanoyl group of 4 to 18 carbon atoms;
  - an alkanoyl group of 2 to 3 carbon atoms substituted by
    - a cycloalkyl group of 3 to 7 carbon atoms,
    - an unsaturated aliphatic hydrocarbon radical of 4 to 16 carbon atoms;

- a benzoyl group possibly substituted by one or more alkyl radicals of 1 to 4 carbon atoms, halogen atoms of $NO_2$ or $OCH_3$ groups;
- a cycloalkyl (3-7 C) carbonyl;

- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7 to 12 carbon atoms, or an alkali or alkaline earth metal cation;
- n is an integer between 1 and 80;

$R_1$ being acyl in a proportion of at least 0.5 to 2 acyl groups per disaccharide unit.

**4.** Process for the preparation of selectively O-acylated glycosaminoglycans according to claims 1 or 2, characterised in that the glycosaminoglycan is chosen from a group constituted by: a mixture of heparin fragments having a molecular mass of less than 10,000 daltons, a mixture of heparin fragments having a mean molecular mass between 2,000 and 7,000 daltons, a mixture of heparin fragments having a mean molecular mass of about 4,500 dalton, a mixture of heparin fragments having a mean molecular mass of about 2,500 daltons, or a mixture of heparin fragments of the same molecular mass, the acyl group being in the proportion 0.5 to 2 groups per disaccharide unit.

**5.** Process for the preparation of selectively O-acylated glycosaminoglycans according to claim 3, characterised in that the glycosaminoglycan is heparin, the acyl group being in the proportion of at least 0.5 to 2 groups per disaccharide unit.

**6.** Process for the preparation of selectively O-acylated glycosaminoglycans according to claim 5, characterised in that the acyl group is in the proportion of 1 acyl group per disaccharide unit.

**7.** Process for the preparation of selectively O-acylated glycosaminoglycans according to any of claims 1 to 4, characterised in that compositions of the following formula (III) are prepared:

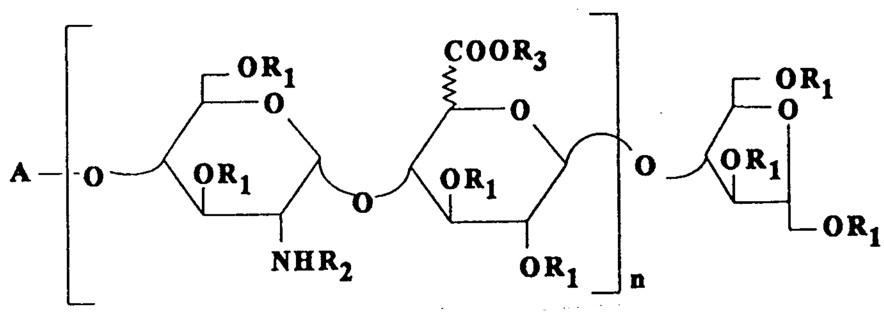

(III)

in which:

- A represents $R_1$, or $R_1$-(c) or $R_1$-(d), such as defined in claim 1;
- $R_1$ represents H and/or $SO_3^-$ and/or an acyl radical, such as that defined in claim 1;
- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom and/or an alkyl radical of 1 to 10 carbon atoms, a phenyl-alkyl radical of 7-12 carbon atoms, or an alkali or alkaline earth metal cation;
- n is an integer between 3 and 12.

**8.** Process for the preparation of selectively O-acylated glycosaminoglycans according to claims 1 to 4 characterised in that compositions of the following formula IV are prepared:

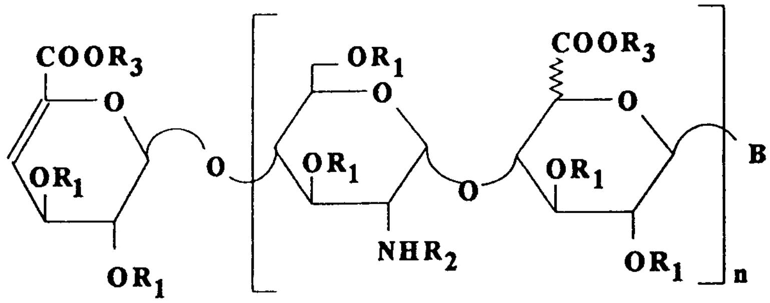

(IV)

in which:

- $R_1$ represents H and/or $SO_3^-$ and/or an acyl radical, such as that defined in claim 1;
- $R_2$ represents $SO_3^-$ and/or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7-12 carbon atoms, or an alkali or alkaline earth metal cation;
- B represents (a)-$OR_1$, such as defined in claim 1, or $OR_1$;
- n is an integer between 2 and 20.

**9.** Process for the preparation of selectively O-acylated glycosaminoglycans according to claims 1 or 2, characterised in that the glycosaminoglycan chosen is heparin, a heparin fraction or fragment lacking the binding site for antithrombin III.

**10.** Process for the preparation of selectively O-acylated glycosaminoglycans according to any of claims 1, 2, 3 and 9, characterised in that compositions of the following formula V are prepared:

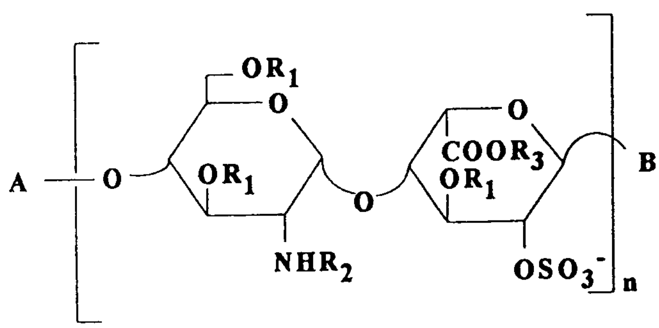

(V)

in which

- A represents $R_1$-(c), $R_1$-(d), or the residue of (c) or (d) after periodic oxidation followed by a $\beta$-elimination or an acid hydrolysis;
- B represents (a)-$OR_1$ or a group (a) to which remains attached a residue of (c) or of (d) such as that present after periodic oxidation followed by a $\beta$-elimination of an acid hydrolysis;
- $R_1$ has the meanings given in claim 1;
- $R_2$ represents $SO_3^-$ or an acetyl radical, the proportion of $SO_3^-$ being about 90%;
- $R_3$ represents a hydrogen atom or an alkali or alkaline earth metal cation;
- n is an integer between 1 and 80.

**11.** Process for the preparation of selectively O-acylated glycosaminoglycans according to claims 1,2,3 and 9, characterised in that compositions of the following formula VI are prepared:

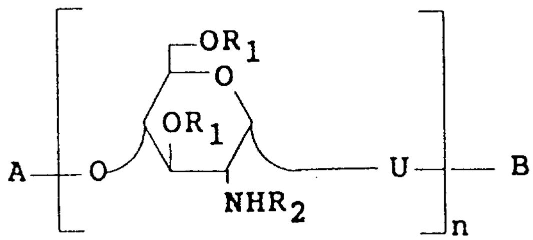

(VI)

in which:

- A represents $R_1$, or $R_1$-(c) or $R_1$-(d), or the residue of (c) or (d) after periodic oxidation followed by a $\beta$-elimination;
- U represents:

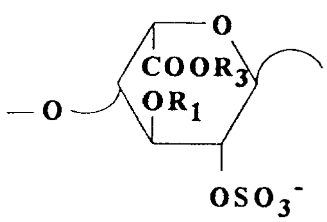

or in a ratio of one residue for at least two chains, a non-sulphated uronic acid (D-glucuronic or L-iduronic) open between the carbon atoms in positions 2 and 3, of formula:

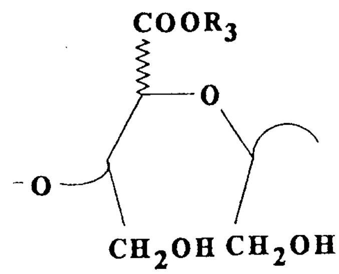

- B represents (a)-$OR_1$, or $OR_1$ or a group (a) to which remains attached a residue of (c) or of (d) such as that present after periodic oxidation followed by a $\beta$-elimination of an acid hydrolysis;
- $R_1$ has the meanings given in claim 1;
- $R_2$ represents $SO_3^-$ or an acetyl radical, the proportion of $SO_3^-$ being about 90%;
- $R_3$ represents a hydrogen atom or an alkali or alkaline earth metal cation;
- n is an integer between 2 and 18.

**12.** Process for the preparation of selectively O-acylated glycosaminoglycans according to any of claims 1,2,3 and 9, characterised in that compositions of formula VI are prepared in which:

- n is an integer between 7 and 15 for the major species,
- $R_1$ is an alkanoyl radical of 2 to 10 carbon atoms.

**13.** Process for the preparation of selectively O-acylated glycosaminoglycans according to claims 11 and 12, characterised in that compositions are prepared of a mixture of fragments of the same molecular mass, in which n is an integer between 2 and 12, and $R_1$ is an alkanoyl radical of 4 to 10 carbon atoms.

**14.** Process for the preparation of selectively O-acylated glycosaminoglycans according to any of claims 1,2,3 and 9, characterised in that compositions of the following formula VII are prepared:

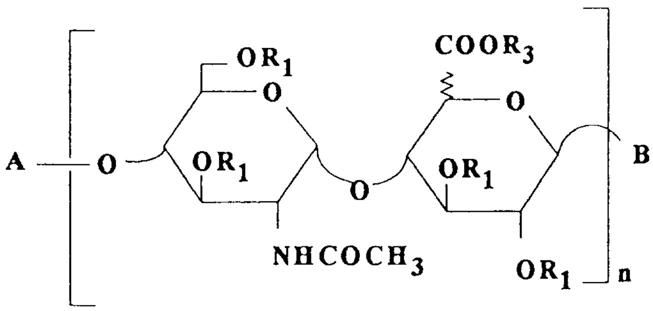

(VII)

in which:

- A represents $R_1$-(c), $R_1$-(d), such as defined in formula (I);
- $R_1$ represents an alkanoyl radical of 2 to 18 carbon atoms;
- $R_3$ represents a hydrogen atom or an alkali or alkaline earth metal cation;
- B represents (a)-$OR_1$ such as defined in formula (I), or $OR_1$
- n is an integer between 1 and 80.

**15.** Process for the preparation of selectively O-acylated glycosaminoglycans according to claim 1 characterised in that compositions of the following formula VIII are prepared:

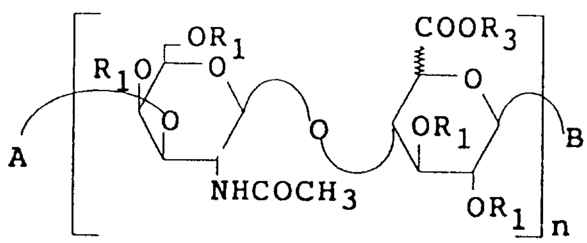

(VIII)

in which:

- A has the meanings of claim 1;
- $R_1$ represents H and/or $SO_3^-$ and/or an acyl radical, such as that defined in claim 1;
- $R_3$ represents a hydrogen atom, an alkyl radical of 1 to 10 carbon atoms, a phenyl-alkyl radical of 7-12 carbon atoms, or an alkali or alkaline earth metal cation;
- B represents (a)-$OR_1$, or (g), such as defined in formula (I), or $OR_1$, or a group (b) to which remains attached a residue of (c) or of (d) such as that present after periodic oxidation followed by a $\beta$-elimination of an acid hydrolysis;
- n is an integer between 1 and 80.

**16.** Process for the preparation of selectively O-acylated glycosaminoglycans according to claims 1 or 15, characterised in that the glycosaminoglycan is chosen from among the group consisting of dermatan sulphate and its fragments, the acyl group being in the proportion of at least 01. to 3 groups per disaccharide unit.

**17.** Process for the preparation of selectively O-acylated glycosaminoglycans according to any of claims 1 to 16, in which $R_1$ is an alkanoyl radical of 4 to 10 carbon atoms.

**18.** Process for the preparation of selectively O-acylated glycosaminoglycans according to claims 1 to 17 characterised in that:

(1) a glycosaminoglycan of formula IX is converted:

$A° \text{-} [G° \text{-} U°]_n \text{-} B°$ (IX)

in which:

- G° represents a group (a)° of formula:

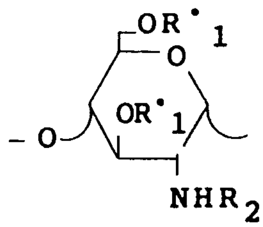

(a)°

or a group (a')° of formula:

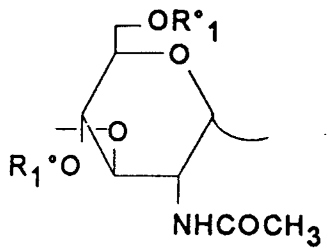

(a')°

or a group (b)° of formula:

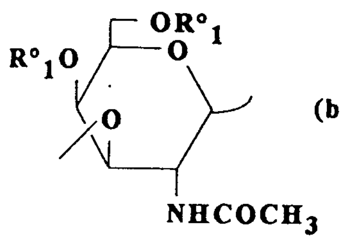

(b)°

- U° represents a group (c)° of formula:

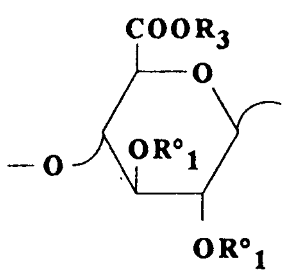

(c)°

or a group (d)° of formula:

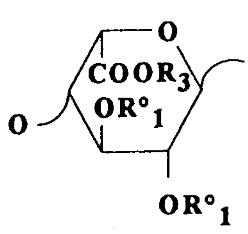

(d)°

or the residue of group (c)° or of group (d)° after periodic oxidation followed by a $\beta$-elimination or an acid hydrolysis;

- A° represents a group $R°_1$, a group $R°_1$-(c)°, a group $R°_1$-(d)°, or a group (e)° of formula:

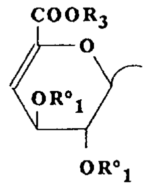

(e)°

or the residue of group (c)° or of group (d)° after periodic oxidation followed by a $\beta$-elimination or an acid hydrolysis;

- B° represents a group $OR°_1$, a group (a)°-$OR°_1$, a group (a')°-$OR°_1$ or a group (f)° of formula:

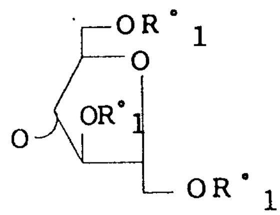

(f)°

or a group (g)° of formula:

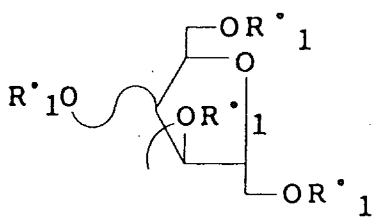

(g)°

or represents the groups (a)°, (a')°, (b)° to which remain attached a residue of (c)° or (d)° such as present after periodic oxidation followed by a $\beta$-elimination or an acid hydrolysis;

- $R_1$° represents H or $SO_3^-$;
- $R_2$ represents $SO_3^-$ or an acetyl radical, provided that the proportion of N-acetyl glucosamine does not exceed that in heparin when $R_1$ represents an acetyl radical;
- $R_3$ represents a hydrogen atom, an alkyl radical of 1 to 10 carbon atoms, a phenyl-alkyl radical of 7-12 carbon atoms, or an alkali or alkaline earth metal cation;
- n is an integer between 1 and 80,

into a tertiary amine salt or a quaternary ammonium salt soluble of a polar aprotic organic solvent;
(2) this salt is treated by an anhydride of the formula:

$R_1$-O-$R_1$

in which $R_1$ represents an acyl radical, as such as that defined in claim 1, in the said polar aprotic organic solvent, in the presence of catalytic amounts of pyridine or a dialkylaminopyridine and a proton acceptor a a temperature situated between 0°C and 100°C;
(3) the product thus obtained is precipitated by the action of a solution sodium acetate in ethanol and,
(4) the selectively O-acylated glycosaminoglycan is isolated by dissolving the precipitate thus obtained in water and by dialysis, and if required , the sodium salt of the selectively O-acylated

glycosaminoglycan thus obtained is converted into another pharmaceutically acceptable salt.

**19.** Process according to claim 18, characterised in that the glycosaminoglycan used in step (1) is chosen from the group constituted by heparin, a mixture of heparin fragments having a molecular mass of less than 10,000 daltons, a mixture of heparin fragments having a mean molecular mass between 2,000 and 7,000 daltons, a mixture of heparin fragments having a mean molecular mass of about 4,500 daltons, a mixture of heparin fragments having a mean molecular mass of about 2,500 daltons, or a mixture of heparin fragments of the same molecular mass.

**20.** Process according to claim 18, characterised in that the glycosaminoglycan used in step (1) is heparin or a fraction or fragment of heparin lacking the binding site for antithrombin III.

**21.** Process according to claim 18, characterised in that the glycosaminoglycan used in step (1) is chosen from the group constituted by dermatan sulphate and its fragments.

**22.** Process according to any of claims 18 or 21, characterised in that the glycosaminoglycan used in step (1) is a salt of tributylamine or tetrabutylammonium.

**23.** Process according to any of claims 18 to 22, characterised in that the anhydride used in step (2) is the anhydride of an alkanoic acid containing 2 to 10 carbon atoms.

**24.** Process according to claims 18 to 23, characterised in that the step (2) is conducted in a polar aprotic solvent chosen from among the group constituted by dimethylformamide, hexamethylphosphorotriamide, pyridine, or a mixture of these solvents with or without dichloromethane.

**25.** Process according to any of claims 18 to 24, characterised in that, as proton acceptors in step (2), a base is chosen from among the group constituted by pyridine, triethylamine and tributylamine.

**26.** Process according to any of claims 18 to 25, characterised in that the dialysis in step (4) is performed in the presence of a weak base.

**Patentansprüche**
**Patentansprüche für folgende Vertragsstaaten : AT, BE, CH, DE, FR, GB, GR, IT, LI, LU, NL, SE**

**1.** Selektiv O-acylierte Glykosaminoglykane der folgenden Formel (I):

A - $[G\text{-}U]_n$-B (I)

in der

- G eine Gruppe (a) der Formel:

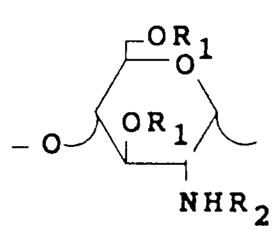

(a)

oder eine Gruppe (a') der Formel:

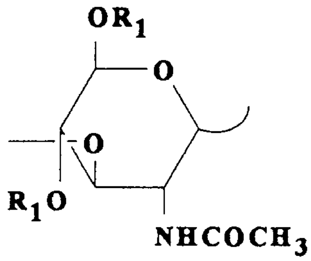

(a')

oder eine Gruppe (b) der Formel:

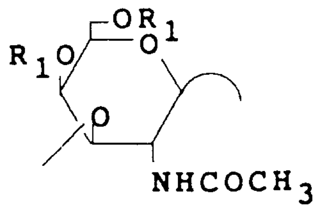

(b)

- U eine Gruppe (c) der Formel:

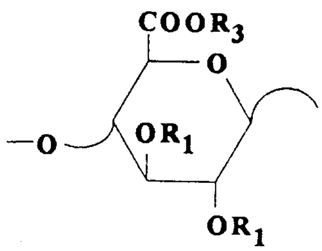

(c)

oder eine Gruppe (d) der Formel:

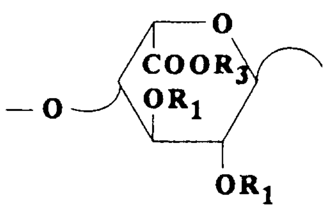

(d)

oder den Rest der Gruppe (c) oder der Gruppe (d) nach der Periodoxidation gefolgt von einer $\beta$-Eliminierung oder einer sauren Hydrolyse;

- A eine Gruppe $R_1$, eine Gruppe $R_1$-(c), eine Gruppe $R_1$-(d) oder eine Gruppe (e) der Formel:

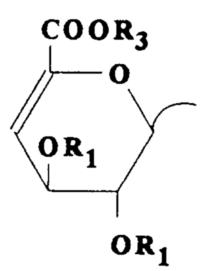

(e)

oder den Rest der Gruppe (c) oder der Gruppe (d) nach der Periodoxidation gefolgt von einer $\beta$-Eliminierung oder einer sauren Hydrolyse;

- B eine Gruppe O-$R_1$, eine Gruppe (a)-O$R_1$, eine Gruppe (a')-O$R_1$, eine Gruppe (b)-O$R_1$ oder eine Gruppe (f) der Formel:

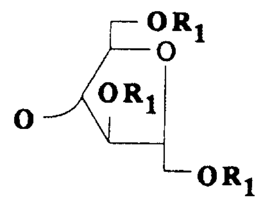

(f)

oder eine Gruppe (g) der Formel:

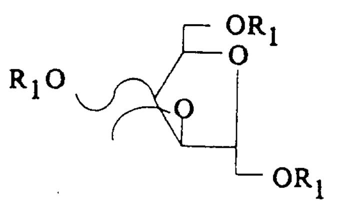

(g)

oder eine Gruppe (a), eine Gruppe (a') oder eine Gruppe (b), an welche ein Rest von (c) oder von (d) gebunden ist, wie er nach der Periodoxidation gefolgt von einer $\beta$-Eliminierung oder einer sauren Hydrolyse vorliegt;

- $R_1$ H, $SO_3^-$ oder eine Acylgruppe einer nicht-$\alpha,\beta$-ungesättigten Carbonsäure oder Dicarbonsäure ausgewählt aus:
  - einer Alkanoylgruppe mit 1 bis 18 Kohlenstoffatomen,
  - einer Alkanoylgruppe mit 2 bis 3 Kohlenstoffatomen substituiert mit:
    - . einer Cycloalkylgruppe mit 3 bis 7 Kohlenstoffatomen oder
    - . einer ungesättigten aliphatischen Kohlenwasserstoffgruppe mit 4 bis 16 Kohlenstoffatomen;
  - einer Benzoylgruppe, die gegebenenfalls durch eine oder mehrere Alkylgruppen mit 1 bis 4 Kohlenstoffatomen, Halogenatome oder $NO_2$- oder $OCH_3$-Gruppen substituiert ist,
  - einer ($C_{3-7}$)-Cycloalkyl-carbonylgruppe;
- $R_2$ $SO_3^-$ und/oder eine Acetylgruppe mit der Maßgabe, daß der Anteil von N-Acetylglucosamin höchstens gleich ist demjenigen von Heparin, wenn $R_1$ eine Acetylgruppe darstellt;
- $R_3$ ein Wasserstoffatom, eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation; und
- n eine ganze Zahl mit einem Wert von 1 bis 80 bedeuten,

wobei $R_1$ mit der Maßgabe eine Acylgruppe bedeutet, daß mindestens 0,1 bis 3 Acylgruppen pro Disaccharid-Einheit vorliegen, und deren pharmazeutisch annehmbare Salze,
erhältlich ausgehend von einem Glykosaminoglykan ausgewählt aus der Gruppe, die gebildet wird durch:
Heparin, einer Mischung von Heparinfragmenten mit einem Molekulargewicht von weniger als 10.000 Dalton, einer Mischung von Heparinfragmenten mit einem mittleren Molekulargewicht zwischen 2.000 und 7.000 Dalton, einer Mischung von Heparinfragmenten mit einem mittleren Molekulargewicht von etwa 4.500 Dalton, einer Mischung von Heparinfragmenten mit einem mittleren Molekulargewicht von etwa 2.500 Dalton oder einer Mischung von Heparinfragmenten mit gleichem Molekulargewicht, einer Heparinfraktion oder einem Heparinfragment, welches frei ist von Bindungsstellen für Antithrombin III, Dermatansulfat und dessen Fragmente,
welches man:

(1) in einem aprotischen polaren Lösungsmittel in ein tertiäres Aminsalz oder ein quaternäres Ammoniumsalz umwandelt;

(2) dieses Salz mit einem Anhydrid der Formel:

$R_1$ - O - $R_1$

in der $R_1$ eine Acylgruppe, wie sie oben definiert worden ist, bedeutet, in einem aprotischen polaren Lösungsmittel und in Gegenwart von katalytischen Mengen von Pyridin oder eines Dialkylaminopyridins und eines Protonenakzeptors bei einer Temperatur zwischen 0 ° C und 100 ° C behandelt;
(3) man das in diese Weise erhaltene Produkt durch Einwirkung einer Lösung von Natriumacetat in Ethanol ausfällt und
(4) das selektiv O-acylierte Glykosaminoglykan durch Auflösen des in dieser Weise erhaltenen Niederschlags in Wasser und Dialyse isoliert und gegebenenfalls das in dieser Weise erhaltene Natriumsalz des selektiv O-acylierten Glykosaminoglykans in ein anderes pharmazeutisch annehmbares Salz umwandelt.

**2.** Selektiv O-acylierte Glykosaminoglykane nach Anspruch 1, **dadurch gekennzeichnet,** daß sie der folgenden Formel (II) entsprechen:

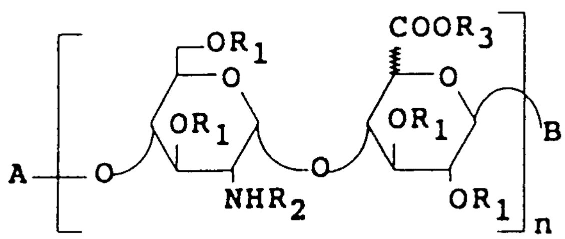

(II)

in der
- A die in Anspruch 1 angegebenen Bedeutungen besitzt;
- $R_1$ H und/oder $SO_3^-$ und/oder eine Acylgruppe, wie sie bezüglich der Formel (I) definiert worden ist;
- $R_2$ $SO_3^-$ und/oder eine Acetylgruppe, mit der Maßgabe, daß der Anteil von N-Acetylglucosamin höchstens gleich ist jenem von Heparin, wenn $R_1$ eine Acetylgruppe darstellt;
- $R_3$ ein Wasserstoffatom, eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation;
- B (a)-$OR_1$ oder (f), wobei (a) und (f) die in Anspruch 1 angegebenen Bedeutungen besitzen, oder $OR_1$ oder eine Gruppe (a), an die ein Rest von (c) oder (d) gebunden ist, wie er nach der Periodoxidation gefolgt von einer $\beta$-Eliminierung oder einer sauren Hydrolyse vorhanden ist; und
- n eine ganze Zahl mit einem Wert von 1 bis 80 mit der Maßgabe bedeuten, daß, wenn A $R_1$, eine Gruppe $R_1$-(c) oder eine Gruppe $R_1$-(d) und B O-$R_1$, eine Gruppe (a)-$OR_1$ oder eine Gruppe (b)-$OR_1$ darstellen, n eine ganze Zahl mit einem Wert von 1 bis 16 bedeutet.

**3.** Selektiv O-acylierte Glykosaminoglykane nach Anspruch 1, **dadurch gekennzeichnet,** daß sie der allgemeinen Formel (II) entsprechen:

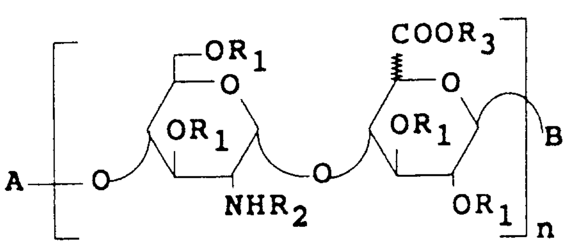

(II)

in der:
- A und B die in Anspruch 1 angegebenen Bedeutungen besitzen;
- $R_1$ H und/oder $SO_3^-$ und/oder eine Acylgruppe einer nicht-$\alpha,\beta$-ungesättigten Carbonsäure oder Dicarbonsäure ausgewählt aus:
  - einer Alkanoylgruppe mit 4 bis 18 Kohlenstoffatomen,
  - einer Alkanoylgruppe mit 2 bis 3 Kohlenstoffatomen substituiert durch:

. eine Cycloalkylgruppe mit 3 bis 7 Kohlenstoffatomen oder
. eine ungesättigte aliphatische Kohlenwasserstoffgruppe mit 4 bis 16 Kohlenstoffatomen;

- einer Benzoylgruppe, die gegebenenfalls durch eine oder mehrere Alkylgruppen mit 1 bis 4 Kohlenstoffatomen, Halogenatome oder $NO_2$- oder $OCH_3$-Gruppen substituiert ist,
- einer ($C_{3-7}$)-Cycloalkyl-carbonylgruppe;

- $R_2$ $SO_3^-$ und/oder eine Acetylgruppe mit der Maßgabe, daß der Anteil von N-Acetylglucosamin höchstens gleich ist jenem von Heparin, wenn $R_1$ eine Acetylgruppe darstellt;
- $R_3$ ein Wasserstoffatom, eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation; und
- n eine ganze Zahl mit einem Wert von 1 bis 80 mit der Maßgabe bedeuten, daß $R_1$ in dem Ausmaß Acyl bedeutet, daß mindestens 0,5 bis 2 Acylgruppen pro Disaccharid-Einheit vorliegen.

**4.** Selektiv O-acylierte Glykosaminoglykane nach den Ansprüchen 1 oder 2, **dadurch gekennzeichnet,** daß das Glykosaminoglykan aus der Gruppe ausgewählt ist, die gebildet wird aus einer Mischung aus Heparinfragmenten mit einem Molekulargewicht von weniger als 10.000 Dalton, einer Mischung von Heparinfragmenten mit einem mittleren Molekulargewicht zwischen 2.000 und 7.000 Dalton, einer Mischung von Heparinfragmenten mit einem mittleren Molekulargewicht von etwa 4.500 Dalton, einer Mischung von Heparinfragmenten mit einem mittleren Molekulargewicht von etwa 2.500 Dalton und einer Mischung von Heparinfragmenten mit gleichem Molekulargewicht, wobei die Acylgruppe in einem Anteil von 0,5 bis 2 Gruppen pro Disaccharid-Einheit vorhanden ist.

**5.** Selektiv O-acylierte Glykosaminoglykane nach Anspruch 3, **dadurch gekennzeichnet,** daß das Glykosaminoglykan Heparin ist und die Acylgruppe in einem Anteil von mindestens 0,5 bis 2 Gruppen pro Disaccharid-Einheit vorliegt.

**6.** Selektiv O-acylierte Glykosaminoglykane nach Anspruch 5, **dadurch gekennzeichnet,** daß die Acylgruppe in einem Anteil von einer Acylgruppe pro Disaccharid-Einheit vorliegt.

**7.** Selektiv O-acylierte Glykosaminoglykane nach einem der Ansprüche 1 bis 4, **dadurch gekennzeichnet,** daß sie der folgenden Formel (III) entsprechen:

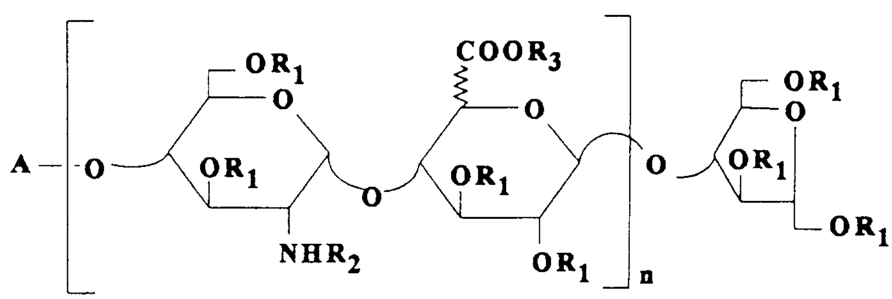

(III)

in der

- A $R_1$ oder $R_1$-(c) oder $R_1$-(d), wie sie in Anspruch 1 definiert worden sind;
- $R_1$ H und/oder $SO_3^-$ und/oder eine Acylgruppe, wie sie in Anspruch 1 definiert worden ist;
- $R_2$ $SO_3^-$ und/oder eine Acetylgruppe mit der Maßgabe, daß der Anteil von N-Acetylglucosamin höchstens gleich ist jenem von Heparin, wenn $R_1$ eine Acetylgruppe darstellt;
- $R_3$ ein Wasserstoffatom und/oder eine Alkylgruppe mit 1 bis 10 Kohlenstoffatomen, eine Phenylalkylgruppe mit 7 bis 12 Kohlenstoffatomen oder ein Alkalimetall- oder Erdalkalimetall-Kation; und
- n eine ganze Zahl mit einem Wert von 3 bis 12 bedeuten.

**8.** Selektiv O-acylierte Glykosaminoglykane nach einem der Ansprüche 1 bis 4, **dadurch gekennzeichnet,** daß sie der folgenden Formel (IV) entsprechen:

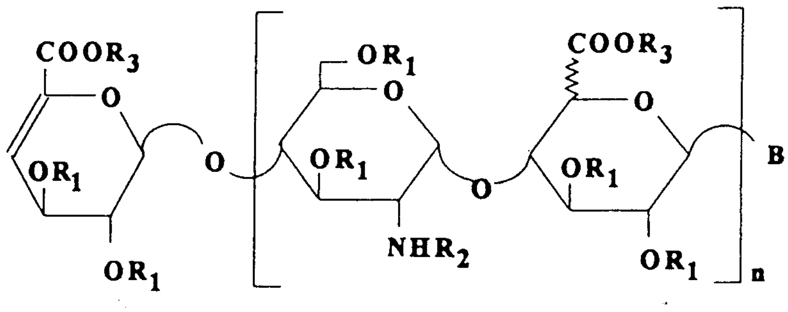

(IV)

in der:

- $R_1$ H und/oder $SO_3^-$ und/oder eine Acylgruppe, wie sie in Anspruch 1 definiert worden ist;
- $R_2$ $SO_3^-$ und/oder eine Acetylgruppe mit der Maßgabe, daß der Anteil von N-Acetylglucosamin höchstens gleich ist jenem von Heparin, wenn $R_1$ eine Acetylgruppe darstellt;
- $R_3$ ein Wasserstoffatom und/oder eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation;
- B (a)-$OR_1$, wie es in Anspruch 1 definiert worden ist, oder $OR_1$; und
- n eine ganze Zahl mit einem Wert von 2 bis 20 bedeuten.

**9.** Selektiv O-acylierte Glykosaminoglykane nach den Ansprüchen 1 oder 2, **dadurch gekennzeichnet,** daß das ausgewählte Glykosaminoglykan Heparin oder eine Heparinfraktion oder ein Heparinfragment, welches frei ist von Bindungsstellen für Antithrombin III, ist.

**10.** Selektiv O-acylierte Glykosaminoglykane nach einem der Ansprüche 1, 2, 3 und 9, **dadurch gekennzeichnet,** daß sie der folgenden Formel (V) entsprechen:

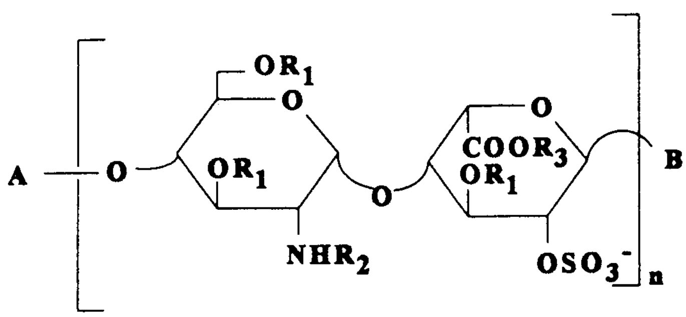

(V)

in der:

- A $R_1$-(c), $R_1$-(d) oder den Rest von (c) oder (d) nach der Periodoxidation gefolgt von einer $\beta$-Eliminierung oder einer sauren Hydrolyse;
- B eine Gruppe (a)-$OR_1$, eine Gruppe (a), an welche ein Rest von (c) oder von (d) gebunden ist, wie er nach der Periodoxidation gefolgt von einer $\beta$-Eliminierung oder einer sauren Hydrolyse vorhanden ist;
- $R_1$ die in Anspruch 1 angegebenen Bedeutungen;
- $R_2$ $SO_3^-$ oder eine Acetylgruppe, wobei der Anteil von $SO_3^-$ etwa 90 % beträgt;
- $R_3$ ein Wasserstoffatom oder ein Alkalimetall- oder ein Erdalkalimetall-Kation; und
- n eine ganze Zahl mit einem Wert von 1 bis 80 bedeuten.

**11.** Selektiv O-acylierte Glykosaminoglykane nach einem der Ansprüche 1, 2, 3 und 9, **dadurch gekennzeichnet,** daß sie der folgenden Formel (VI) entsprechen:

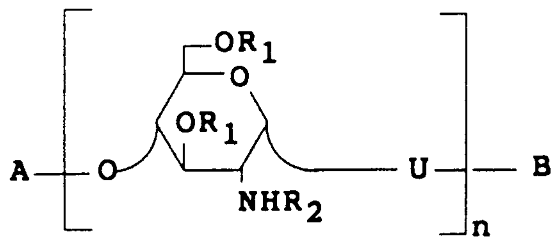

(VI)

in der:

- A $R_1$, $R_1$-(c), $R_1$-(d) oder den Rest von (c) oder von (d) nach der Periodoxidation gefolgt von einer $\beta$-Eliminierung;
- U eine Gruppe der Formel:

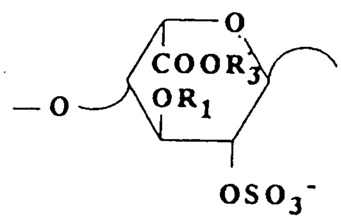

aus der sich für mindestens zwei Ketten eine geöffnete nichtsulfatierte Uronsäure (D-Glucuronsäure oder L-Iduronsäure) zwischen den Kohlenstoffatomen in den Positionen 2 und 3 ergibt, der Formel:

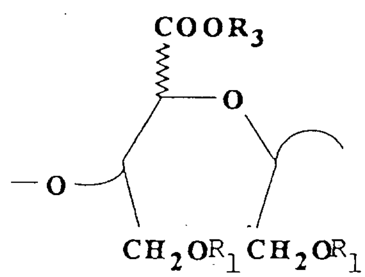

- B (a)-$OR_1$ oder $OR_1$ oder eine Gruppe (a), an die ein Rest von (c) oder von (d) gebunden ist, wie er nach der Periodoxidation gefolgt von einer $\beta$-Eliminierung vorhanden ist;
- $R_1$ die in Anspruch 1 angegebenen Bedeutungen;
- $R_2$ $SO_3^-$ oder Acetylgruppe, wobei der Anteil von $SO_3^-$ etwa 90 % beträgt;
- $R_3$ ein Wasserstoffatom oder ein Alkalimetall- oder Erdalkalimetall-Kation; und
- n eine ganze Zahl mit einem Wert von 2 bis 18 bedeuten.

**12.** Selektiv O-acylierte Glykosaminoglykane nach einem der Ansprüche 1, 2, 3 und 9, **dadurch gekennzeichnet,** daß sie der Formel (VI) entsprechen, in der:

- n für die Mehrheit der vorhandenen Moleküle eine ganze Zahl zwischen 7 bis 15 bedeutet und
- $R_1$ eine Alkanoylgruppe mit 2 bis 10 Kohlenstoffatomen darstellt.

**13.** Selektiv O-acylierte Glykosaminoglykane gemäß den Ansprüchen 11 und 12, **dadurch gekennzeichnet,** daß sie aus einer Mischung von Fragmenten mit gleichem Molekulargewicht gebildet sind, in denen n eine ganze Zahl mit einem Wert von 2 bis 12 und $R_1$ eine Alkanoylgruppe mit 4 bis 10 Kohlenstoffatomen bedeuten.

**14.** Selektiv O-acylierte Glykosaminoglykane nach einem der Ansprüche 1, 2, 3 und 9, **dadurch gekennzeichnet,** daß sie der folgenden Formel (VII) entsprechen:

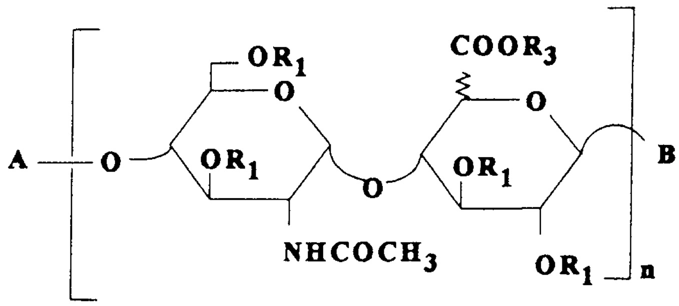

(VII)

in der:

- A $R_1$, $R_1$-(c) oder $R_1$-(d), wie sie bezüglich der Formel (I) definiert worden sind;
- $R_1$ eine Alkanoylgruppe mit 2 bis 18 Kohlenstoffatomen;
- $R_3$ ein Wasserstoffatom oder ein Alkalimetall- oder Erdalkalimetall-Kation;
- B (a)-$OR_1$, wie es bezüglich der Formel (I) definiert worden ist, oder $OR_1$; und
- n eine ganze Zahl mit einem Wert von 1 bis 80 bedeuten.

**15.** Selektiv O-acylierte Glykosaminoglykane nach Anspruch 1, **dadurch gekennzeichnet,** daß sie der folgenden Formel (VIII) entsprechen:

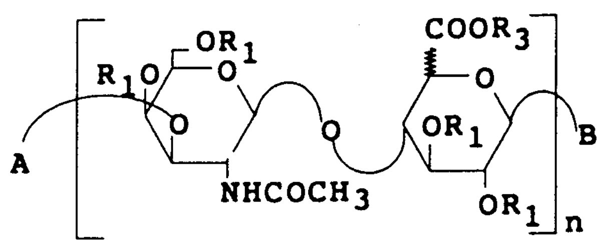

(VIII)

in der:

- A die in Anspruch 1 angegebenen Bedeutungen besitzt;
- $R_1$ H und/oder $SO_3^-$ und/oder eine Acylgruppe, wie sie in Anspruch 1 definiert worden ist;
- $R_3$ ein Wasserstoffatom, eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation;
- B (b)-$OR_1$ oder (g), wie sie bezüglich der Formel (I) definiert worden sind, oder $OR_1$ oder eine Gruppe (b), an die ein Rest von (c) oder von (d) gebunden ist, wie er nach der Periodoxidation gefolgt von einer $\beta$-Eliminierung oder einer sauren Hydrolyse vorhanden ist; und
- n eine ganze Zahl mit einem Wert von 1 bis 80 bedeuten.

**16.** Selektiv O-acylierte Glykosaminoglykane nach den Ansprüchen 1 oder 15, **dadurch gekennzeichnet,** daß das Glykosaminoglykan aus der Gruppe ausgewählt ist, die Dermatansulfat und dessen Fragmente umfaßt, wobei die Acylgruppe in einem Anteil von mindestens 0,1 bis 3 Gruppen pro Disaccharid-Einheit vorhanden ist.

**17.** Selektiv O-acylierte Glykosaminoglykane nach einem der Ansprüche 1 bis 16, worin $R_1$ eine Alkanoylgruppe mit 4 bis 10 Kohlenstoffatomen darstellt.

**18.** Verfahren zur Herstellung von selektiv O-acylierten Glykosaminoglykanen nach einem der Ansprüche 1 bis 17, **dadurch gekennzeichnet,** daß man:

(1) ein Glykosaminoglykan der Formel IX:

$A° - [G° - U°]_n - B°$ (IX)

in der:

- G° eine Gruppe (a)° der Formel:

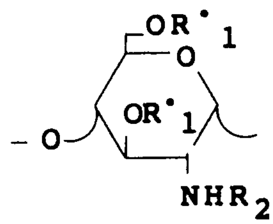

(a)°

oder eine Gruppe (a')° der Formel:

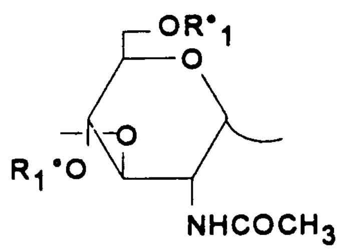

(a')°

oder eine Gruppe (b)° der Formel:

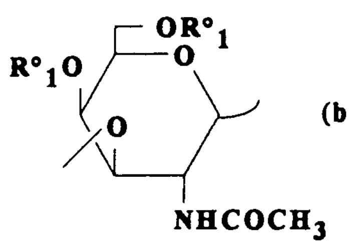

(b)°

- U° eine Gruppe (c)° der Formel:

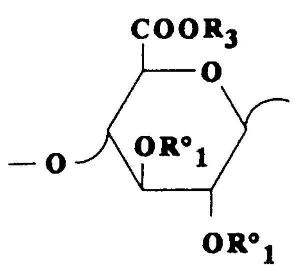

(c)°

oder eine Gruppe (d)° der Formel:

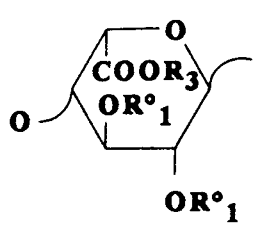

(d)°

oder den Rest der Gruppe (c)° oder der Gruppe (d)° nach der Periodoxidation gefolgt von einer β-Eliminierung oder einer sauren Hydrolyse;

- A° eine Gruppe $R°_1$ oder eine Gruppe $R°_1$-(c)°, eine Gruppe $R°_1$-(d)° oder eine Gruppe (e)-° der Formel:

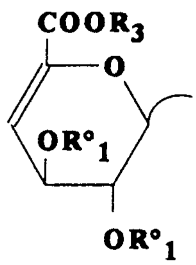

(e)°

oder den Rest der Gruppe (c)° oder der Gruppe (d)° nach der Periodoxidation gefolgt von einer β-Eliminierung oder einer sauren Hydrolyse;

- B° eine Gruppe $OR°_1$, eine Gruppe (a)°-$OR°_1$, eine Gruppe (a')°-$OR°_1$, eine Gruppe (b)°-$OR°_1$ oder eine Gruppe (f)° der Formel:

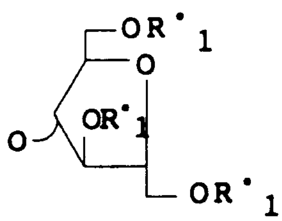

(f)°

oder eine Gruppe (g)° der Formel:

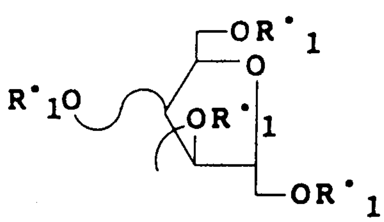

(g)°

oder Gruppen (a)°, (a')°, (b)°, an die ein Rest von (c)° oder von (d)° gebunden ist, wie er nach der Periodoxidation gefolgt von einer β-Eliminierung oder einer sauren Hydrolyse vorhanden ist;

- $R_1$° H oder $SO_3^-$;
- $R_2$ $SO_3^-$ oder eine Acetylgruppe mit der Maßgabe, daß der Anteil von N-Acetylglucosamin höchstens gleich ist dem von Heparin, wenn $R_1$ eine Acetylgruppe darstellt;
- $R_3$ ein Wasserstoffatom, eine Alkylgruppe mit 1 bis 10 Kohlenstoffatmen, eine Phenylalkylgruppe mit 7 bis 12 Kohlenstoffatomen oder ein Alkalimetall-oder Erdalkalimetall-Kation; und
- n eine ganze Zahl mit einem Wert von 1 bis 80 bedeuten,

in ein tertiäres Aminsalz oder ein quaternäres Ammoniumsalz des Glykosaminoglykans umwandelt, welches in einem aprotischen polaren Lösungsmittel löslich ist;
(2) dieses Salz mit einem Anhydrid der Formel:

$R_1$ - O - $R_1$

in der $R_1$ eine Acylgruppe bedeutet, wie sie in Anspruch 1 definiert worden ist, in dem polaren

aprotischen organischen Lösungsmittel in Gegenwart von katalytischen Mengen von Pyridin oder eines Dialkylaminopyridins und eines Protonenakzeptors bei einer Temperatur zwischen 0 °C und 100 °C behandelt;
(3) das in dieser Weise erhaltene Produkt durch Einwirkung einer Lösung von Natriumacetat in Ethanol ausfällt und
(4) das selektiv O-acylierte Glykosaminoglykan durch Auflösen des in dieser Weise erhaltenen Niederschlags in Wasser und Dialyse isoliert und das in dieser Weise erhaltene Natriumsalz des selektiv O-acylierten Glykosaminoglykans gegebenenfalls in ein anderes pharmazeutisch annehmbares Salz umwandelt.

**19.** Verfahren nach Anspruch 18, **dadurch gekennzeichnet,** daß das in der Stufe (1) eingesetzte Glykosaminoglykan aus der Gruppe ausgewählt ist, die durch Heparin, einer Mischung von Heparinfragmenten mit einem Molekulargewicht von weniger als 10.000 Dalton, einer Mischung von Heparinfragmenten mit einem mittleren Molekulargewicht zwischen 2.000 und 7.000 Dalton, einer Mischung von Heparinfragmenten mit einem mittleren Molekulargewicht von etwa 4.500 Dalton, einer Mischung von Heparinfragmenten mit einem Molekulargewicht von etwa 2.500 Dalton oder einer Mischung von Heparinfragmenten mit dem gleichen Molekulargewicht gebildet wird.

**20.** Verfahren nach Anspruch 18, **dadurch gekennzeichnet,** daß das in der Stufe (1) eingesetzte Glykosaminoglykan Heparin oder eine Heparinfraktion oder ein Heparinfragment ist, welches frei von Bindungsstellen für Antithrombin III ist.

**21.** Verfahren nach Anspruch 18, **dadurch gekennzeichnet,** daß das in der Stufe (1) eingesetzte Glykosaminoglykan aus der Gruppe ausgewählt wird, die durch Dermatansulfat und seinen Fragmenten gebildet wird.

**22.** Verfahren nach einem der Ansprüche 18 bis 21, **dadurch gekennzeichnet,** daß das Glykosaminoglykansalz der Stufe (1) ein Tributylaminsalz oder ein Tetrabutylammoniumsalz ist.

**23.** Verfahren nach einem der Ansprüche 18 bis 22, **dadurch gekennzeichnet,** daß das in der Stufe (2) eingesetzte Anhydrid ein Anhydrid einer Alkansäure mit 2 bis 10 Kohlenstoffatomen ist.

**24.** Verfahren nach einem der Ansprüche 18 bis 23, **dadurch gekennzeichnet,** daß die Stufe (2) in einem polaren aprotischen Lösungsmittel durchgeführt wird ausgewählt aus der Gruppe, die durch Dimethylformamid, Hexamethylphosphortriamid, Pyridin oder einer Mischung dieser Lösungsmittel untereinander oder mit Dichlormethan gebildet wird.

**25.** Verfahren nach einem der Ansprüche 18 bis 24, **dadurch gekennzeichnet,** daß man als Protonenakzeptor in der Stufe (2) eine Base verwendet, die aus der Gruppe ausgewählt ist, die durch Pyridin, Triethylamin und Tributylamin gebildet wird.

**26.** Verfahren nach einem der Ansprüche 18 bis 25, **dadurch gekennzeichnet,** daß die Dialyse der Stufe (4) in Gegenwart einer schwachen Base durchgeführt wird.

**27.** Pharmazeutische Zubereitung, **dadurch gekennzeichnet,** daß sie als Wirkstoff eine wirksame Menge mindestens eines selektiv O-acylierten Glykosaminoglykans nach einem der Ansprüche 1 bis 17 in Kombination mit einem pharmazeutischen Trägermaterial umfaßt.

**28.** Pharmazeutische Zubereitung nach Anspruch 27 zur Behandlung oder zur Vorbeugung von durch Hüllviren verursachte Erkrankungen.

**29.** Pharmazeutische Zubereitung nach Anspruch 27 zur Behandlung oder zur Vorbeugung von durch Retroviren verursachten Erkrankungen.

**Patentansprüche für folgenden Vertragsstaat : ES**

**1.** Verfahren zur Herstellung von selektiv O-acylierten Glykosaminoglykanen der folgenden Formel (I):

A - $[G\text{-}U]_n$-B (I)

in der

- G eine Gruppe (a) der Formel:

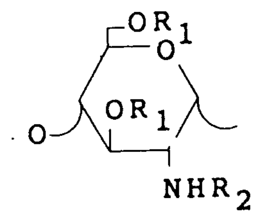

(a)

oder eine Gruppe (a') der Formel:

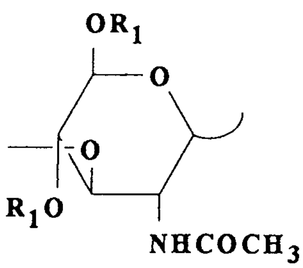

(a')

oder eine Gruppe (b) der Formel:

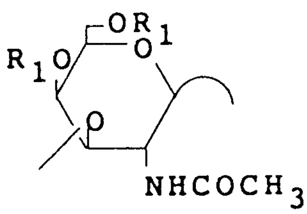

(b)

- U eine Gruppe (c) der Formel:

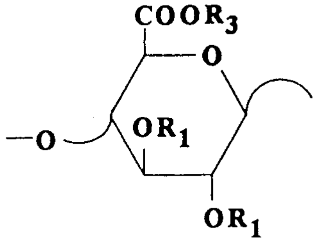

(c)

oder eine Gruppe (d) der Formel:

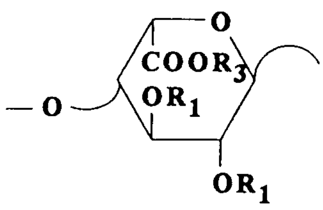

(d)

oder den Rest der Gruppe (c) oder der Gruppe (d) nach der Periodoxidation gefolgt von einer β-Eliminierung oder einer sauren Hydrolyse;

- A eine Gruppe $R_1$, eine Gruppe $R_1$-(c), eine Gruppe $R_1$-(d) oder eine Gruppe (e) der Formel:

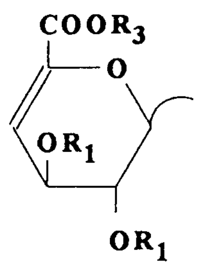

(e)

oder den Rest der Gruppe (c) oder der Gruppe (d) nach der Periodoxidation gefolgt von einer β-Eliminierung oder einer sauren Hydrolyse;

- B eine Gruppe O-$R_1$, eine Gruppe (a)-O$R_1$, eine Gruppe (a')-O$R_1$, eine Gruppe (b)-O$R_1$ oder eine Gruppe (f) der Formel:

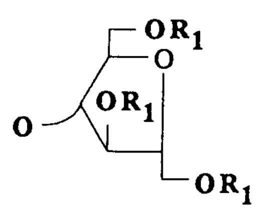

(f)

oder eine Gruppe (g) der Formel:

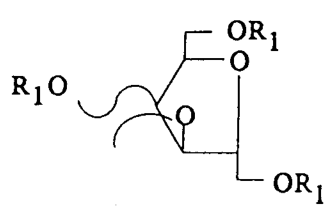

(g)

oder eine Gruppe (a), eine Gruppe (a') oder eine Gruppe (b), an welche ein Rest von (c) oder von (d) gebunden ist, wie er nach der Periodoxidation gefolgt von einer β-Eliminierung oder einer sauren Hydrolyse vorliegt;

- $R_1$ H, $SO_3^-$ oder eine Acylgruppe einer nicht-α,β-ungesättigten Carbonsäure oder Dicarbonsäure ausgewählt aus:
  - einer Alkanoylgruppe mit 1 bis 18 Kohlenstoffatomen,
  - einer Alkanoylgruppe mit 2 bis 3 Kohlenstoffatomen substituiert mit:
    - . einer Cycloalkylgruppe mit 3 bis 7 Kohlenstoffatomen oder
    - . einer ungesättigten aliphatischen Kohlenwasserstoffgruppe mit 4 bis 16 Kohlenstoffatomen;
  - einer Benzoylgruppe, die gegebenenfalls durch eine oder mehrere Alkylgruppen mit 1 bis 4 Kohlenstoffatomen, Halogenatome oder $NO_2$- oder $OCH_3$-Gruppen substituiert ist,

- einer ($C_{3-7}$)-Cycloalkyl-carbonylgruppe;
- $R_2$ $SO_3^-$ und/oder eine Acetylgruppe mit der Maßgabe, daß der Anteil von N-Acetylglucosamin höchstens gleich ist demjenigen von Heparin, wenn $R_1$ eine Acetylgruppe darstellt;
- $R_3$ ein Wasserstoffatom, eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation; und
- n eine ganze Zahl mit einem Wert von 1 bis 80 bedeuten,

wobei $R_1$ mit der Maßgabe eine Acylgruppe bedeutet, daß mindestens 0,1 bis 3 Acylgruppen pro Disaccharid-Einheit vorliegen,
und von deren pharmazeutisch annehmbaren Salzen,
**dadurch gekennzeichnet,** daß man

(1) ein Glykosaminoglykan ausgewählt aus der Gruppe, die durch Heparin, einer Mischung von Heparinfragmenten mit einem Molekulargewicht von weniger als 10.000 Dalton, einer Mischung von Heparinfragmenten mit einem mittleren Molekulargewicht zwischen 2.000 und 7.000 Dalton, einer Mischung von Heparinfragmenten mit einem mittleren Molekulargewicht von etwa 4.500 Dalton, einer Mischung von Heparinfragmenten mit einem mittleren Molekulargewicht von etwa 2.500 Dalton oder einer Mischung von Heparinfragmenten mit gleichem Molekulargewicht oder einer Heparinfraktion oder einem Heparinfragment, das frei ist von Bindungsstellen für Antithrombin III, Dermatansulfat und seinen Fragmenten gebildet wird,
in ein tertiäres Aminsalz oder ein quaternäres Ammoniumsalz umwandelt, welches in einem polaren aprotischen organischen Lösungsmittel löslich ist;
(2) dieses Salz mit einem Anhydrid der Formel:

$R_1$ - O - $R_1$

in der $R_1$ eine Acylgruppe, wie sie oben definiert worden ist, darstellt, in dem polaren aprotischen organischen Lösungsmittel in Gegenwart von katalytischen Mengen von Pyridin oder eines Dialkylaminopyridins und eines Protonenakzeptors bei einer Temperatur zwischen 0°C und 100°C behandelt;
(3) das in diese Weise erhaltene Produkt durch Einwirkung einer Lösung von Natriumacetat in Ethanol ausfällt und
(4) das selektiv O-acylierte Glykosaminoglykan durch Auflösen des in dieser Weise erhaltenen Niederschlags in Wasser und Dialyse isoliert und das in dieser Weise erhaltene Natriumsalz des selektiv O-acylierten Glykosaminoglykans gegebenenfalls in ein anderes pharmazeutisch annehmbares Salz umwandelt.

2. Verfahren zur Herstellung von selektiv O-acylierten Glykosaminoglykanen nach Anspruch 1, **dadurch gekennzeichnet,** daß man Verbindungen der folgenden Formel II herstellt:

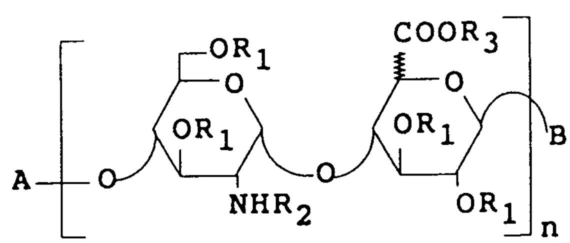

(II)

in der
- A die in Anspruch 1 angegebenen Bedeutungen besitzt;
- $R_1$ H und/oder $SO_3^-$und/oder eine Acylgruppe, wie sie bezüglich der Formel (I) definiert worden ist;
- $R_2$ $SO_3^-$ und/oder eine Acetylgruppe, mit der Maßgabe, daß der Anteil von N-Acetylglucosamin höchstens gleich ist jenem von Heparin, wenn $R_1$ eine Acetylgruppe darstellt;
- $R_3$ ein Wasserstoffatom, eine Alkylgruppe mit 1 bis 10 Kohlenstoffatomen, eine Phenylalkylgruppe mit 7 bis 12 Kohlenstoffatomen oder ein Alkalimetall- oder Erdalkalimetall-Kation;
- B (a)-$OR_1$ oder (f), wobei (a) und (f) die in Anspruch 1 angegebenen Bedeutungen besitzen, oder $OR_1$ oder eine Gruppe (a), an die ein Rest von (c) oder (d) gebunden ist, wie er nach der Periodoxidation gefolgt von einer $\beta$-Eliminierung oder einer sauren Hydrolyse vorhanden ist; und

- n eine ganze Zahl mit einem Wert von 1 bis 80 mit der Maßgabe bedeuten, daß, wenn A $R_1$, eine Gruppe $R_1$-(c) oder eine Gruppe $R_1$-(d) und B O-$R_1$, eine Gruppe (a)-$OR_1$ oder eine Gruppe (b)-$OR_1$ darstellen, n eine ganze Zahl mit einem Wert von 1 bis 16 bedeuten.

**3.** Verfahren zur Herstellung von selektiv O-acylierten Glykosaminoglykanen nach Anspruch 1, **dadurch gekennzeichnet,** daß man Verbindungen der allgemeinen Formel II herstellt:

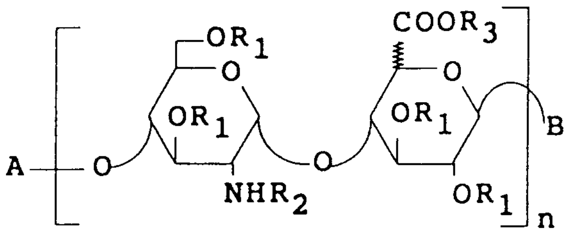

(II)

in der:

- A und B die in Anspruch 1 angegebenen Bedeutungen besitzen;
- $R_1$ H und/oder $SO_3^-$ und/oder eine Acylgruppe einer nicht-$\alpha$,$\beta$-ungesättigten Carbonsäure oder Dicarbonsäure ausgewählt aus:
  - einer Alkanoylgruppe mit 4 bis 18 Kohlenstoffatomen,
  - einer Alkanoylgruppe mit 2 bis 3 Kohlenstoffatomen substituiert durch:
    - . eine Cycloalkylgruppe mit 3 bis 7 Kohlenstoffatomen oder
    - . eine ungesättigte aliphatische Kohlenwasserstoffgruppe mit 4 bis 16 Kohlenstoffatomen;
  - einer Benzoylgruppe, die gegebenenfalls durch eine oder mehrere Alkylgruppen mit 1 bis 4 Kohlenstoffatomen, Halogenatome oder $NO_2$- oder $OCH_3$-Gruppen substituiert ist,
  - einer ($C_{3-7}$)-Cycloalkyl-carbonylgruppe;
- $R_2$ $SO_3^-$ und/oder eine Acetylgruppe mit der Maßgabe, daß der Anteil von N-Acetylglucosamin höchstens gleich ist jenem von Heparin, wenn $R_1$ eine Acetylgruppe darstellt;
- $R_3$ ein Wasserstoffatom, eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation; und
- n eine ganze Zahl mit einem Wert von 1 bis 80 mit der Maßgabe bedeuten, daß $R_1$ in dem Ausmaß Acyl bedeutet, daß mindestens 0,5 bis 2 Acylgruppen pro Disaccharid-Einheit vorliegen.

**4.** Verfahren zur Herstellung von selektiv O-acylierten Glykosaminoglykanen nach den Ansprüchen 1 oder 2, **dadurch gekennzeichnet,** daß das Glykosaminoglykan aus der Gruppe ausgewählt ist, die gebildet wird aus einer Mischung aus Heparinfragmenten mit einem Molekulargewicht von weniger als 10.000 Dalton, einer Mischung von Heparinfragmenten mit einem mittleren Molekulargewicht zwischen 2.000 und 7.000 Dalton, einer Mischung von Heparinfragmenten mit einem mittleren Molekulargewicht von etwa 4.500 Dalton, einer Mischung von Heparinfragmenten mit einem mittleren Molekulargewicht von etwa 2.500 Dalton und einer Mischung von Heparinfragmenten mit gleichem Molekulargewicht, wobei die Acylgruppe in einem Anteil von 0,5 bis 2 Gruppen pro Disaccharid-Einheit vorhanden ist.

**5.** Verfahren zur Herstellung von selektiv O-acylierten Glykosaminoglykanen nach Anspruch 3, **dadurch gekennzeichnet,** daß das Glykosaminoglykan Heparin ist und die Acylgruppe in einem Anteil von mindestens 0,5 bis 2 Gruppen pro Disaccharid-Einheit vorliegt.

**6.** Verfahren zur Herstellung von selektiv O-acylierten Glykosaminoglykanen nach Anspruch 5, **dadurch gekennzeichnet,** daß die Acylgruppe in einem Anteil von einer Acylgruppe pro Disaccharid-Einheit vorliegt.

**7.** Verfahren zur Herstellung von selektiv O-acylierten Glykosaminoglykanen nach einem der Ansprüche 1 bis 4, **dadurch gekennzeichnet,** daß man Verbindungen der folgenden Formel III herstellt:

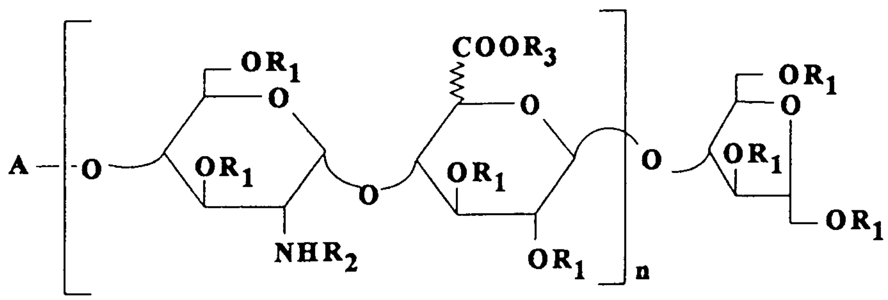

(III)

in der

- A $R_1$ oder $R_1$-(c) oder $R_1$-(d), wie sie in Anspruch 1 definiert worden sind;
- $R_1$ H und/oder $SO_3^-$ und/oder eine Acylgruppe, wie sie in Anspruch 1 definiert worden ist;
- $R_2$ $SO_3^-$ und/oder eine Acetylgruppe mit der Maßgabe, daß der Anteil von N-Acetylglucosamin höchstens gleich ist jenem von Heparin, wenn $R_1$ eine Acetylgruppe darstellt;
- $R_3$ ein Wasserstoffatom und/oder eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation; und
- n eine ganze Zahl mit einem Wert von 3 bis 12 bedeuten.

**8.** Verfahren zur Herstellung von selektiv O-acylierten Glykosaminoglykanen nach einem der Ansprüche 1 bis 4, **dadurch gekennzeichnet,** daß man Verbindungen der folgenden Formel IV herstellt:

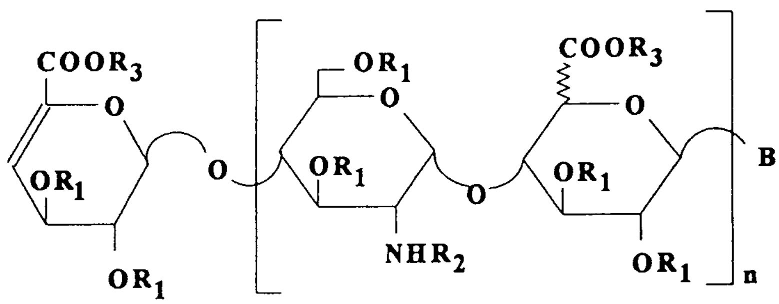

(IV)

in der:

- $R_1$ H und/oder $SO_3^-$ und/oder eine Acylgruppe, wie sie in Anspruch 1 definiert worden ist;
- $R_2$ $SO_3^-$ und/oder eine Acetylgruppe mit der Maßgabe, daß der Anteil von N-Acetylglucosamin höchstens gleich ist jenem von Heparin, wenn $R_1$ eine Acetylgruppe darstellt;
- $R_3$ ein Wasserstoffatom und/oder eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation;
- B (a)-$OR_1$, wie es in Anspruch 1 definiert worden ist, oder $OR_1$; und
- n eine ganze Zahl mit einem Wert von 2 bis 20 bedeuten.

**9.** Verfahren zur Herstellung von selektiv O-acylierten Glykosaminoglykanen nach den Ansprüchen 1 oder 2, **dadurch gekennzeichnet,** daß das ausgewählte Glykosaminoglykan Heparin oder eine Heparinfraktion oder ein Heparinfragment, welches frei ist von Bindungsstellen für Antithrombin III, ist.

**10.** Verfahren zur Herstellung von selektiv O-acylierten Glykosaminoglykanen nach einem der Ansprüche 1, 2, 3 und 9, **dadurch gekennzeichnet,** daß man Verbindungen der folgenden Formel V herstellt:

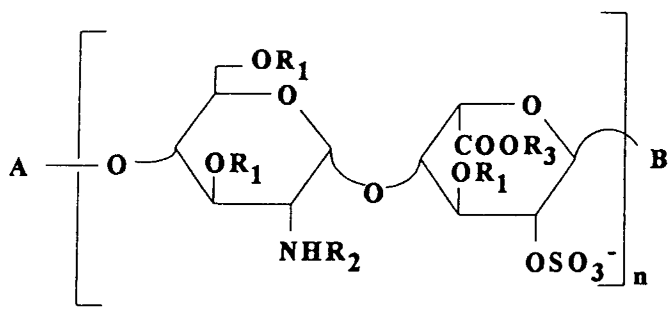

(V)

in der:

- A $R_1$-(c), $R_1$-(d) oder den Rest von (c) oder (d) nach der Periodoxidation gefolgt von einer $\beta$-Eliminierung oder einer sauren Hydrolyse;
- B eine Gruppe (a)-$OR_1$, eine Gruppe (a), an welche ein Rest von (c) oder von (d) gebunden ist, wie er nach der Periodoxidation gefolgt von einer $\beta$-Eliminierung oder einer sauren Hydrolyse vorhanden ist;
- $R_1$ die in Anspruch 1 angegebenen Bedeutungen;
- $R_2$ $SO_3^-$ oder eine Acetylgruppe, wobei der Anteil von $SO_3^-$ etwa 90 % beträgt;
- $R_3$ ein Wasserstoffatom oder ein Alkalimetall- oder ein Erdalkalimetall-Kation; und
- n eine ganze Zahl mit einem Wert von 1 bis 80 bedeuten.

**11.** Verfahren zur Herstellung von selektiv O-acylierten Glykosaminoglykanen nach einem der Ansprüche 1, 2, 3 und 9, **dadurch gekennzeichnet,** daß man Verbindungen der folgenden Formel VI herstellt:

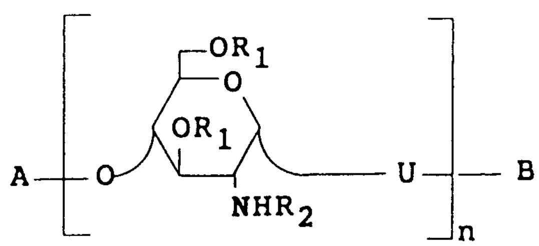

(VI)

in der:

- A $R_1$, $R_1$-(c), $R_1$-(d) oder den Rest von (c) oder von (d) nach der Periodoxidation gefolgt von einer $\beta$-Eliminierung;
- U eine Gruppe der Formel:

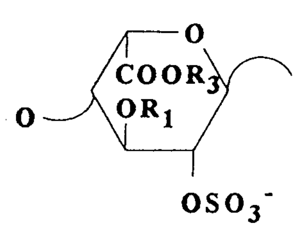

aus der sich für mindestens zwei Ketten eine geöffnete nichtsulfatierte Uronsäure (D-Glucuronsäure oder L-Iduronsäure) zwischen den Kohlenstoffatomen in den Positionen 2 und 3 ergibt, der Formel:

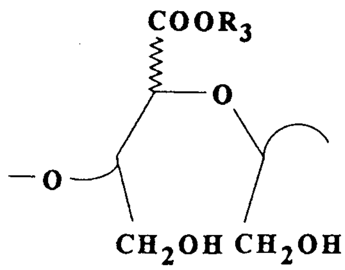

- B (a)-$OR_1$ oder $OR_1$ oder eine Gruppe (a), an die ein Rest von (c) oder von (d) gebunden ist, wie er nach der Periodoxidation gefolgt von einer $\beta$-Eliminierung vorhanden ist;
- $R_1$ die in Anspruch 1 angegebenen Bedeutungen;
- $R_2$ $SO_3^-$ oder Acetylgruppe, wobei der Anteil von $SO_3^-$ etwa 90 % beträgt;
- $R_3$ ein Wasserstoffatom oder ein Alkalimetall- oder Erdalkalimetall-Kation; und
- n eine ganze Zahl mit einem Wert von 2 bis 18 bedeuten.

**12.** Verfahren zur Herstellung von selektiv O-acylierten Glykosaminoglykanen nach einem der Ansprüche 1, 2, 3 und 9, **dadurch gekennzeichnet,** daß man Verbindungen der Formel VI herstellt, in der:
- n für die Mehrheit der vorhandenen Moleküle eine ganze Zahl zwischen 7 bis 15 bedeutet und
- $R_1$ eine Alkanoylgruppe mit 2 bis 10 Kohlenstoffatomen darstellt.

**13.** Verfahren zur Herstellung von selektiv O-acylierten Glykosaminoglykanen gemäß den Ansprüchen 11 und 12, **dadurch gekennzeichnet,** daß sie aus einer Mischung von Fragmenten mit gleichem Molekulargewicht gebildet sind, in denen n eine ganze Zahl mit einem Wert von 2 bis 12 und $R_1$ eine Alkanoylgruppe mit 4 bis 10 Kohlenstoffatomen bedeuten.

**14.** Verfahren zur Herstellung von selektiv O-acylierten Glykosaminoglykanen nach einem der Ansprüche 1, 2, 3 und 9, **dadurch gekennzeichnet,** daß man Verbindungen der folgenden Formel VII herstellt:

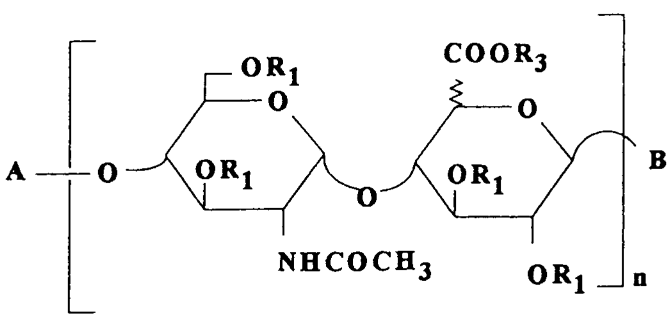

(VII)

in der:
- A $R_1$, $R_1$-(c) oder $R_1$-(d), wie sie bezüglich der Formel (I) definiert worden sind;
- $R_1$ eine Alkanoylgruppe mit 2 bis 18 Kohlenstoffatomen;
- $R_3$ ein Wasserstoffatom oder ein Alkalimetall- oder Erdalkalimetall-Kation;
- B (a)-$OR_1$, wie es bezüglich der Formel (I) definiert worden ist, oder $OR_1$; und
- n eine ganze Zahl mit einem Wert von 1 bis 80 bedeuten.

**15.** Verfahren zur Herstellung von selektiv O-acylierten Glykosaminoglykanen nach Anspruch 1, **dadurch gekennzeichnet,** daß man Verbindungen der folgenden Formel VIII herstellt:

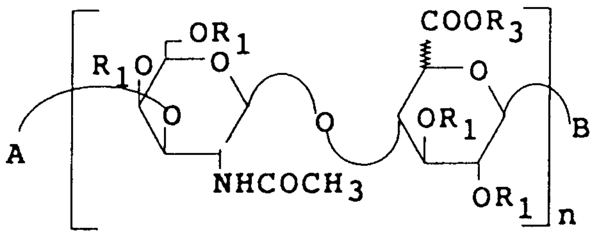

(VIII)

in der:

- A die in Anspruch 1 angegebenen Bedeutungen besitzt;
- $R_1$ H und/oder $SO_3^-$ und/oder eine Acylgruppe, wie sie in Anspruch 1 definiert worden ist;
- $R_3$ ein Wasserstoffatom, eine Alkylgruppe mit 1 bis 10 Kohlenstoffatomen, eine Phenylalkylgruppe mit 7 bis 12 Kohlenstoffatomen oder ein Alkalimetall-oder Erdalkalimetall-Kation;
- B (b)-$OR_1$ oder (g), wie sie bezüglich der Formel (I) definiert worden sind, oder $OR_1$ oder eine Gruppe (b), an die ein Rest von (c) oder von (d) gebunden ist, wie er nach der Periodoxidation gefolgt von einer $\beta$-Eliminierung oder einer sauren Hydrolyse vorhanden ist; und
- n eine ganze Zahl mit einem Wert von 1 bis 80 bedeuten.

**16.** Verfahren zur Herstellung von selektiv O-acylierten Glykosaminoglykanen nach den Ansprüchen 1 oder 15, **dadurch gekennzeichnet,** daß das Glykosaminoglykan aus der Gruppe ausgewählt ist, die Dermatansulfat und dessen Fragmente umfaßt, wobei die Acylgruppe in einem Anteil von mindestens 0,1 bis 3 Gruppen pro Disaccharid-Einheit vorhanden ist.

**17.** Verfahren zur Herstellung von selektiv O-acylierten Glykosaminoglykanen nach einem der Ansprüche 1 bis 16, worin $R_1$ eine Alkanoylgruppe mit 4 bis 10 Kohlenstoffatomen darstellt.

**18.** Verfahren zur Herstellung von selektiv O-acylierten Glykosaminoglykanen nach einem der Ansprüche 1 bis 17, **dadurch gekennzeichnet,** daß man:

(1) ein Glykosaminoglykan der Formel IX:

$$A° - [G° - U°]_n - B° \qquad (IX)$$

in der:

- G° eine Gruppe (a)° der Formel:

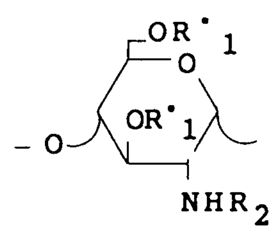

(a)°

oder eine Gruppe (a')° der Formel:

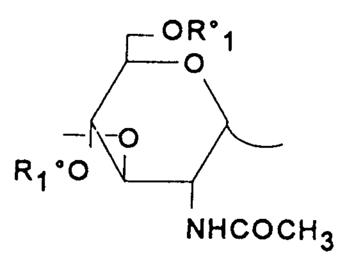

(a')°

oder eine Gruppe (b)° der Formel:

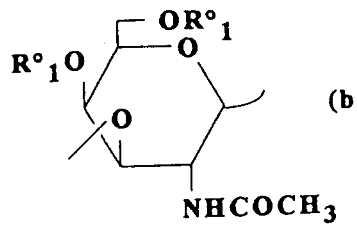

(b)°

- U° eine Gruppe (c)° der Formel:

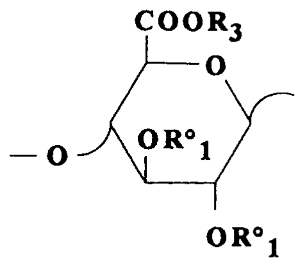

(c)°

oder eine Gruppe (d)° der Formel:

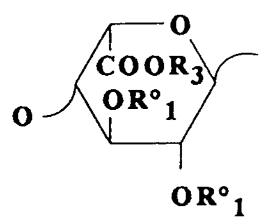

(d)°

oder den Rest der Gruppe (c)° oder der Gruppe (d)° nach der Periodoxidation gefolgt von einer $\beta$-Eliminierung oder einer sauren Hydrolyse;

- A° eine Gruppe $R°_1$ oder eine Gruppe $R°_1$-(c)°, eine Gruppe $R°_1$-(d)° oder eine Gruppe (e)-° der Formel:

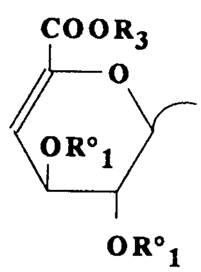

(e)°

oder den Rest der Gruppe (c)° oder der Gruppe (d)° nach der Periodoxidation gefolgt von einer $\beta$-Eliminierung oder einer sauren Hydrolyse;

- B° eine Gruppe $OR°_1$, eine Gruppe (a)°-$OR°_1$, eine Gruppe (a')°-$OR°_1$, eine Gruppe (b)°-$OR°_1$ oder eine Gruppe (f)° der Formel:

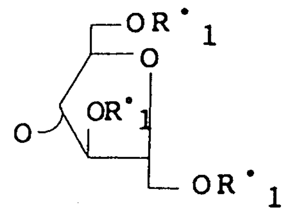

(f)°

oder eine Gruppe (g)° der Formel:

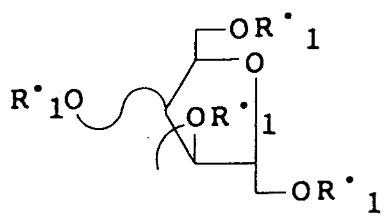

(g)°

oder Gruppen (a)°, (a')°, (b)°, an die ein Rest von (c)° oder von (d)° gebunden ist, wie er nach der Periodoxidation gefolgt von einer β-Eliminierung oder einer sauren Hydrolyse vorhanden ist;

- $R_1$° H oder $SO_3^-$;
- $R_2$ $SO_3^-$ oder eine Acetylgruppe mit der Maßgabe, daß der Anteil von N-Acetylglucosamin höchstens gleich ist dem von Heparin, wenn $R_1$ eine Acetylgruppe darstellt;
- $R_3$ ein Wasserstoffatom, eine Alkylgruppe mit 1 bis 10 Kohlenstoffatmen, eine Phenylalkylgruppe mit 7 bis 12 Kohlenstoffatomen oder ein Alkalimetall-oder Erdalkalimetall-Kation; und
- n eine ganze Zahl mit einem Wert von 1 bis 80 bedeuten,

in ein tertiäres Aminsalz oder ein quaternäres Ammoniumsalz des Glykosaminoglykans umwandelt, welches in einem aprotischen polaren Lösungsmittel löslich ist;

(2) dieses Salz mit einem Anhydrid der Formel:

$R_1$ - O - $R_1$

in der $R_1$ eine Acylgruppe bedeutet, wie sie in Anspruch 1 definiert worden ist, in dem polaren aprotischen organischen Lösungsmittel in Gegenwart von katalytischen Mengen von Pyridin oder eines Dialkylaminopyridins und eines Protonenakzeptors bei einer Temperatur zwischen 0°C und 100°C behandelt;

(3) das in dieser Weise erhaltene Produkt durch Einwirkung einer Lösung von Natriumacetat in Ethanol ausfällt und

(4) das selektiv O-acylierte Glykosaminoglykan durch Auflösen des in dieser Weise erhaltenen Niederschlags in Wasser und Dialyse isoliert und das in dieser Weise erhaltene Natriumsalz des selektiv O-acylierten Glykosaminoglykans gegebenenfalls in ein anderes pharmazeutisch annehmbares Salz umwandelt.

**19.** Verfahren nach Anspruch 18, **dadurch gekennzeichnet,** daß das in der Stufe (1) eingesetzte Glykosaminoglykan aus der Gruppe ausgewählt ist, die durch Heparin, einer Mischung von Heparinfragmenten mit einem Molekulargewicht von weniger als 10.000 Dalton, einer Mischung von Heparinfragmenten mit einem mittleren Molekulargewicht zwischen 2.000 und 7.000 Dalton, einer Mischung von Heparinfragmenten mit einem mittleren Molekulargewicht von etwa 4.500 Dalton, einer Mischung von Heparinfragmenten mit einem Molekulargewicht von etwa 2.500 Dalton oder einer Mischung von Heparinfragmenten mit dem gleichen Molekulargewicht gebildet wird.

**20.** Verfahren nach Anspruch 18, **dadurch gekennzeichnet,** daß das in der Stufe (1) eingesetzte Glykosaminoglykan Heparin oder eine Heparinfraktion oder ein Heparinfragment ist, welches frei von Bindungsstellen für Antithrombin III ist.

21. Verfahren nach Anspruch 18, **dadurch gekennzeichnet,** daß das in der Stufe (1) eingesetzte Glykosaminoglykan aus der Gruppe ausgewählt ist, die durch Dermatansulfat und seinen Fragmenten gebildet wird.

22. Verfahren nach einem der Ansprüche 18 bis 21, **dadurch gekennzeichnet,** daß das Glykosaminoglykansalz der Stufe (1) ein Tributylaminsalz oder ein Tetrabutylammoniumsalz ist.

23. Verfahren nach einem der Ansprüche 18 bis 22, **dadurch gekennzeichnet,** daß das in der Stufe (2) eingesetzte Anhydrid ein Anhydrid einer Alkansäure mit 2 bis 10 Kohlenstoffatomen ist.

24. Verfahren nach einem der Ansprüche 18 bis 23, **dadurch gekennzeichnet,** daß die Stufe (2) in einem polaren aprotischen Lösungsmittel durchgeführt wird ausgewählt aus der Gruppe, die durch Dimethylformamid, Hexamethylphosphortriamid, Pyridin oder einer Mischung dieser Lösungsmittel untereinander oder mit Dichlormethan gebildet wird.

25. Verfahren nach einem der Ansprüche 18 bis 24, **dadurch gekennzeichnet,** daß man als Protonenakzeptor in der Stufe (2) eine Base verwendet, die aus der Gruppe ausgewählt ist, die durch Pyridin, Triethylamin und Tributylamin gebildet wird.

26. Verfahren nach einem der Ansprüche 18 bis 25, **dadurch gekennzeichnet,** daß die Dialyse der Stufe (4) in Gegenwart einer schwachen Base durchgeführt wird.

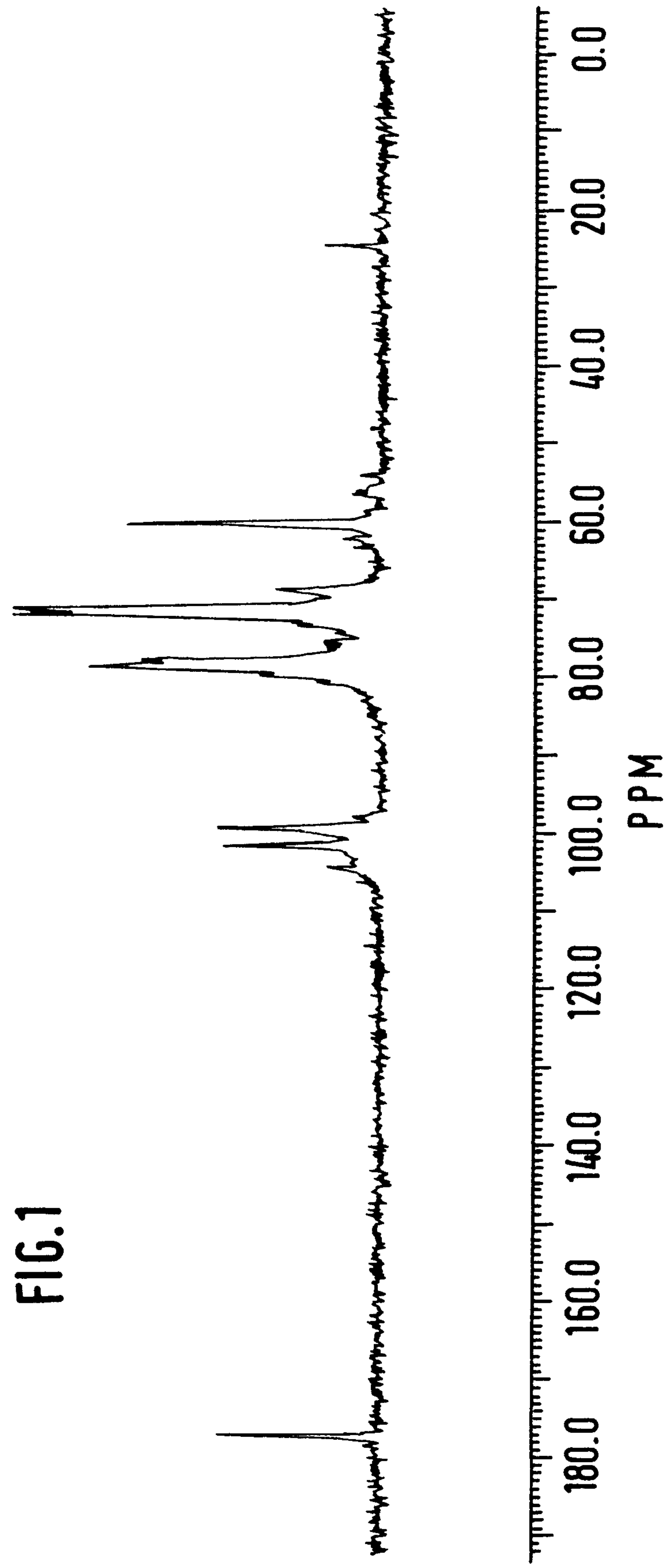


FIG.1

**Figure 2**

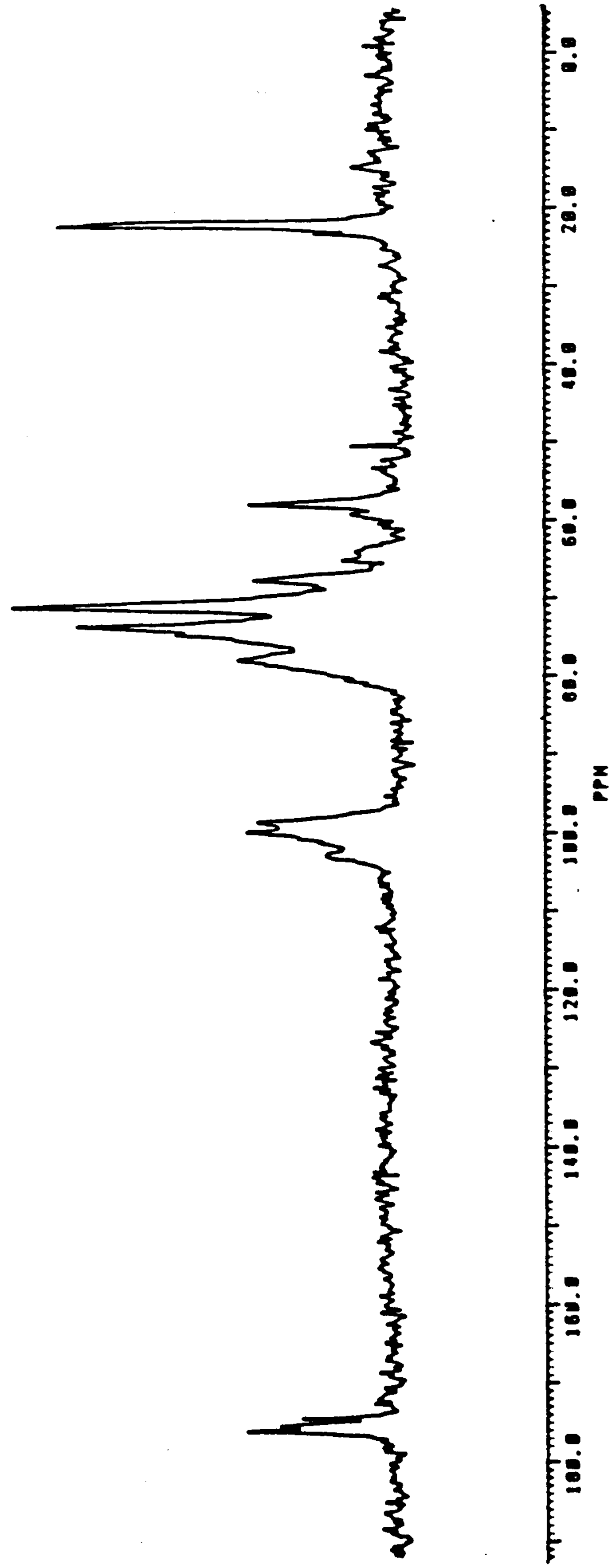

Figure 3

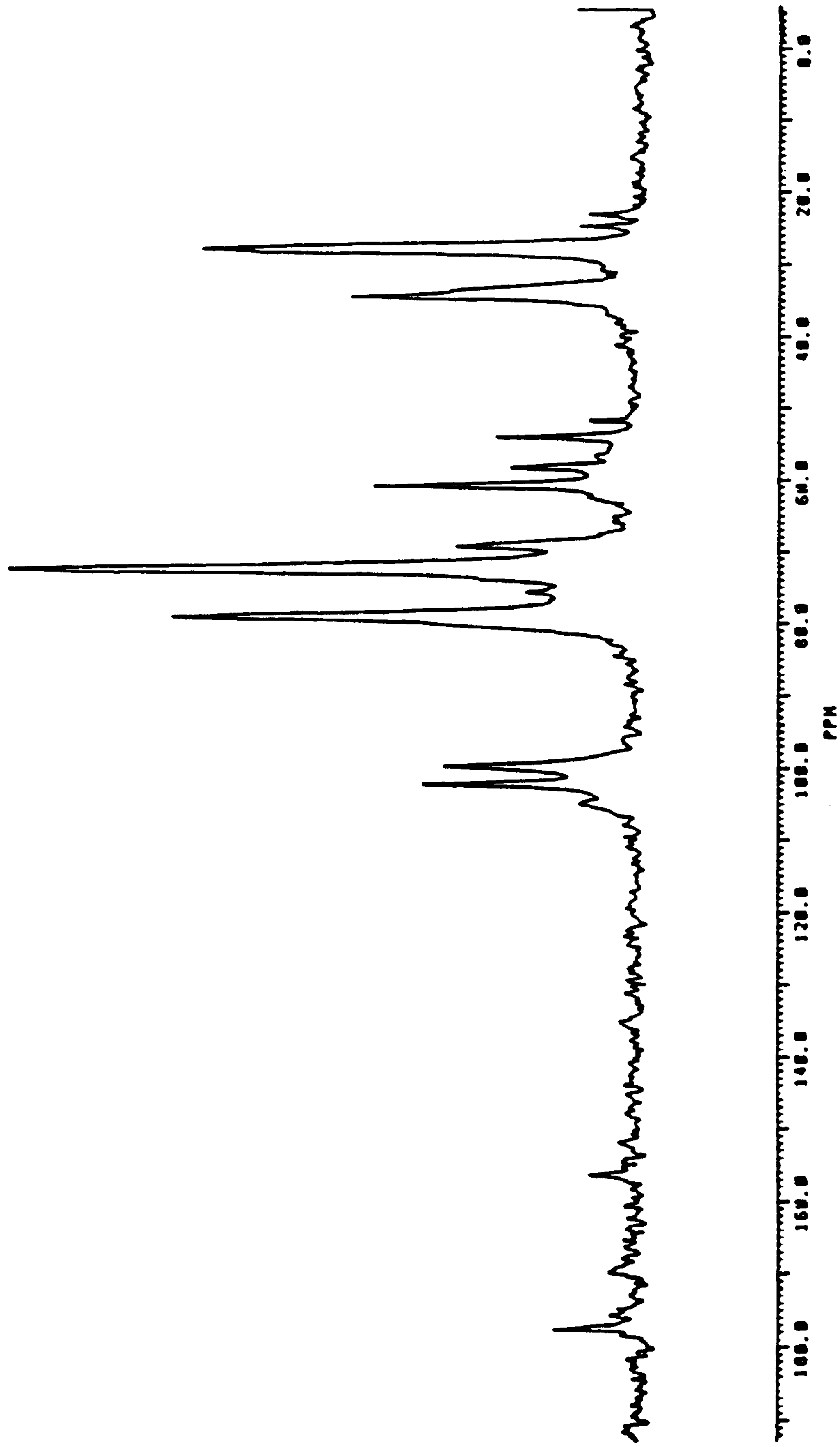

**Figure 4**

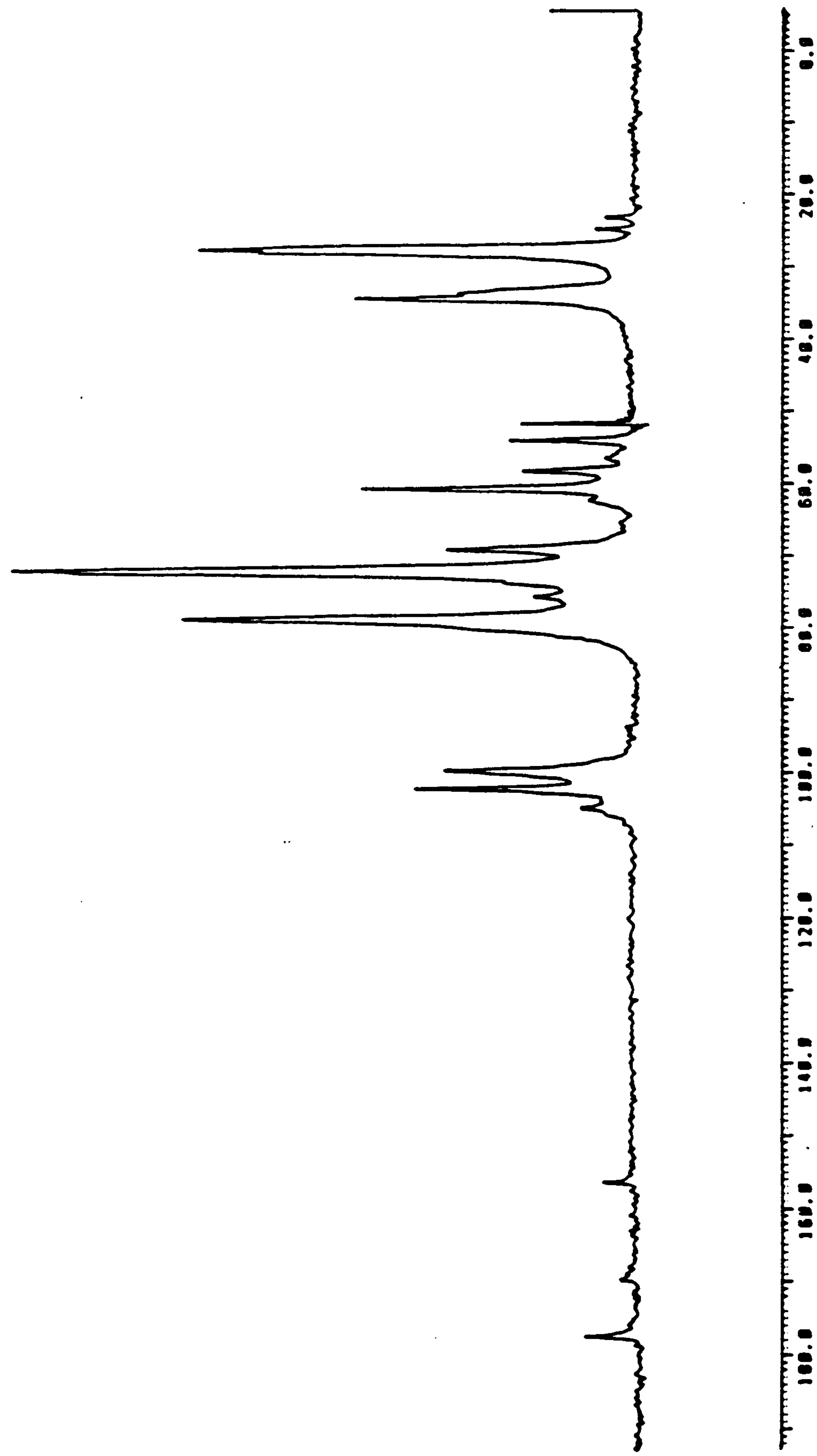

Figure 5

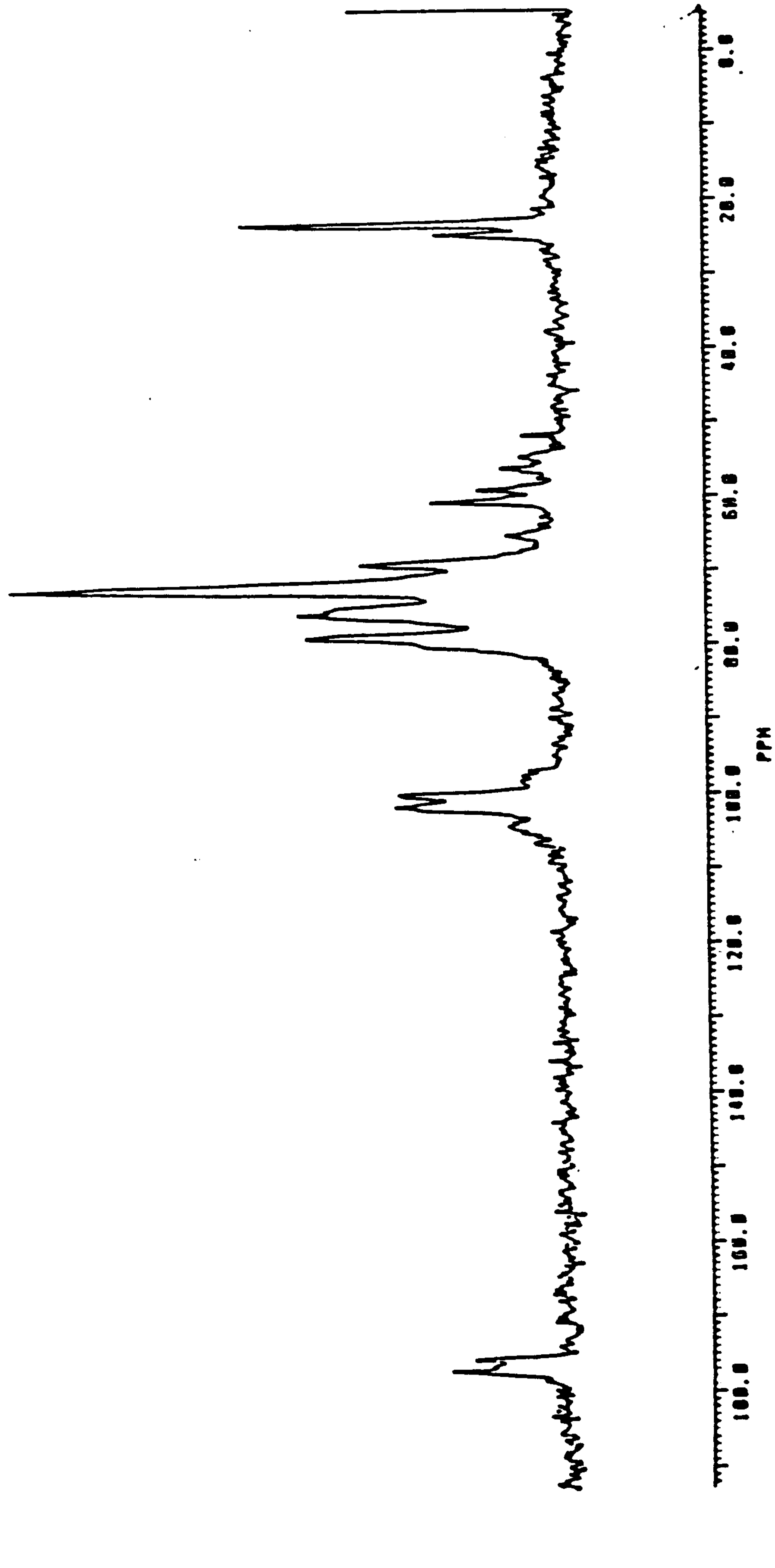

Figure 6

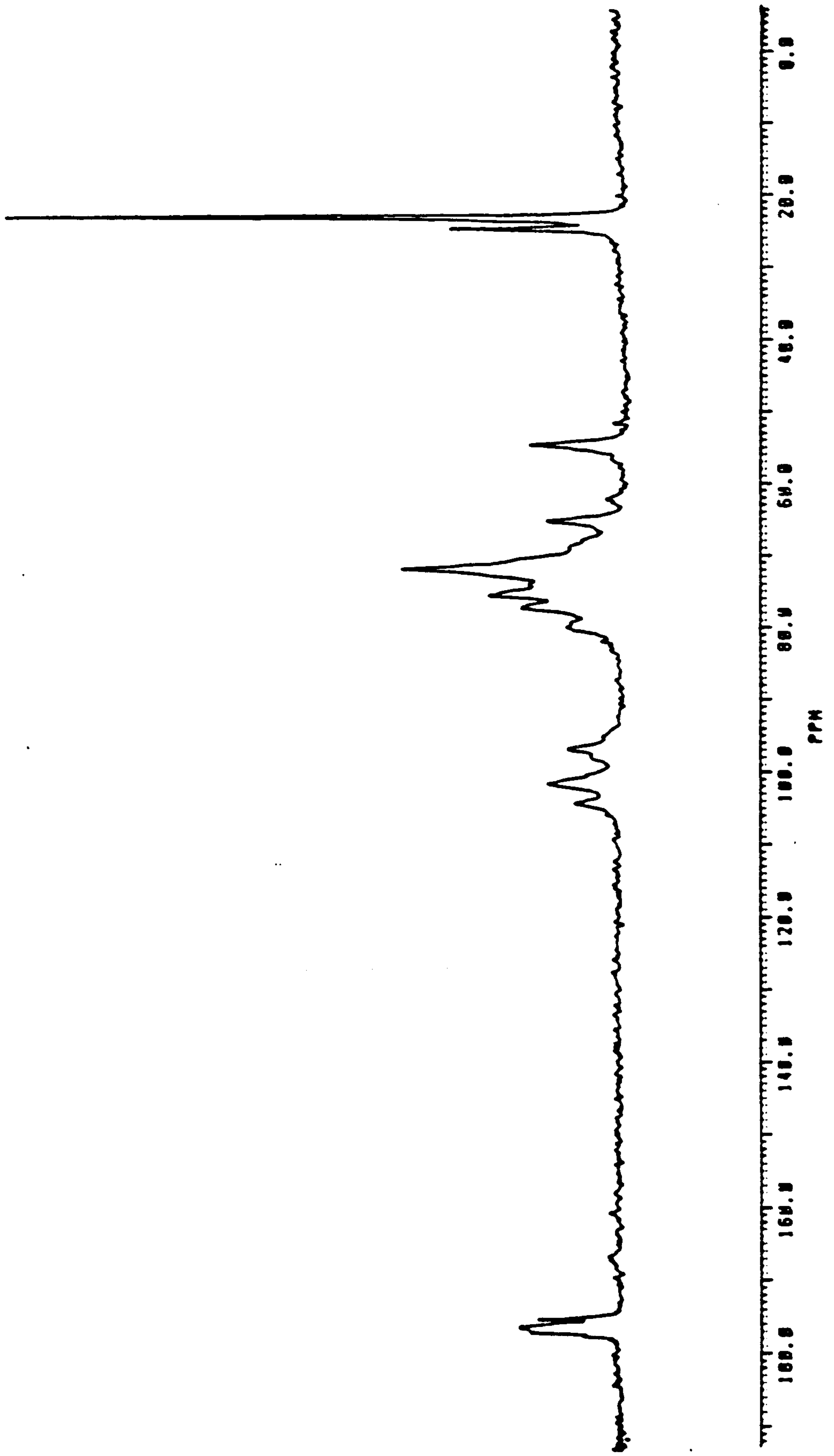

Figure 7

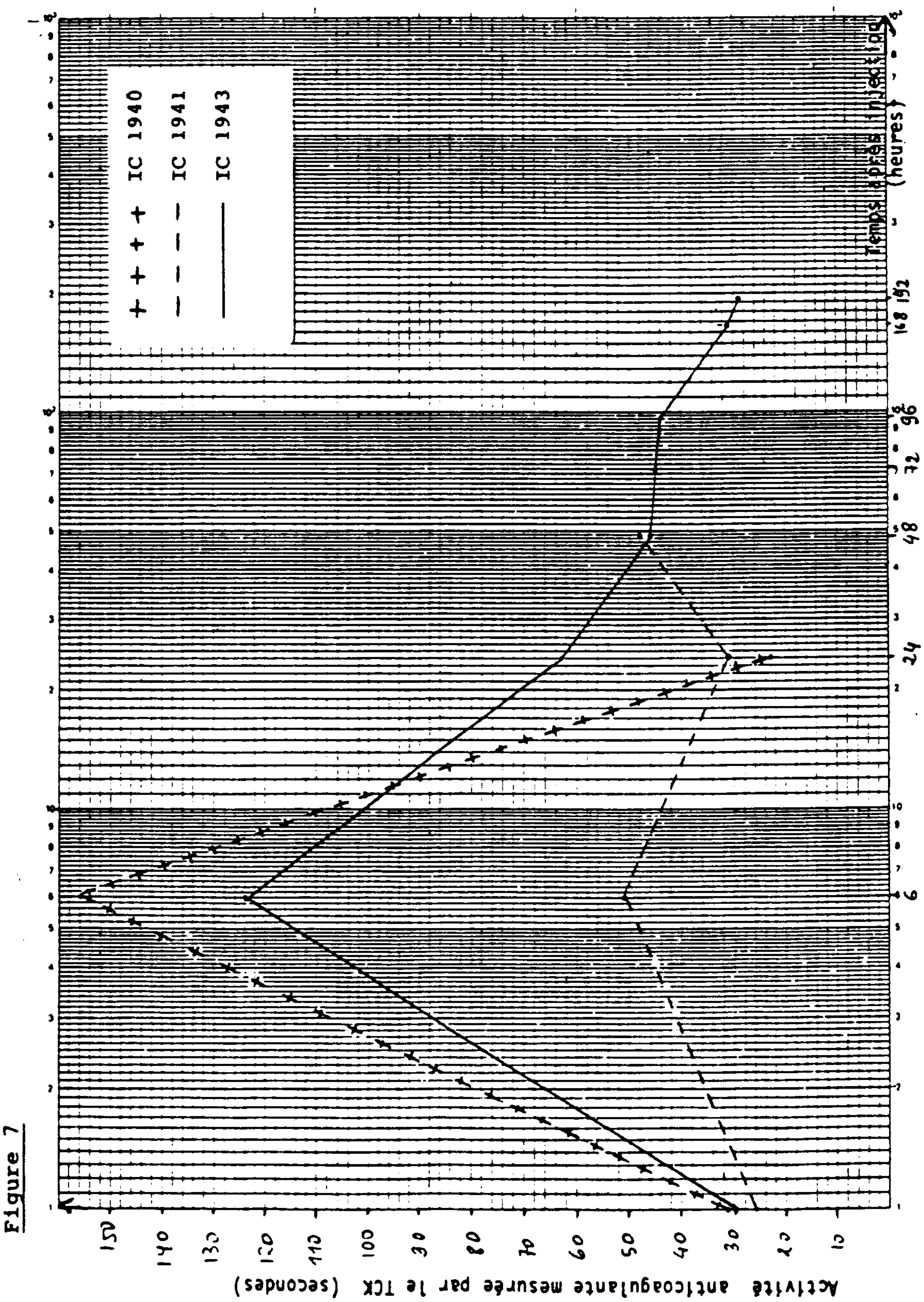
+ + + + IC 1940
— — — IC 1941
——— IC 1943
Temps après injection (heures)
Activité anticoagulante mesurée par le TCK (secondes)
150
140
130
120
110
100
90
80
70
60
50
40
30
20
10
6
24
48
72
96
168
192

Figure 8

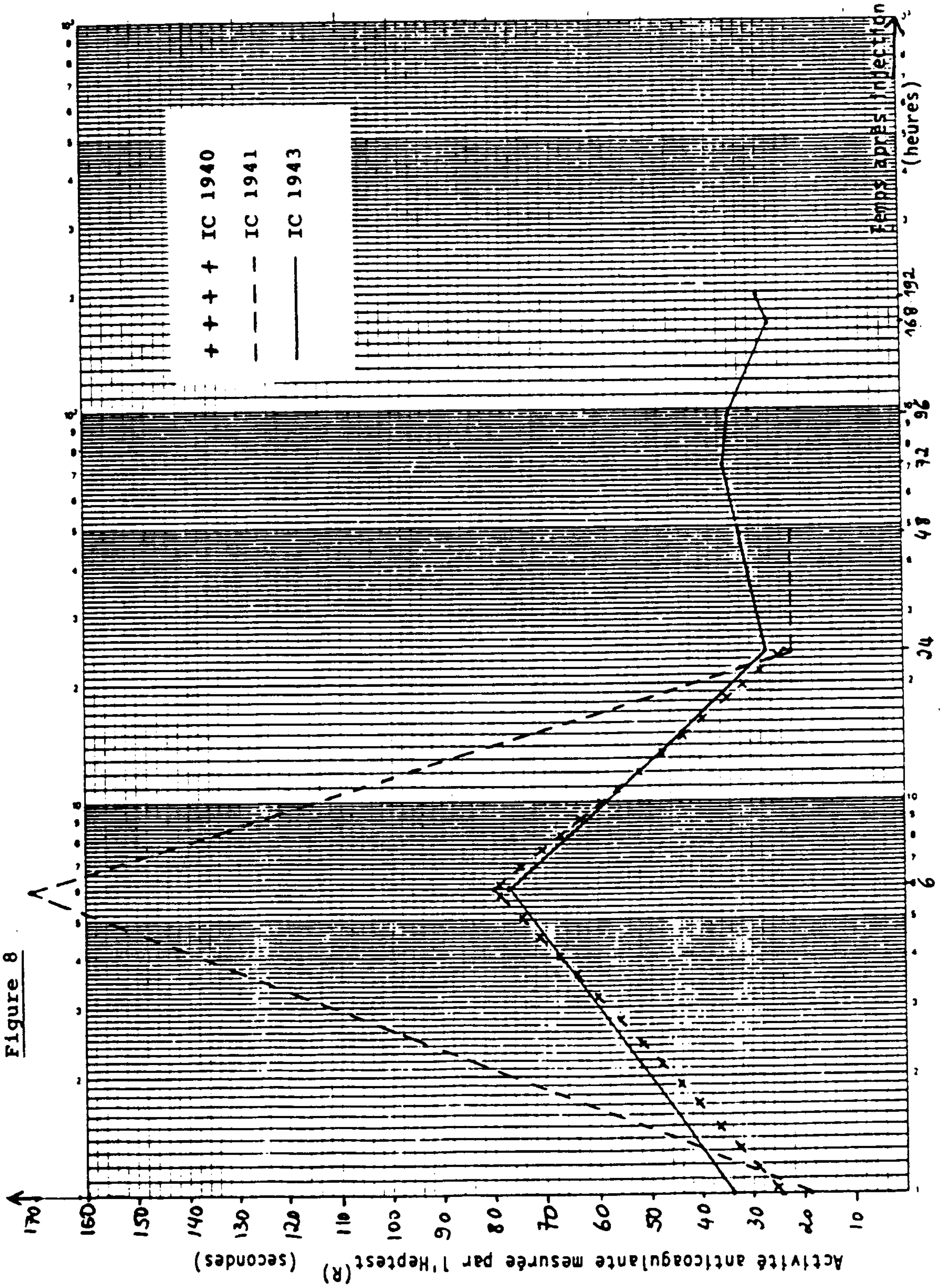
+ + + + IC 1940
— — — — IC 1941
———— IC 1943
Temps après injection (heures)
Activité anticoagulante mesurée par l'Heptest(R) (secondes)
170
160
150
140
130
120
110
100
90
80
70
60
50
40
30
20
10
6
24
48
72
96
168
192